# EUROPEAN PATENT APPLICATION

(11) **EP 4 725 534 A2**
(43) Date of publication of application: **15.04.2026**
(21) Application number: 26152504.2
(22) Date of filing: 31.03.2022
(51) Int. Cl.: A61M 16/08

(54) **VENT ARRANGEMENTS FOR PATIENT INTERFACES**

(30) Priority: 31.03.2021 AU 2021900947
(62) Divisional of application: 22778216.6
(71) Applicant: ResMed Pty Ltd, Bella Vista, NSW 2153 (AU)
(72) Inventor: The designation of the inventor has not yet been filed
(74) Representative: Vossius & Partner Patentanwälte Rechtsanwälte mbB

(57) **Abstract**

The present invention discloses a patient interface comprising: a plenum chamber pressurisable to a therapeutic pressure of at least 6 cmH2O above ambient air pressure, wherein the plenum chamber comprises an inlet configured to receive a flow of air at the therapeutic pressure for breathing by a patient; a seal-forming structure configured to form a seal with a region of the patient's face surrounding an entrance to the patient's airways, wherein the seal-forming structure is configured to maintain said therapeutic pressure in the plenum chamber throughout the patient's respiratory cycle in use; a vent structure configured to allow a flow of gases exhaled by the patient from an interior of the plenum chamber to ambient, wherein the vent structure is configured to maintain the therapeutic pressure in the plenum chamber in use; and a positioning and stabilising structure to provide a force to hold the seal-forming structure in a therapeutically effective position on the patient's head, wherein the positioning and stabilising structure comprises at least one headgear strap, wherein the at least one headgear strap comprises a diffuser, wherein the diffuser is positioned in use to diffuse the flow of gases exiting the vent structure.

## Description

### 1 BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO2 to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 1.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube or endotracheal tube. In some forms, the comfort and effectiveness of these therapies may be improved.

### 1.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that may be held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO2 from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched air at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 1.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH2O relative to ambient pressure.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

Certain masks may cause some patients a feeling of claustrophobia, unease and/or may feel overly obtrusive.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, noisy or difficult to use a patient may not comply with therapy.

Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 1.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO2004/073,778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063,328 and WO 2006/130,903 (describing amongst other things aspects of the ResMed Limited MIRAGE LIBERTYTM full-face mask); International Patent Application WO 2009/052,560 (describing amongst other things aspects of the ResMed Limited SWIFTTM FX nasal pillows).

### 1.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

Several factors may be considered when comparing different positioning and stabilising techniques. These include: how effective the technique is at maintaining the seal-forming structure in the desired position and in sealed engagement with the face during use of the patient interface; how comfortable the interface is for the patient; whether the patient feels intrusiveness and/or claustrophobia when wearing the patient interface; and aesthetic appeal.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 1.2.3.1.3 Pressurised Air Conduit

In one type of treatment system, a flow of pressurised air is provided to a patient interface through a conduit in an air circuit that fluidly connects to the patient interface at a location that is in front of the patient's face when the patient interface is positioned on the patient's face during use. The conduit may extend from the patient interface forwards away from the patient's face.

### 1.2.3.1.4 Pressurised Air Conduit used for Positioning / Stabilising the Seal-Forming Structure

Another type of treatment system comprises a patient interface in which a tube that delivers pressurised air to the patient's airways also functions as part of the headgear to position and stabilise the seal-forming portion of the patient interface at the appropriate part of the patient's face. This type of patient interface may be referred to as having "conduit headgear" or "headgear tubing". Such patient interfaces allow the conduit in the air circuit providing the flow of pressurised air from a respiratory pressure therapy (RPT) device to connect to the patient interface in a position other than in front of the patient's face. One example of such a treatment system is disclosed in US Patent Publication No. US 2007/0246043, the contents of which are incorporated herein by reference, in which the conduit connects to a tube in the patient interface through a port positioned in use on top of the patient's head.

It is desirable for patient interfaces incorporating headgear tubing to be comfortable for a patient to wear over a prolonged duration when the patient is asleep, form an air-tight and stable seal with the patient's face, while also able to fit a range of patient head shapes and sizes.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 1.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 1.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

### 1.2.3.5 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

**Table of noise of prior masks (ISO 17510-2:2007, 10 cmH2O pressure at 1m)**

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed MirageTM (*) | nasal | 29.5 | 21.5 | 1998 |
| ResMed UltraMirageTM | nasal | 36 (3) | 28 (3) | 2000 |
| ResMed Mirage ActivaTM | nasal | 32 (3) | 24 (3) | 2002 |
| ResMed Mirage MicroTM | nasal | 30 (3) | 22 (3) | 2008 |
| ResMed MirageTM SoftGel | nasal | 29 (3) | 22 (3) | 2008 |
| ResMed MirageTM FX | nasal | 26 (3) | 18 (3) | 2010 |
| ResMed Mirage SwiftTM (*) | nasal pillows | 37 | 29 | 2004 |
| ResMed Mirage SwiftTM II | nasal pillows | 28 (3) | 20 (3) | 2005 |
| ResMed Mirage SwiftTM LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO 3744 in CPAP mode at 10 cmH2O) | | | | |

Sound pressure values of a variety of objects are listed below

| Object | A-weighted sound pressure dB(A) | Notes |
|---|---|---|
| Vacuum cleaner: Nilfisk | 68 | ISO 3744 at 1m distance |
| Walter Broadly Litter Hog: B+ Grade | | |
| Conversational speech | 60 | 1m distance |
| Average home | 50 | |
| Quiet library | 40 | |
| Quiet bedroom at night | 30 | |
| Background in TV studio | 20 | |

The shearing effect of or contact between air flowing in different directions within the patient interface may cause turbulence, which may cause noise. This effect may be affected by the patient's breathing cycle, causing the noise produced to be cyclic. Inside the patient interface air received from the air delivery tube may travel in a direction that is different from, and may be opposing, the air exhaled by the patient. During exhalation, the flow of air exhaled by the patient may shear / contact the flow of air entering the patient interface from the tube. This may cause turbulence, and consequently noise. During inhalation or breath pause (i.e. a period between inhalation and exhalation), the flow of air entering the patient interface from the tube may shear / contact the flow of air within the patient interface. This may cause turbulence, and consequently noise. The cyclic nature of the noise may be particularly undesirable.

Noise may also be created when a patient interface is being used by a patient lying on their side when air is vented sidewards from the patient interface. The air flow may contact an object, e.g. a pillow, or another part of the patient's body, e.g. the patient's hand, while they are sleeping, which may create noise. This may increase discomfort and therefore may cause non-compliance with therapy. In some forms, when a patient using a patient interface is lying on their back, air exhausted to the side from the patient interface may flow towards and disrupt the patient's sleeping partner. This may disrupt or bring discomfort to the patient's sleeping partner, and may increase patient non-compliance.

Diffusing vented air flow from the patient interface can help decrease noise. It may also reduce disturbance to the patient's sleeping partner by diffusing vented air flow directed towards the sleeping partner during use. However, a diffuser requires additional components / parts for the mask, which may increase manufacturing costs and complexity.

### 2 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of the technology relates to a vent structure for a patient interface. Another aspect relates to a patient interface comprising a vent structure. A still further aspect relates to a positioning and stabilising structure for holding a patient interface in a therapeutically effective position on a patient's head.

Another aspect of the present technology relates to a patient interface. The patient interface may comprise a plenum chamber pressurisable to a therapeutic pressure of at least 6 cmH2O above ambient air pressure. The plenum chamber may comprise an inlet configured to receive a flow of air at the therapeutic pressure for breathing by a patient. The patient interface may further comprise a seal-forming structure configured to form a seal with a region of the patient's face surrounding an entrance to the patient's airways. The seal-forming structure may be configured to maintain said therapeutic pressure in the plenum chamber throughout the patient's respiratory cycle in use. The patient interface may further comprise a vent structure configured to allow a flow of gases exhaled by the patient from an interior of the plenum chamber to ambient. The vent structure may be configured to maintain the therapeutic pressure in the plenum chamber in use.

In examples:
- The plenum chamber may comprise an anterior portion.
- The anterior portion may comprise the inlet.
- The plenum chamber may comprise a medial portion.
- The medial portion may be located so that, in use, the mid-sagittal plane of the patient passes through the medial portion.
- The inlet may be located on the medial portion.
- The patient interface may comprise an air delivery tube which connects to the plenum chamber and is configured to be in fluid communication with the inlet.

In examples:
- The vent structure may be configured to vent the flow of gases from the interior of the plenum chamber in a substantially lateral direction in use.
- The vent structure may comprise a plurality of vent holes.
- The vent structure may be located on a portion of the patient interface which is lateral to the inlet in use.
- The vent structure may be located on the plenum chamber.
- The vent structure may comprise a vent module.
- The patient interface may comprise a vent module opening.
- The vent module may be configured to attach to a portion of the patient interface comprising the vent module opening.
- The vent structure may be a first vent structure configured to vent a first flow of gases from the interior of the plenum chamber in use, and wherein the patient interface may comprise a second vent structure configured to vent a second flow of gases from the interior of the plenum chamber.
- The first vent structure may be configured to vent the first flow of gases from the interior of the plenum chamber in a first substantially lateral direction in use.
- The second vent structure may configured to vent the second flow of gases from the interior of the plenum chamber in a second substantially lateral direction in use.
- The first substantially lateral direction may be generally opposite to the second substantially lateral direction.
- The first vent structure may be located lateral to the inlet on one side of the patient interface.
- The second vent structure may be located lateral to the inlet on the other side of the patient interface.

In examples:
- The patient interface may comprise a deflector configured to redirect at least a portion of the vented flow of gases in use.
- The patient interface may comprise a deflector configured to redirect a substantial amount of the vented flow of gases in use.
- The patient interface may comprise a deflector configured to redirect, in use, at least a portion of the laterally vented flow of gases in a direction having an anterior component relative to the patient.
- The patient interface may comprise a deflector configured to redirect, in use, a substantial amount of the laterally vented flow of gases in a direction having an anterior component relative to the patient.
- The patient interface may comprise a deflector configured to redirect, in use, a substantial amount of the laterally vented flow of gases in a substantially anterior direction.
- The deflector may comprise an arrangement of one or more deflector walls.
- The deflector may be configured to redirect a/the substantial amount of the laterally vented flow of gases towards a/the medial portion.
- The deflector may be configured to redirect a/the substantial amount of the laterally vented flow of gases towards an/the anterior portion of the plenum chamber in use.
- The deflector may be configured to redirect a/the substantial amount of the laterally vented flow of gases towards a portion of the air delivery tube in use.
- The deflector may be comprised as part of the vent structure.
- The deflector may comprise a first deflector configured to redirect a first vented flow of gases in use, and wherein the patient interface comprises a second deflector configured to redirect a second vented flow of gases.
- The patient interface may comprise a first deflector configured to redirect a substantial amount of the first flow of gases in at least a first direction in use, and wherein the patient interface comprises a second deflector configured to redirect a substantial amount of the second flow of gases in a second direction in use.
- The positioning and stabilising structure may comprise a first deflector configured to redirect a substantial amount of the first flow of gases in at least a first direction in use, and wherein the positioning and stabilising structure comprises a second deflector configured to redirect a substantial amount of a/the second flow of gases in a second direction in use.
- The first vented flow of gases may be a first laterally vented flow of gases.
- The second vented flow of gases may be a second laterally vented flow of gases.
- The first deflector may be configured to redirect a substantial amount of the first laterally vented flow of gases in an anterior direction in use.
- The second deflector may be configured to redirect a substantial amount of the second laterally vented flow of gases in an anterior direction in use.

In examples:
- The patient interface may comprise a spacer to provide a gap between a surface of the vent structure and a surface of the deflector.
- The spacer may comprise at least one rib, preferably a plurality of ribs.
- The ribs may provide a plurality of flow paths to direct the flow of gases exiting the vent structure.
- The spacer may comprise part of the vent structure.
- The spacer may comprise part of the deflector.
- The spacer may comprise at least one of the deflector walls.

In examples:
- The patient interface may comprise a pair of connectors to facilitate attachment of the plenum chamber to a positioning and stabilising structure.
- At least one of the connectors may be comprised as part of the vent structure.
- At least one of the connectors may be comprised as part of the deflector.

In examples:
- The patient interface may comprise a positioning and stabilising structure to provide a force to hold the seal-forming structure in a therapeutically effective position on the patient's head.
- The positioning and stabilising structure may comprise at least one headgear strap, preferably a plurality of headgear straps.
- The at least one headgear strap may be removably attached to the connector by buttoning.
- The at least one headgear strap may comprise a button-hole.
- The at least one headgear strap comprises a sleeve-like configuration to receive the insertion of a portion of the connector into the at least one headgear strap via the button-hole.

In examples:
- The position and stabilising structure may comprise a diffuser.
- The diffuser may be positioned in use to diffuse the flow of gases exiting the vent structure.
- The positioning and stabilising structure may comprise a component.
- The component may comprise a diffuser.
- The component may comprise a vent-facing surface.
- The vent-facing surface may be positioned in use in the path of the vent flow of gases exiting a vent structure.
- The diffuser may be located on the vent-facing surface of the component.
- The component may comprise a frame.
- The frame may be configured to facilitate indirect attachment of the plenum chamber to the at least one headgear strap.
- The component may comprise at least one tube.
- The at least one tube may be configured, in use, to convey the flow of air from an air circuit fluidly connected to the at least one tube to an interior of the plenum chamber through the inlet.
- The at least one tube may be a headgear tube.
- The component may comprise a rigidiser arm.
- The rigidiser arm may be configured to rigidise at least one headgear strap of the positioning and stabilising structure.
- The diffuser may be removably attached to the component.
- The diffuser may be removably attached to the frame.
- The diffuser may be removably attached to the at least one tube.
- The diffuser may be removably attached to the rigidiser arm.

In examples:
- The positioning and stabilising structure may comprise a frame.
- The frame may be configured to facilitate indirect attachment of the plenum chamber to the at least one headgear strap.
- The frame may be attached to the plenum chamber and the at least one headgear strap is attached to the frame.
- The air delivery tube may be attached to at least one of the frame and the plenum chamber.
- The air delivery tube may be attached to the frame.
- The frame may comprise an opening.
- The air delivery tube may be configured to be, in use, in fluid communication with the inlet through the opening of the frame.
- The frame may comprise a diffuser, wherein the diffuser is positioned in use to diffuse the flow of gases exiting the vent structure.
- A cover portion of the frame may cover at least a portion of the vent structure in use.
- The diffuser may be located on the cover portion of the frame.
- The diffuser may be removably attached to the frame.
- The frame may comprise at least one connector, preferably a pair of connectors, to facilitate attachment of the frame to the at least one headgear strap.
- The at least one headgear strap may be removably attached to the connector(s) of the frame.
- The frame may comprise the deflector.
- A part of the frame may be positioned in use to redirect the flow of gases exiting the vent structure.

In examples:
- The positioning and stabilising structure may comprise at least one tube.
- The at least one tube may be configured, in use, to convey the flow of air from an air circuit fluidly connected to the at least one tube to an interior of the plenum chamber through the inlet.
- The at least one tube may be a headgear tube.
- The at least one tube may comprise a diffuser, wherein the diffuser is positioned in use to diffuse the flow of gases exiting the vent structure.
- A cover portion of the at least one tube may cover at least a portion of the vent structure in use.
- The diffuser may be located on the cover portion of the at least one tube.
- The diffuser may be removably attached to the at least one tube.

In examples:
- The vent structure may be located on the positioning and stabilising structure.
- The positioning and stabilising structure may comprise a conduit portion.
- The plenum chamber may comprise an opening, wherein the conduit portion may be configured to be, in use, in fluid communication with an interior of the plenum chamber through the opening.
- The patient interface may comprise a connector configured to facilitate attachment of the conduit portion to the opening.
- The vent structure may be located on the conduit portion.
- The conduit portion may be a first conduit portion comprising the vent structure which is a first vent structure and the opening which is a first opening, wherein the first conduit portion is configured to be, in use, in fluid communication with an interior of the plenum chamber through the first opening, and wherein the positioning and stabilising structure may comprise a second conduit portion comprising a second vent structure and the plenum chamber comprises a second opening, wherein the second conduit portion is configured to be, in use, in fluid communication with an interior of the plenum chamber through the second opening.
- The connector may be a first connector configured to facilitate attachment of the first conduit portion to the first opening, and wherein the patient interface may comprise a second connector configured to facilitate attachment of the second conduit portion to the second opening.
- The conduit portion may be configured to be relatively rigid.
- The conduit portion may be configured to be relatively flexible.
- The conduit portion may comprise a support structure to limit and/or prevent occlusion of the conduit portion.
- The support structure may be provided by one or more of a reinforcing structure, a thickened region, and a stiffened region.
- The positioning and stabilising structure may comprise an inferior end which connects to the plenum chamber in use, and wherein the conduit portion comprises the inferior end.
- The vent structure may be located proximate to the inferior end of the positioning and stabilising structure in use.
- The plenum chamber may comprise a laterally projecting connection portion, wherein the opening is located on the laterally projecting connection portion.
- The laterally projecting connection portion may be a first laterally projecting connection portion comprising the first opening, and wherein the plenum chamber may comprise a second laterally projecting connection portion, wherein the second opening is located on the second laterally projecting connection portion.
- The at least one headgear strap may be connected to the conduit portion.
- At least a portion of the at least one headgear strap covers at least a portion of the conduit portion and at least a portion of the vent structure.
- The vent structure may be located lateral to the plenum chamber in use.
- The vent structure may be located proximate to the inferior end of the positioning and stabilising structure in use.
- The conduit portion may comprise the deflector.

In examples:
- The patient interface may comprise a diffuser, wherein the diffuser is positioned in use to diffuse the flow of gases exiting the vent structure.
- The diffuser may be configured to diffuse a flow of gases vented from an interior of the patient interface to ambient through the vent structure.
- The at least one headgear strap may comprise the diffuser.
- The at least one headgear strap may comprise a cover portion, wherein of the cover portion may be configured to cover at least a portion of the vent structure in use.
- The diffuser may be located on the cover portion of the at least one headgear strap.
- The diffuser may be removably attached to the at least one headgear strap.
- The diffuser may be formed from a textile material.
- The diffuser may be formed by napping a portion of a surface of the textile material.
- The diffuser may be formed separately and attached to the at least one headgear strap.
- The diffuser may be formed from a headgear strap material.
- The diffuser may be formed by napping a portion of a surface of the headgear strap material.
- The diffuser may form at least part of the deflector.

In examples:
- The patient interface may be in the form of a nasal mask.
- The seal-forming structure may be configured to form a seal in use with the underside of the nose around the nares.
- The seal-forming structure may be configured to form a seal around the patient's nares but not the patient's mouth in use.

In examples:
- The seal-forming structure may be configured to form a seal in use around the patient's nose and mouth.

One form of the present technology relates to a patient interface. The patient interface may comprise a plenum chamber pressurisable to a therapeutic pressure of at least 6 cmH2O above ambient air pressure. The plenum chamber may comprise an anterior portion comprising an inlet configured to receive a flow of air at the therapeutic pressure for breathing by a patient. The patient interface may further comprise a seal-forming structure configured to form a seal with a region of the patient's face surrounding an entrance to the patient's airways. The seal-forming structure may be configured to maintain said therapeutic pressure in the plenum chamber throughout the patient's respiratory cycle in use. The patient interface may further comprise a vent structure configured to allow a flow of gases exhaled by the patient from an interior of the plenum chamber to ambient. The vent structure may be configured to maintain the therapeutic pressure in the plenum chamber in use. The vent structure may be configured to vent the flow of gases from the interior of the plenum chamber in a substantially lateral direction in use. The patient interface may further comprise a deflector configured to redirect, in use, a substantial amount of the laterally vented flow of gases in a direction having an anterior component relative to the patient.

In an example the patient interface may comprise the patient interface according to any one or more of the previously described aspects and/or examples of the invention.
Another form of the present technology relates to a patient interface. The patient interface may comprise a plenum chamber pressurisable to a therapeutic pressure of at least 6 cmH2O above ambient air pressure. The plenum chamber may comprise an inlet configured to receive a flow of air at the therapeutic pressure for breathing by a patient. The patient interface may further comprise a seal-forming structure configured to form a seal with a region of the patient's face surrounding an entrance to the patient's airways. The seal-forming structure may be configured to maintain said therapeutic pressure in the plenum chamber throughout the patient's respiratory cycle in use. The patient interface may further comprise a vent structure configured to allow a flow of gases exhaled by the patient from an interior of the plenum chamber to ambient. The vent structure may be configured to maintain the therapeutic pressure in the plenum chamber in use. The patient interface may further comprise a positioning and stabilising structure to provide a force to hold the seal-forming structure in a therapeutically effective position on the patient's head. The positioning and stabilising structure may comprise at least one headgear strap, wherein the at least one headgear strap comprises a diffuser. The diffuser may be positioned in use to diffuse the flow of gases exiting the vent structure.

In an example the patient interface may comprise the patient interface according to any one or more of the previously described aspects and/or examples of the invention.

Another form of the present technology relates to a patient interface. The patient interface may comprise a plenum chamber pressurisable to a therapeutic pressure of at least 6 cmH2O above ambient air pressure. The plenum chamber may comprise an inlet configured to receive a flow of air at the therapeutic pressure for breathing by a patient. The patient interface may further comprise a seal-forming structure configured to form a seal with a region of the patient's face surrounding an entrance to the patient's airways. The seal-forming structure may be configured to maintain said therapeutic pressure in the plenum chamber throughout the patient's respiratory cycle in use. The patient interface may further comprise a vent structure configured to allow a vent flow of gases exhaled by the patient from an interior of the plenum chamber to ambient. The vent structure may be configured to maintain the therapeutic pressure in the plenum chamber in use. The patient interface may further comprise a positioning and stabilising structure to provide a force to hold the seal-forming structure in a therapeutically effective position on the patient's head. The positioning and stabilising structure may comprise a component. The component may comprise a vent-facing surface. The vent-facing surface may be positioned in use in the path of the vent flow of gases exiting the vent structure. The patient interface may further comprise a diffuser. The diffuser may be located on the vent-facing surface of the component. The diffuser may be positioned in use to diffuse the vent flow of gases exiting the vent structure.

In an example the patient interface may comprise the patient interface according to any one or more of the previously described aspects and/or examples of the invention.

Another form of the present technology relates to a positioning and stabilising structure for holding a patient interface in a therapeutically effective position on a patient's head. The positioning and stabilising structure may comprise a diffuser. The diffuser may be configured to diffuse a flow of gases vented from an interior of the patient interface to ambient through a vent structure configured on the patient interface.

In an example the positioning and stabilising structure may comprise the positioning and stabilising structure according to any one or more of the previously described aspects and/or examples of the invention.

Another form of the present technology relates to a positioning and stabilising structure for holding a patient interface in a therapeutically effective position on a patient's head. The positioning and stabilising structure may comprise a component. The component may comprise a vent-facing surface. The vent-facing surface may be positioned in use in the path of the vent flow of gases exiting a vent structure on the patient interface. The positioning and stabilising structure may further comprise a diffuser. The diffuser may be located on the vent-facing surface of the component. The diffuser may be configured to diffuse the vent flow of gases exiting the vent structure.

In an example the positioning and stabilising structure may comprise the positioning and stabilising structure according to any one or more of the previously described aspects and/or examples of the invention.

Another form of the present technology relates to a patient interface. The patient interface may comprise a plenum chamber pressurisable to a therapeutic pressure of at least 6 cmH2O above ambient air pressure. The plenum chamber may comprise an inlet configured to receive a flow of air at the therapeutic pressure for breathing by a patient. The patient interface may further comprise a seal-forming structure configured to form a seal with a region of the patient's face surrounding an entrance to the patient's airways. The seal-forming structure may be configured to maintain said therapeutic pressure in the plenum chamber throughout the patient's respiratory cycle in use. The patient interface may further comprise a positioning and stabilising structure for holding the seal-forming structure in a therapeutically effective position on a patient's head. The positioning and stabilising structure may comprise a conduit portion and the plenum chamber comprises an opening. The conduit portion may be configured to be, in use, in fluid communication with an interior of the plenum chamber through the opening. The conduit portion may comprise a vent structure configured to allow a flow of gases exhaled by the patient from the interior of the plenum chamber to ambient. The vent structure may be configured to maintain the therapeutic pressure in the patient interface in use.

In an example the patient interface may comprise the patient interface according to any one or more of the previously described aspects and/or examples of the invention.

Another form of the present technology relates to a positioning and stabilising structure for holding a patient interface in a therapeutically effective position on a patient's head. The positioning and stabilising structure may comprise a conduit portion configured to be in fluid communication with an interior of the patient interface in use. The conduit portion may comprise a vent structure configured to allow a flow of gases exhaled by the patient from the interior of the patient interface to ambient. The vent structure may be configured to maintain the therapeutic pressure in the interior of the patient interface in use.

In an example the positioning and stabilising structure may comprise the positioning and stabilising structure according to any one or more of the previously described aspects and/or examples of the invention.

Another form of the present technology relates to a patient interface. The patient interface may comprise a plenum chamber pressurisable to a therapeutic pressure of at least 6 cmH2O above ambient air pressure. The plenum chamber may comprise an inlet configured to receive a flow of air at the therapeutic pressure for breathing by a patient. The patient interface may further comprise a seal-forming structure configured to form a seal with a region of the patient's face surrounding an entrance to the patient's airways. The seal-forming structure may be configured to maintain said therapeutic pressure in the plenum chamber throughout the patient's respiratory cycle in use. The patient interface may further comprise a positioning and stabilising structure to provide a force to hold the seal-forming structure in a therapeutically effective position on the patient's head. The patient interface may further comprise a vent structure configured to allow a flow of gases exhaled by the patient from an interior of the plenum chamber to ambient. The vent structure may be configured to maintain the therapeutic pressure in the plenum chamber in use. The patient interface may further comprise a deflector configured to redirect a substantial amount of the vented flow of gases in use. The positioning and stabilising structure may comprise the deflector.

In an example the patient interface may comprise the patient interface according to any one or more of the previously described aspects and/or examples of the invention.

Another form of the present technology relates to a method of manufacturing a diffuser comprised as part of a headgear strap of a positioning and stabilising structure for holding a patient interface in a therapeutically effective position on a patient's head. The method may comprise the following steps, occurring in any order:
(a) forming a diffuser layer on a surface of a headgear strap material, wherein the diffuser layer is configured to, in use, diffuse a flow of gases vented through a vent structure configured on the patient interface; and
(b) forming the headgear strap from the headgear strap material.

In examples:
- Step (a) may comprise napping a portion of the surface of the headgear strap material.
- Step (a) may comprise trimming the napped portion of the surface of the headgear strap material.
- Step (a) may comprise forming a diffuser layer from a textile material and attaching the diffuser layer to the surface of the headgear strap material using an adhesive or other well-known method of attachment.
- The diffuser layer may be formed by cutting the textile material.

In examples:
- Step (b) may comprise cutting the headgear strap material into headgear straps.
- The headgear strap material may comprise a textile material.

Another form of the present technology relates to a method of manufacturing a diffuser comprised as part of at least one headgear tube of a positioning and stabilising structure for holding a patient interface in a therapeutically effective position on a patient's head. The method may comprise the following steps, occurring in any order:
(a) forming a diffuser layer on a surface of at least one headgear tube, wherein the diffuser layer is configured to, in use, diffuse a flow of gases vented through a vent structure configured on the patient interface; and
(b) forming the headgear tube.

In examples:
- Step (a) may comprise napping a portion of the surface of the headgear tube, wherein the headgear conduit is formed with a layer of textile material.
- Step (a) may comprise trimming the napped portion of the surface of the textile material.
- Step (a) may comprise forming a diffuser layer from a textile material and attaching the diffuser layer to the surface of the headgear conduit using an adhesive or other well-known method of attachment.
- The diffuser layer may be formed by cutting the textile material and napping the textile material, occurring in any order.

In examples:
- Step (b) may comprise forming the headgear tube 3350 from one or more materials, e.g. silicone.
- The textile material may comprise a fleece material.

Another form of the present technology relates to a method of manufacturing a diffuser comprised as part of a frame of a positioning and stabilising structure for holding a patient interface in a therapeutically effective position on a patient's head. The method may comprise the following steps, occurring in any order:
(a) forming a diffuser layer on a surface of the frame, wherein the diffuser layer is configured to, in use, diffuse a flow of gases vented through a vent structure configured on the patient interface; and
(b) forming the frame.

In examples:
- Step (a) may comprise napping a portion of the surface of the frame, wherein the frame is formed with a layer of textile material.
- Step (a) may comprise trimming the napped portion of the surface of the textile material.
- Step (a) may comprise forming a diffuser layer from a textile material and attaching the diffuser layer to the surface of the frame using an adhesive or other well-known method of attachment.
- The diffuser layer may be formed by cutting the textile material and napping the textile material, occurring in any order.

In examples:
- Step (b) may comprise forming the frame from one or more materials, e.g. polymers. Suitable polymers may include thermoplastics or elastomers, e.g. silicone.
- The textile material may comprise a fleece material.

Another aspect of one form of the present technology is a patient interface that is moulded or otherwise constructed with a perimeter shape which is complementary to that of an intended wearer.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 3 BRIEF DESCRIPTION OF THE DRAWINGS

The present technology is illustrated by way of example, and not by way of limitation, in the figures of the accompanying drawings, in which like reference numerals refer to similar elements including:

### 3.1 RESPIRATORY THERAPY SYSTEMS

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.
Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.
Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 3.2 RESPIRATORY SYSTEM AND FACIAL ANATOMY

Fig. 2A shows an overview of a human respiratory system including the nasal and oral cavities, the larynx, vocal folds, oesophagus, trachea, bronchus, lung, alveolar sacs, heart and diaphragm.
Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.
Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.
Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.
Fig. 2E is a further side view of a head. The approximate locations of the Frankfort horizontal and nasolabial angle are indicated. The coronal plane is also indicated.
Fig. 2F shows a base view of a nose with several features identified including naso-labial sulcus, lip inferior, upper Vermilion, naris, subnasale, columella, pronasale, the major axis of a naris and the mid-sagittal plane.
Fig. 2G shows a side view of the superficial features of a nose.
Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 2I shows a medial dissection of a nose, approximately several millimeters from the mid-sagittal plane, amongst other things showing the septum cartilage and medial crus of greater alar cartilage.
Fig. 2J shows a front view of the bones of a skull including the frontal, nasal and zygomatic bones. Nasal concha are indicated, as are the maxilla, and mandible.
Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.
Fig. 2L shows an anterolateral view of a nose.

### 3.3 PATIENT INTERFACE

Fig. 3A shows a patient interface in the form of a nasal mask in accordance with one form of the present technology.
Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
Fig. 3D shows a schematic of a cross-section through a structure at a point. An outward normal at the point is indicated. The curvature at the point has a value of zero.
Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
Fig. 3G shows a cushion for a mask that includes two pillows. An exterior surface of the cushion is indicated. An edge of the surface is indicated. Dome and saddle regions are indicated.
Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
Fig. 3I shows the surface of a structure, with a one dimensional hole in the surface. The illustrated plane curve forms the boundary of a one dimensional hole.
Fig. 3J shows a cross-section through the structure of Fig.3I. The illustrated surface bounds a two dimensional hole in the structure of Fig. 3I.
Fig. 3K shows a perspective view of the structure of Fig. 3I, including the two dimensional hole and the one dimensional hole. Also shown is the surface that bounds a two dimensional hole in the structure of Fig. 3I.
Fig. 3L shows a mask having an inflatable bladder as a cushion.
Fig. 3M shows a cross-section through the mask of Fig. 3L, and shows the interior surface of the bladder. The interior surface bounds the two dimensional hole in the mask.
Fig. 3N shows a further cross-section through the mask of Fig. 3L. The interior surface is also indicated.
Fig. 3O illustrates a left-hand rule.
Fig. 3P illustrates a right-hand rule.
Fig. 3Q shows a left ear, including the left ear helix.
Fig. 3R shows a right ear, including the right ear helix.
Fig. 3S shows a right-hand helix.
Fig. 3T shows a view of a mask, including the sign of the torsion of the space curve defined by the edge of the sealing membrane in different regions of the mask.
Fig. 3U shows a view of a plenum chamber 3200 showing a sagittal plane and a mid-contact plane.
Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

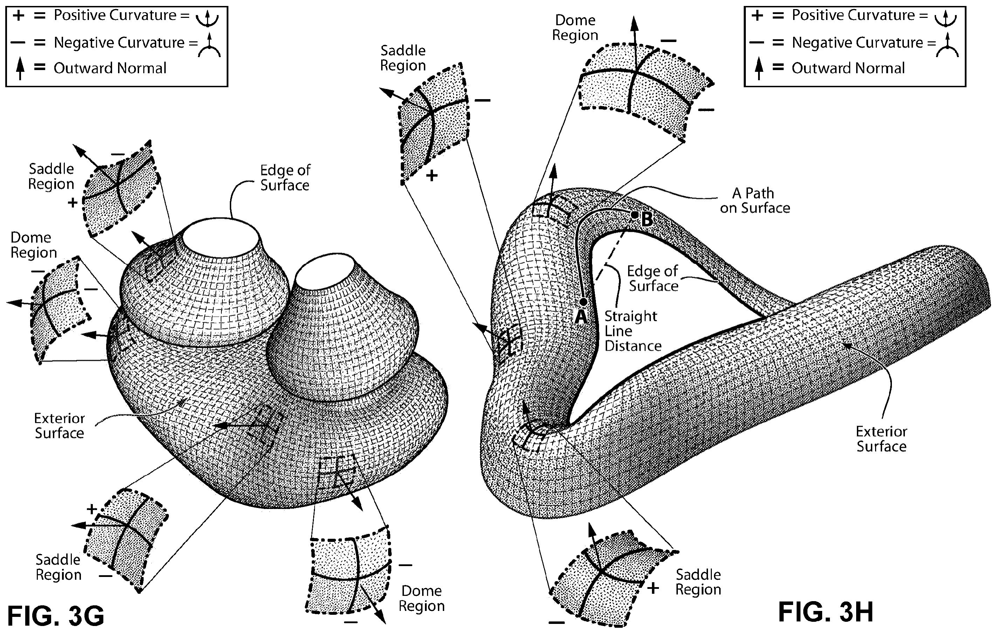

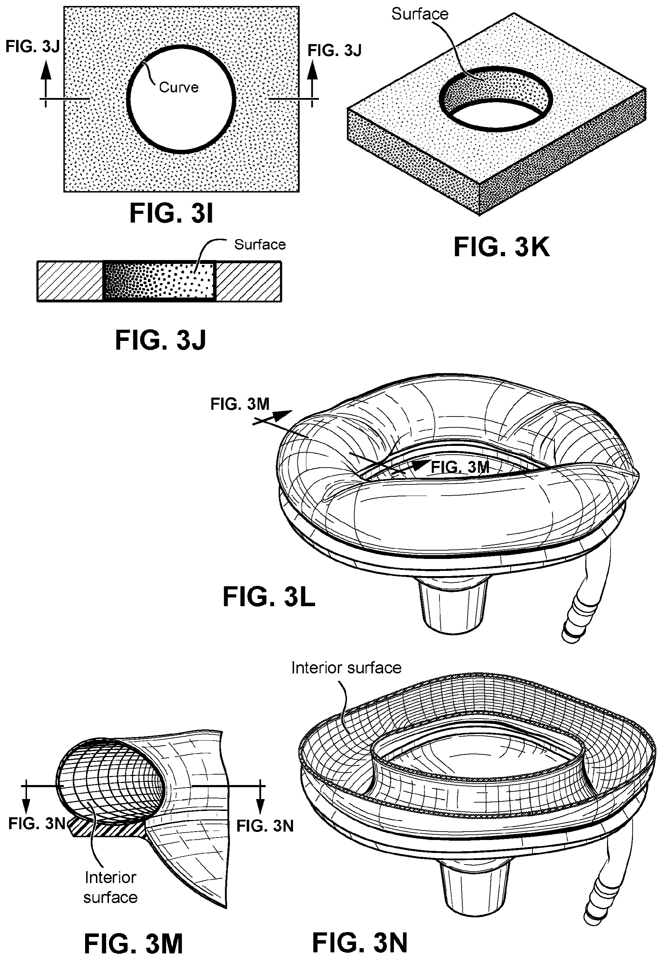

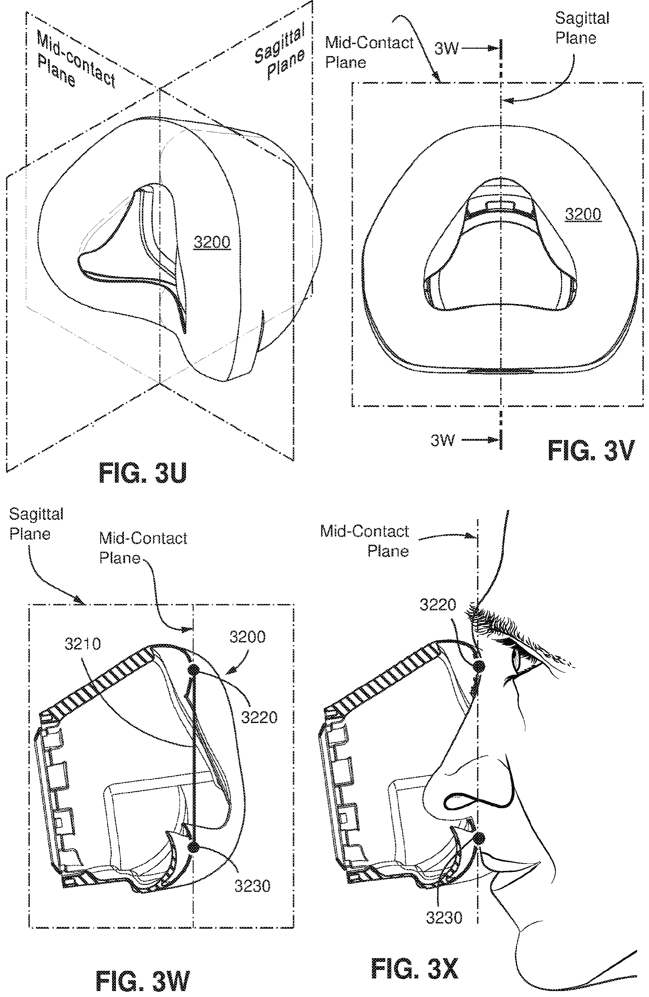

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.
Fig. 3Y shows a patient interface having conduit headgear, in accordance with one form of the present technology.

### 3.4 RPT DEVICE

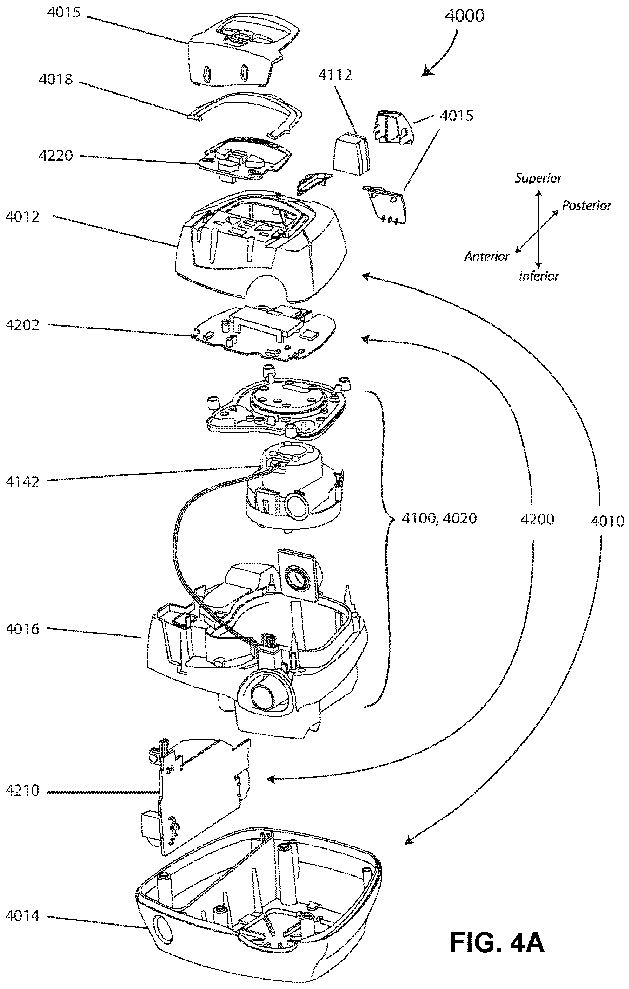

Fig. 4A shows an RPT device in accordance with one form of the present technology.

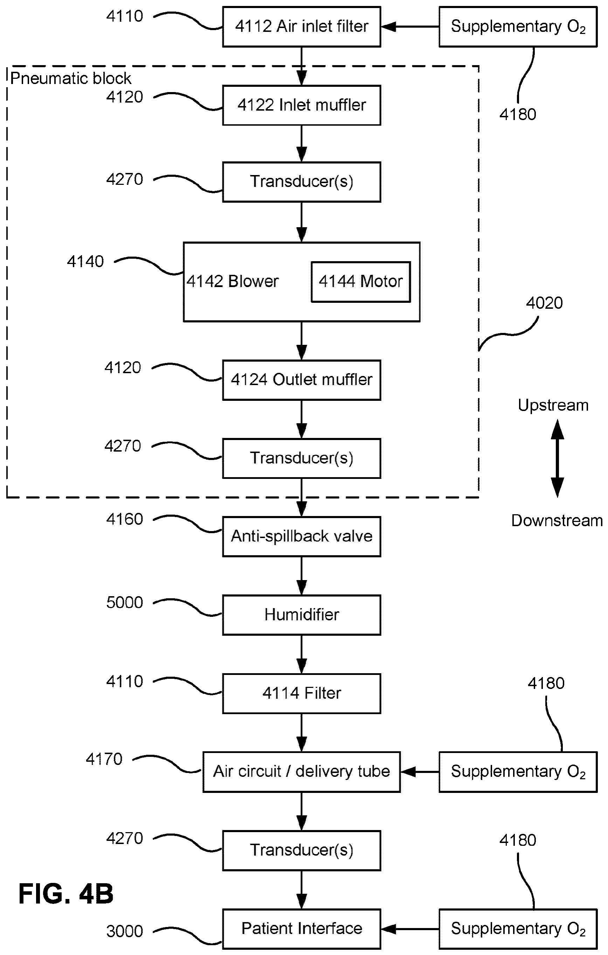

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 3.5 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 3.6 FURTHER FORMS OF PATIENT INTERFACE

Fig. 6 shows a cross-sectional view through a first section of a prior art patient interface.
Fig. 6A shows a perspective view of a patient interface in use, in accordance with one form of the present technology.
Fig. 6B shows a close-up perspective view of the patient interface of Fig. 6A.
Fig. 6C shows a cross-sectional view through a first section of the patient interface of Fig. 6A.

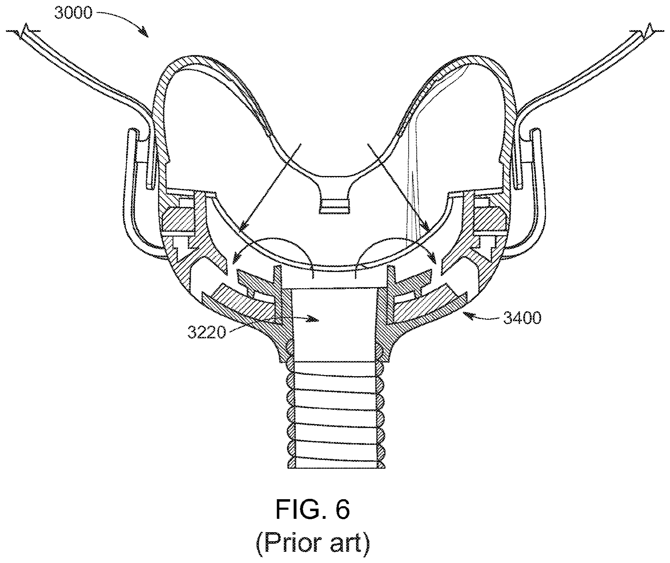

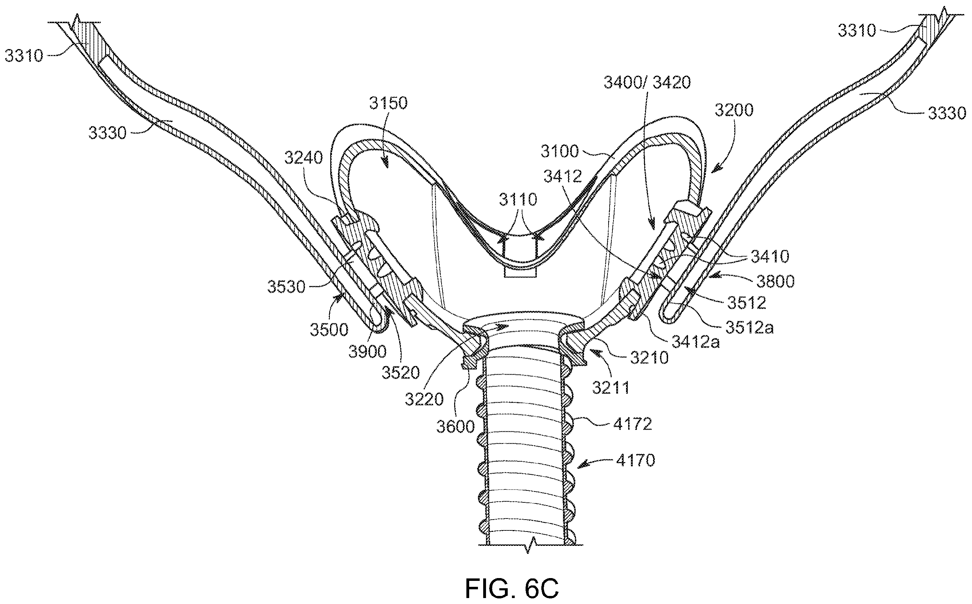

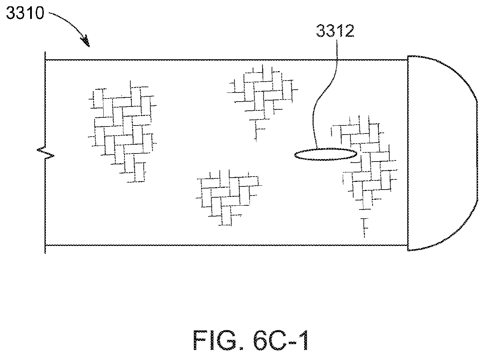

Fig. 6C-1 shows a planar view of an exemplary headgear strap comprising a button-hole in accordance with one form of the present technology.

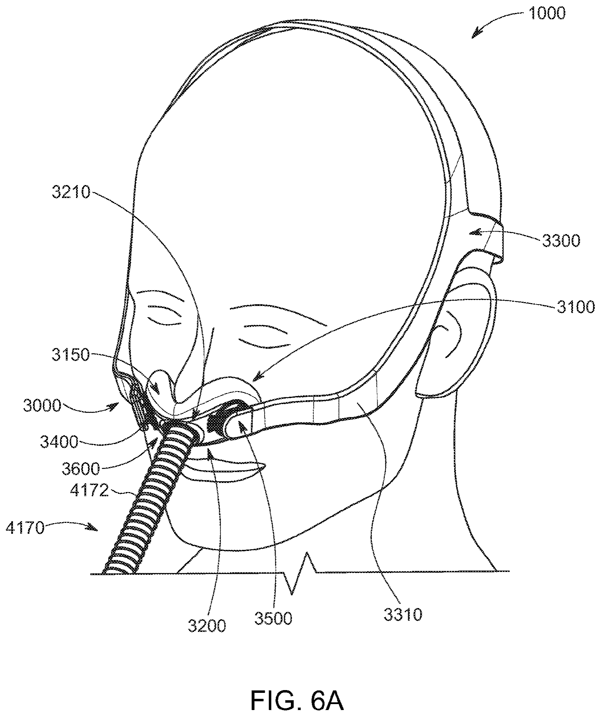

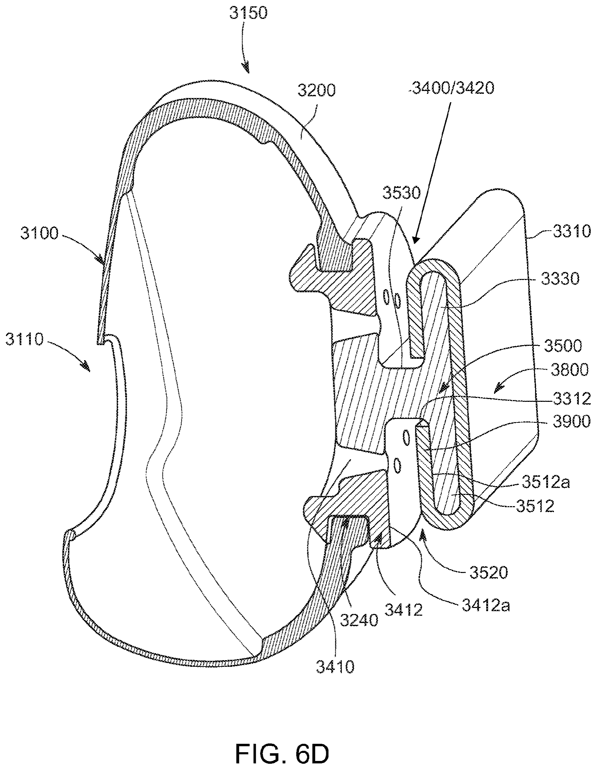

Fig. 6D shows a cross-sectional view through a second section of the patient interface of Fig. 6A.

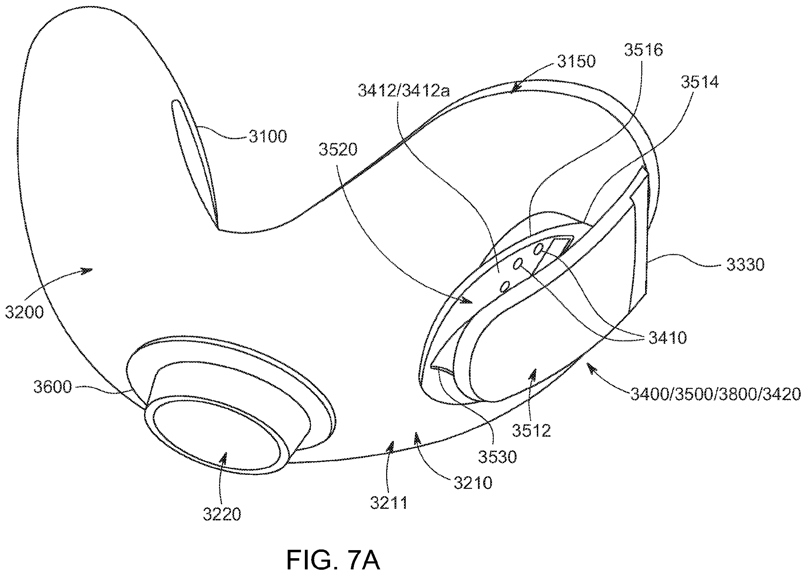

Fig. 7A shows a perspective view of a patient interface in accordance with another form of the present technology, showing the patient interface without a positioning and stabilising structure connected to it.

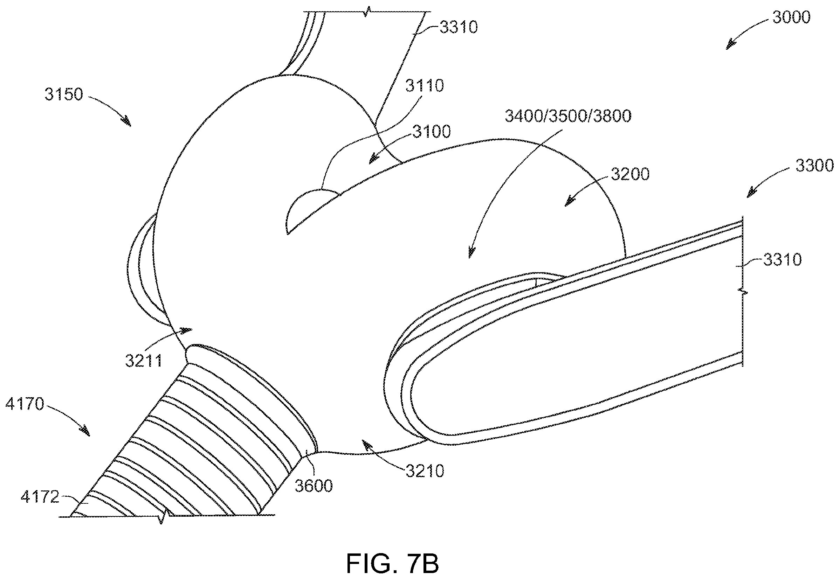

Fig. 7B shows a perspective view of the patient interface of Fig. 7A showing part of a positioning and stabilising structure connected to it.

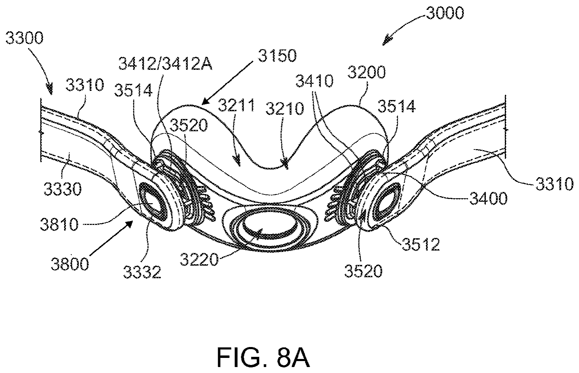

Fig. 8A shows a perspective view of a patient interface in accordance with another form of the present technology.

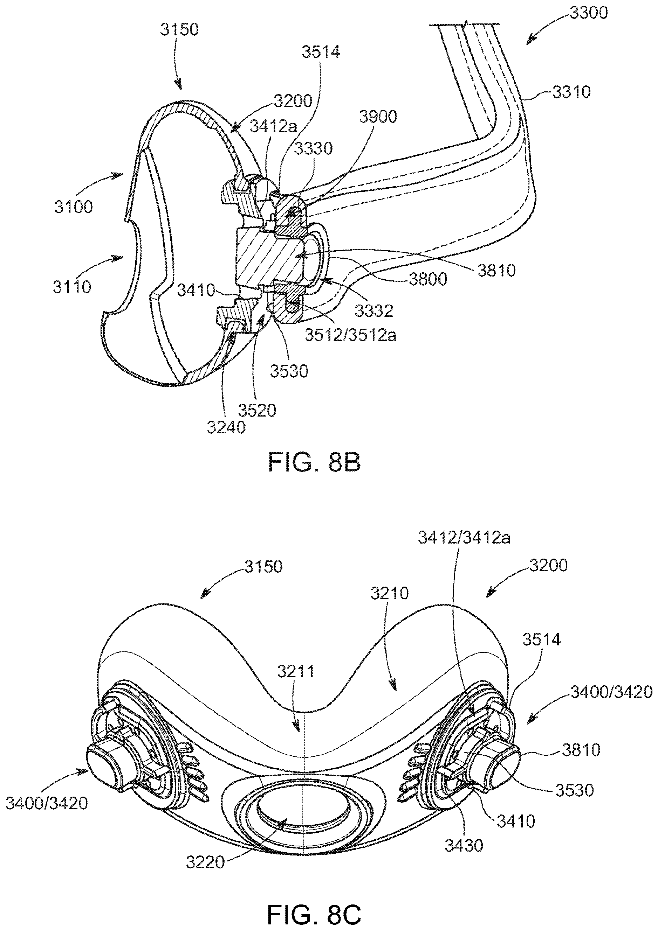

Fig. 8B shows a cross-sectional view through a first section of the patient interface of Fig. 8A.
Fig. 8C shows a perspective view of the patient interface of Fig. 8A without a positioning and stabilising structure connected to it.

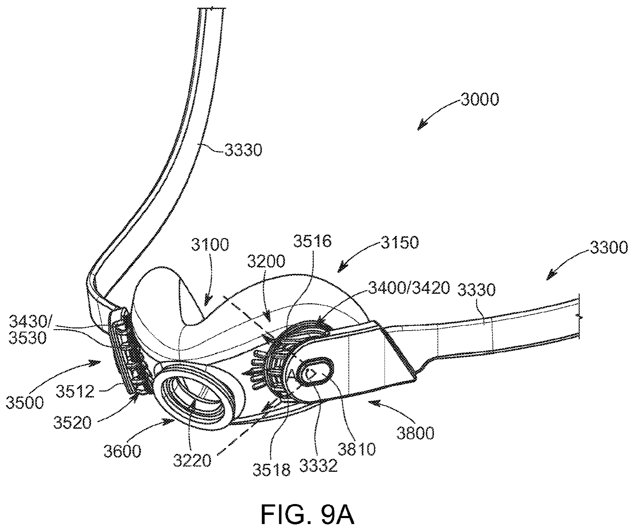

Fig. 9A shows a perspective view of a patient interface in accordance with another form of the present technology.

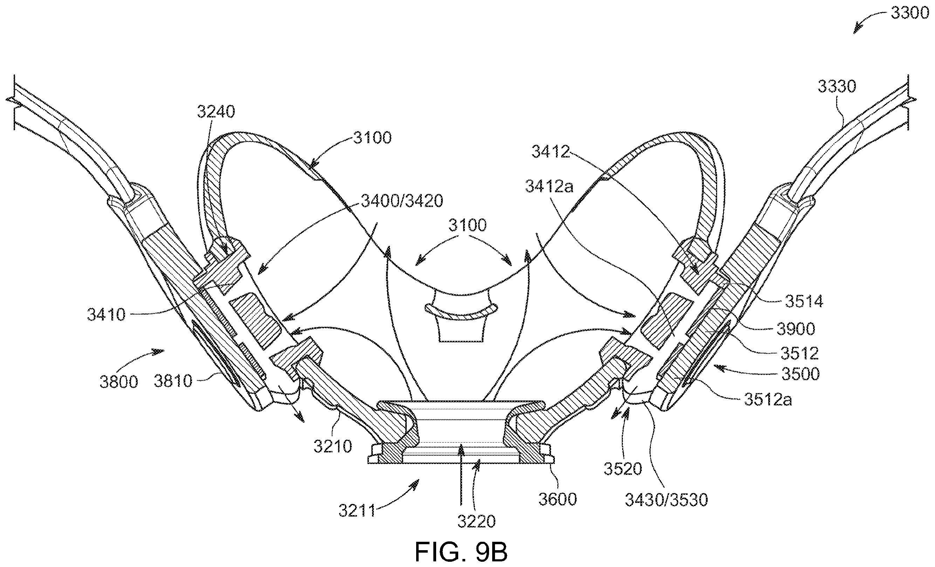

Fig. 9B shows a cross-sectional view through a first section of the patient interface of Fig. 9A.

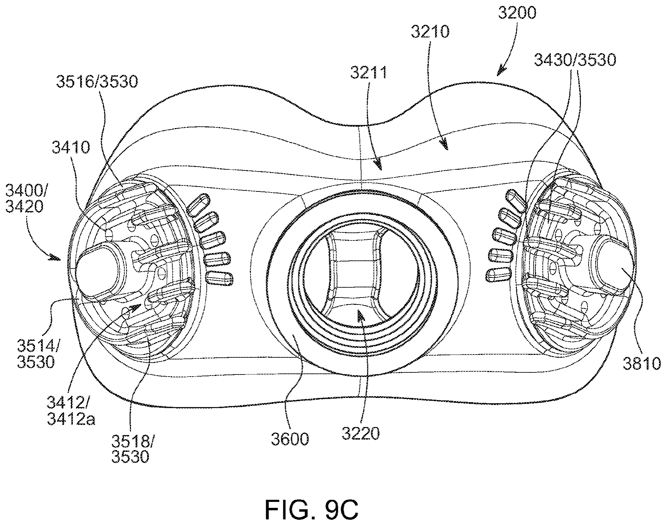

Fig. 9C shows a perspective view of the patient interface of Fig. 9A without a positioning and stabilising structure connected to it.

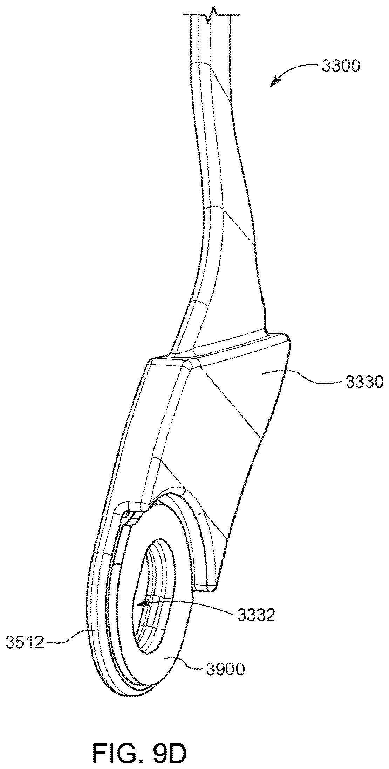

Fig. 9D shows a perspective view of part of a positioning and stabilising structure for use with the patient interface of Fig. 9A.

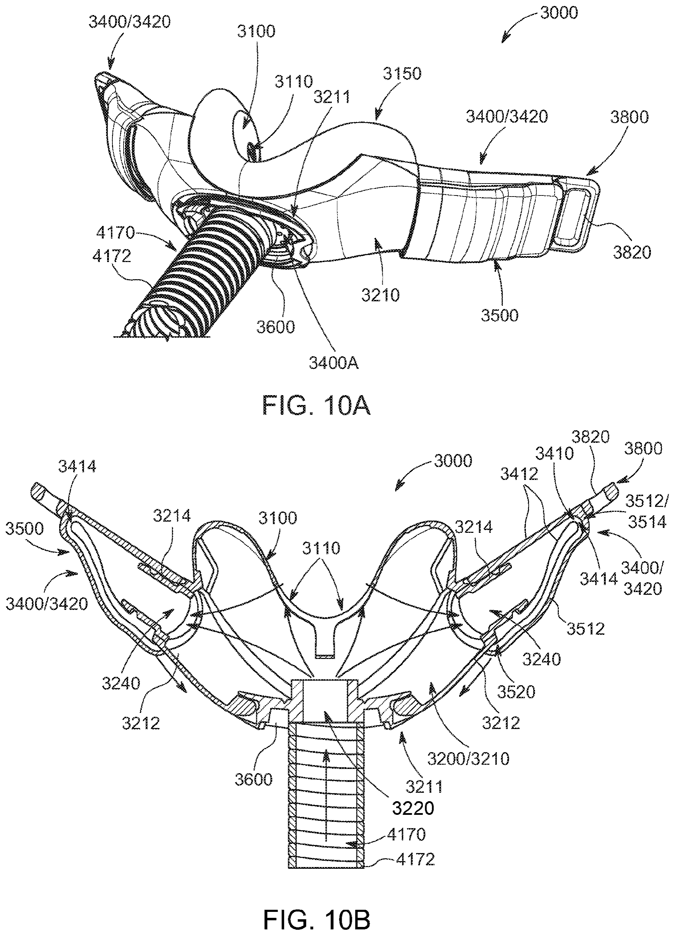

Fig. 10A shows a perspective view of a patient interface in accordance with another form of the present technology.
Fig. 10B shows a cross-sectional view through a first section of the patient interface of Fig. 10A.

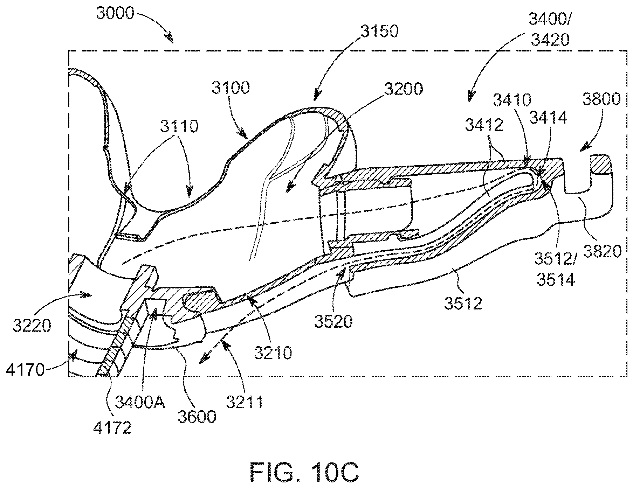

Fig. 10C shows an enlarged cross-sectional view through the first section of the patient interface of Fig. 10A.

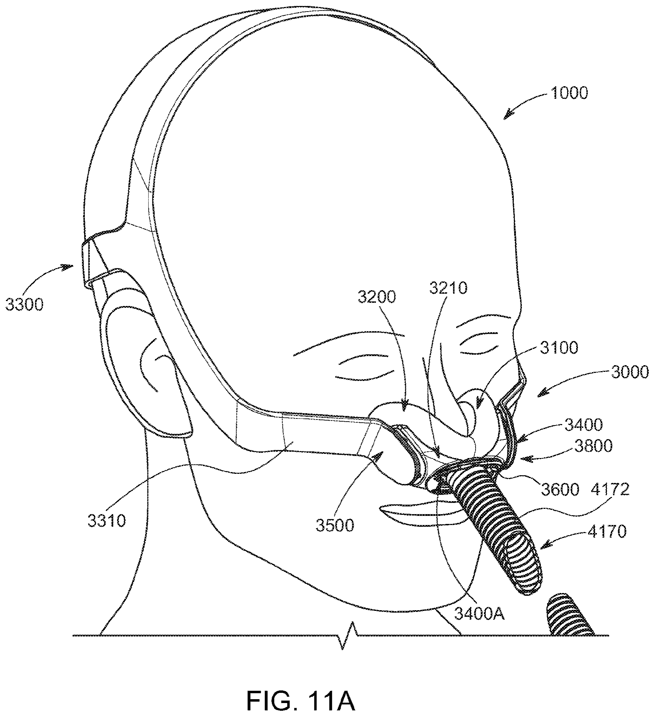

Fig. 11A shows a perspective view of a patient interface in use, in accordance with another form of the present technology.

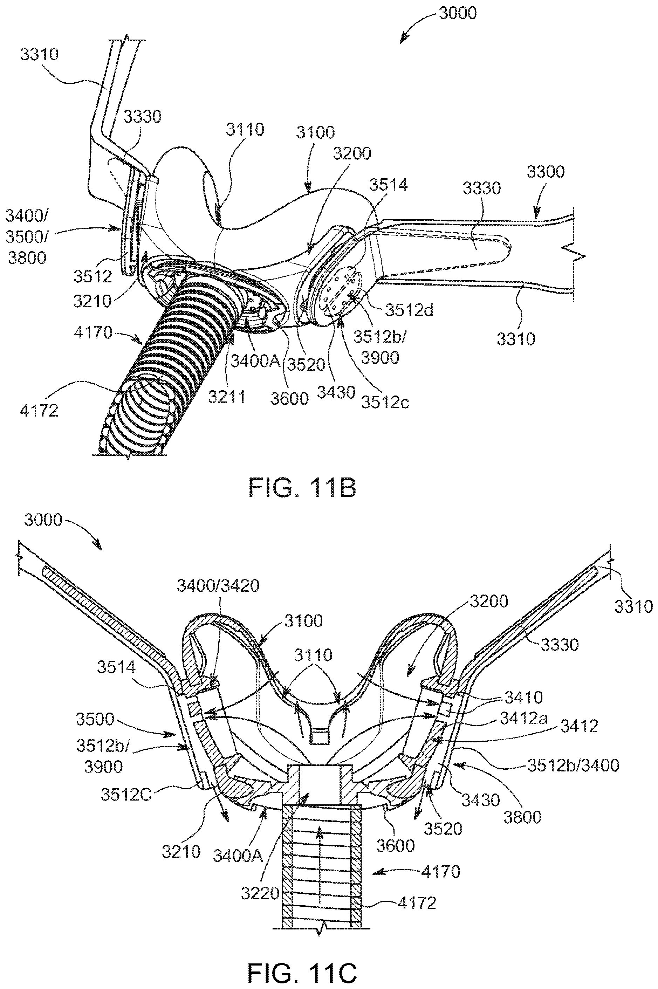

Fig. 11B shows a perspective view of the patient interface of Fig. 11A.
Fig. 11C shows a cross-sectional view through a first section of the patient interface of Fig. 11A.

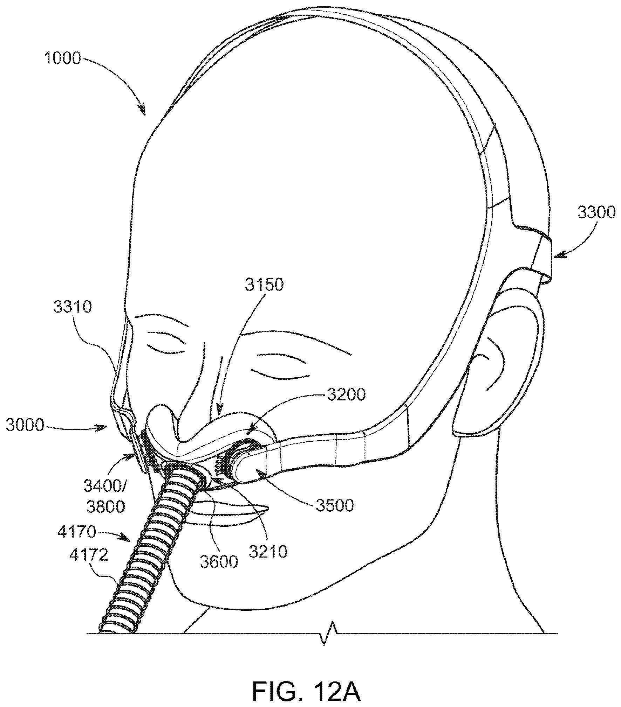

Fig. 12A shows a perspective view of a patient interface in use, in accordance with another form of the present technology.

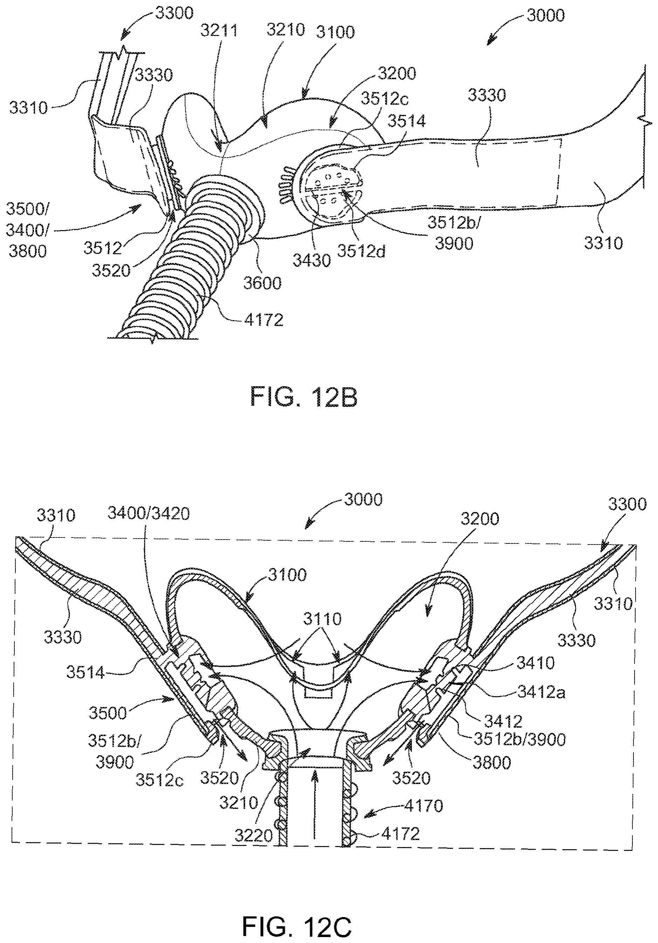

Fig. 12B shows a perspective view of the patient interface of Fig. 12A.
Fig. 12C shows a cross-sectional view through a first section of the patient interface of Fig. 12A.

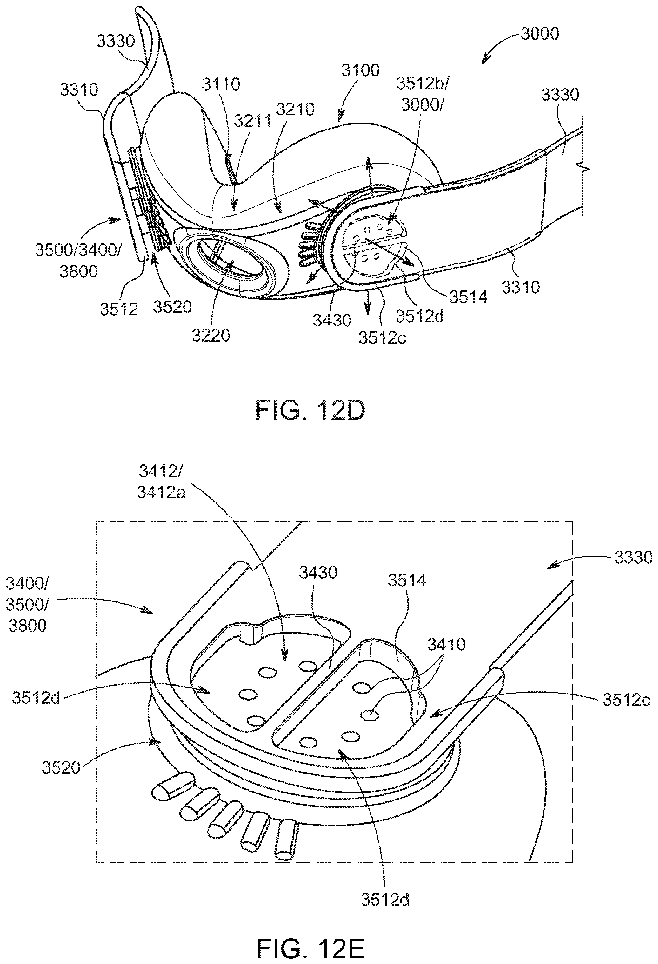

Fig. 12D shows a perspective view of the patient interface of Fig. 12A without an air circuit connected to it.
Fig. 12E shows an enlarged perspective view of part of the patient interface of Fig. 12A without headgear strap material attached to the vent module.

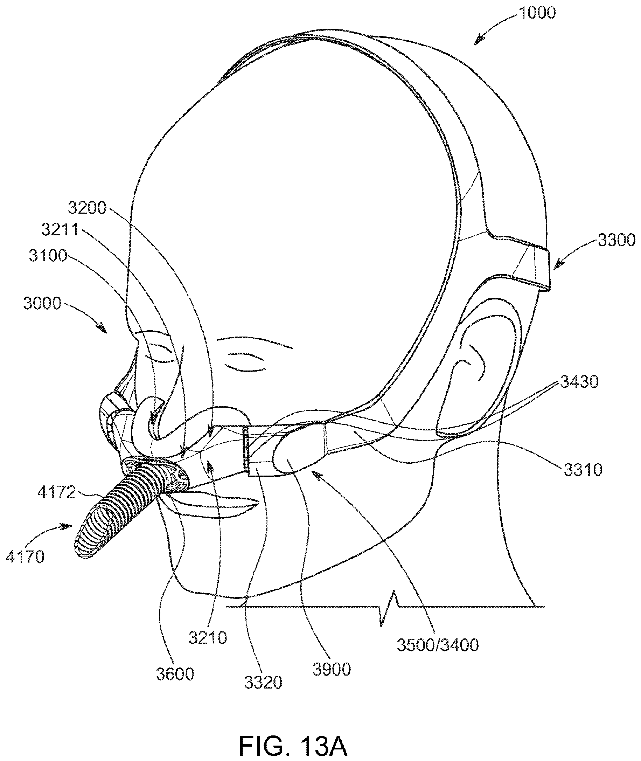

Fig. 13A shows a perspective view of a patient interface in use, in accordance with another form of the present technology.

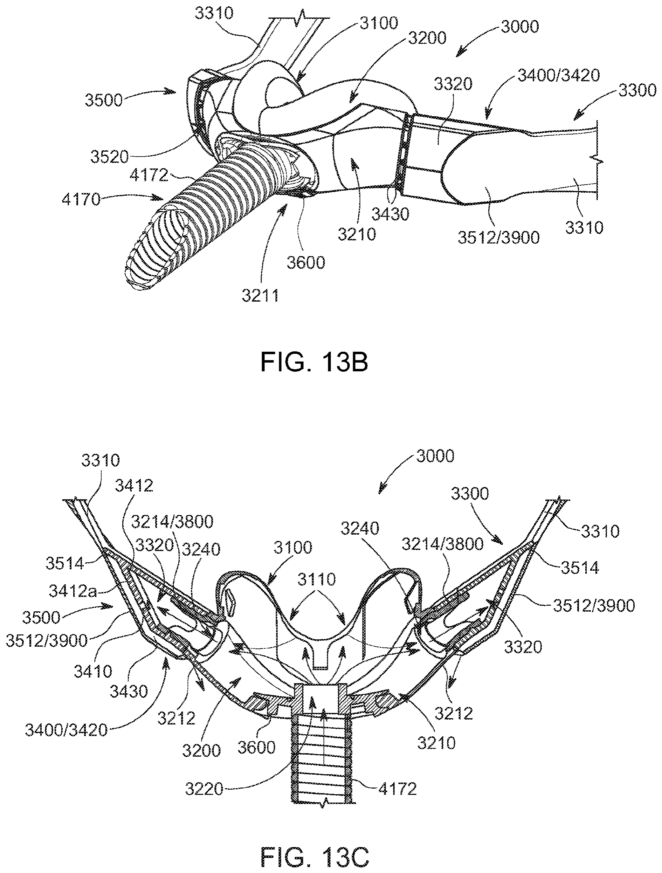

Fig. 13B shows a perspective view of the patient interface of Fig. 13A.
Fig. 13C shows a cross-sectional view through a first section of the patient interface of Fig. 13A.

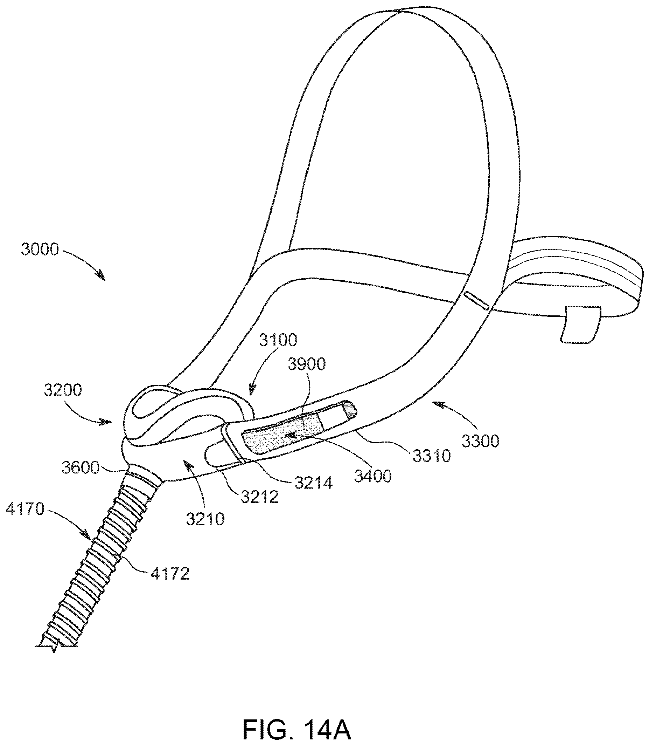

Fig. 14A shows a perspective view of a patient interface in use, in accordance with another form of the present technology.

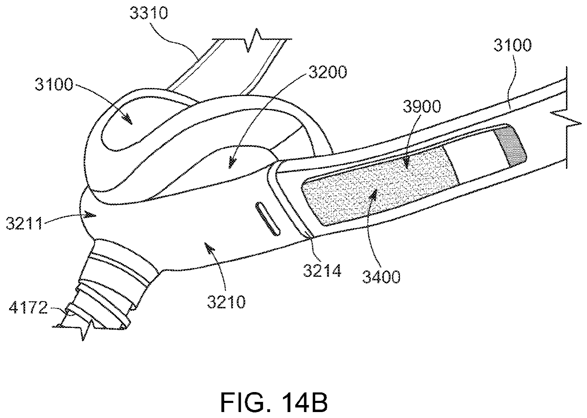

Fig. 14B shows an enlarged perspective view of the patient interface of Fig. 14A.

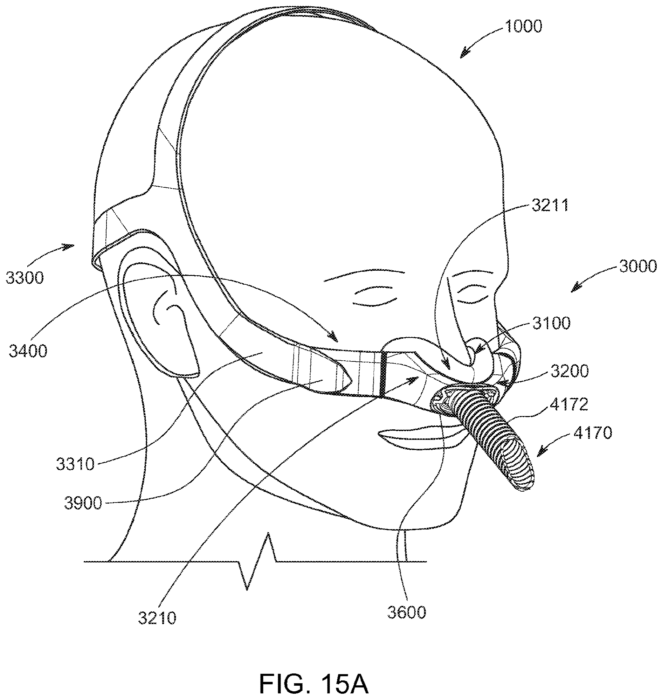

Fig. 15A shows a perspective view of a patient interface in use, in accordance with another form of the present technology.

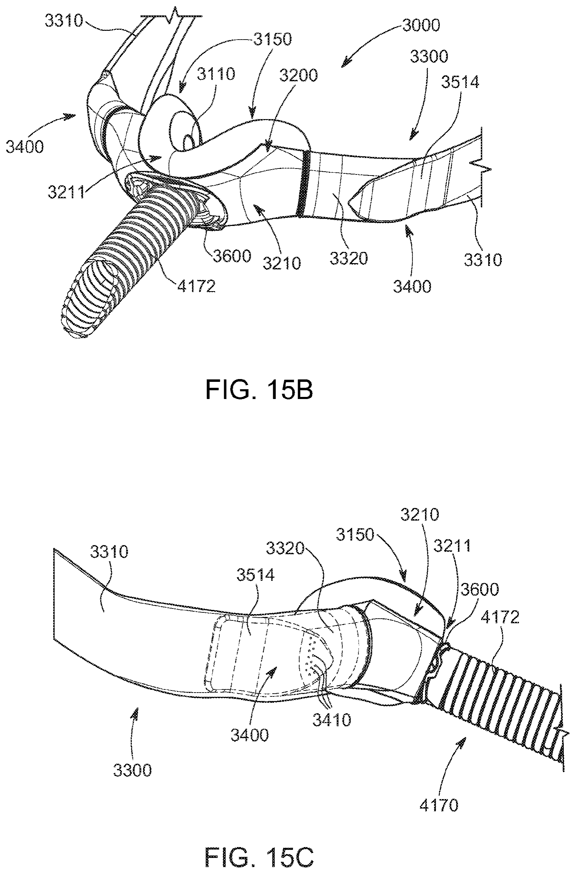

Fig. 15B shows a perspective view of the patient interface of Fig. 15A.
Fig. 15C shows a side view of the patient interface of Fig. 15A.

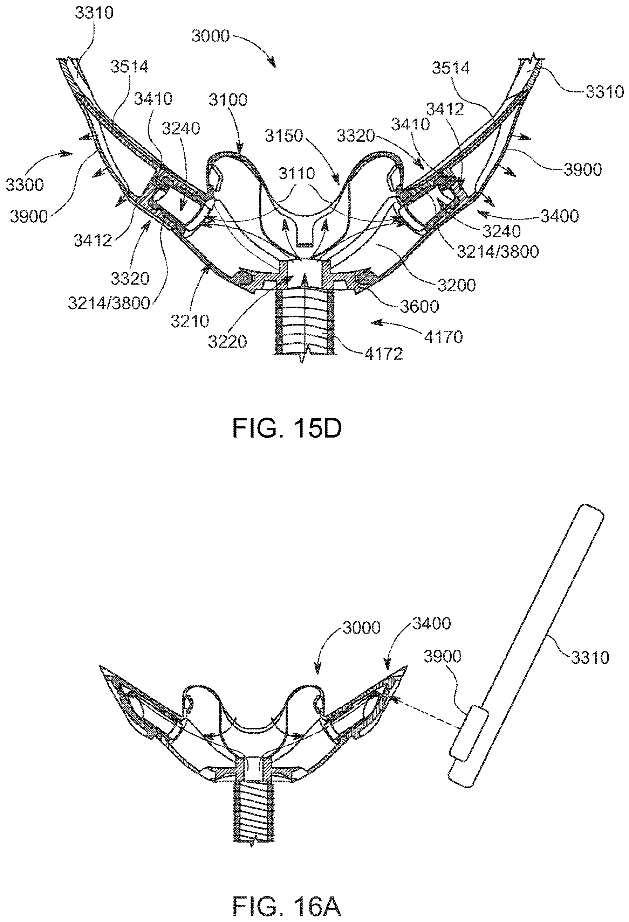

Fig. 15D shows a cross-sectional view through a first section of the patient interface of Fig. 15A.
Fig. 16A shows a diffuser comprised as part of the positioning and stabilising structure which is provided to a patient interface, in accordance with a form of the present technology.

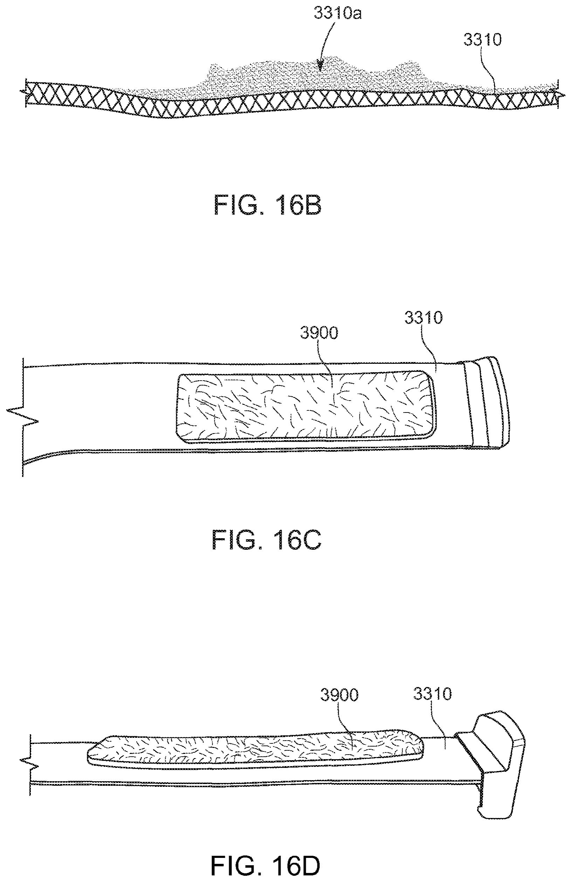

Fig. 16B shows a side view of a part of a headgear strap comprising a diffuser in accordance with one form of the present technology.
Fig. 16C shows a top view of a part of a headgear strap comprising a diffuser in accordance with another form of the present technology.
Fig. 16D shows a side perspective view of the headgear strap of Fig. 16C.

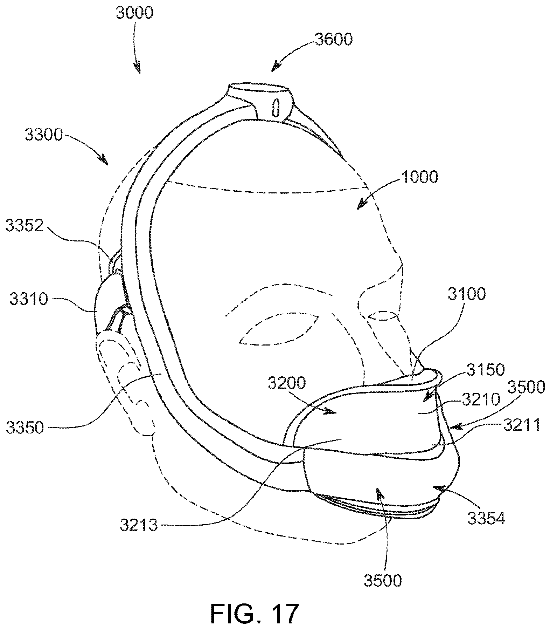

Fig. 17 shows a perspective view of a patient interface in use, in accordance with another form of the present technology.

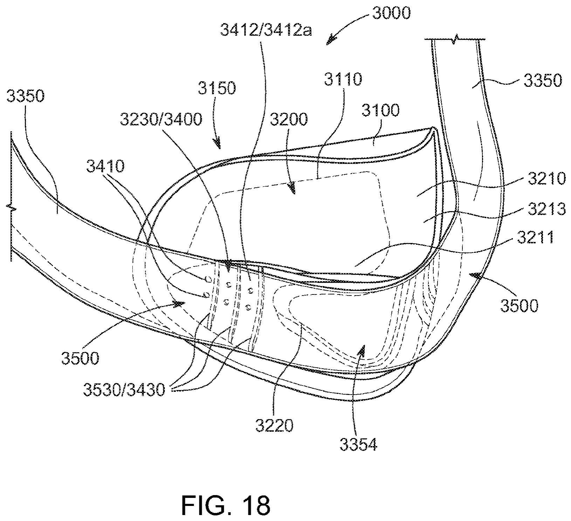

Fig. 18 shows a close-up part cross-sectional perspective view of the patient interface of Fig. 17.

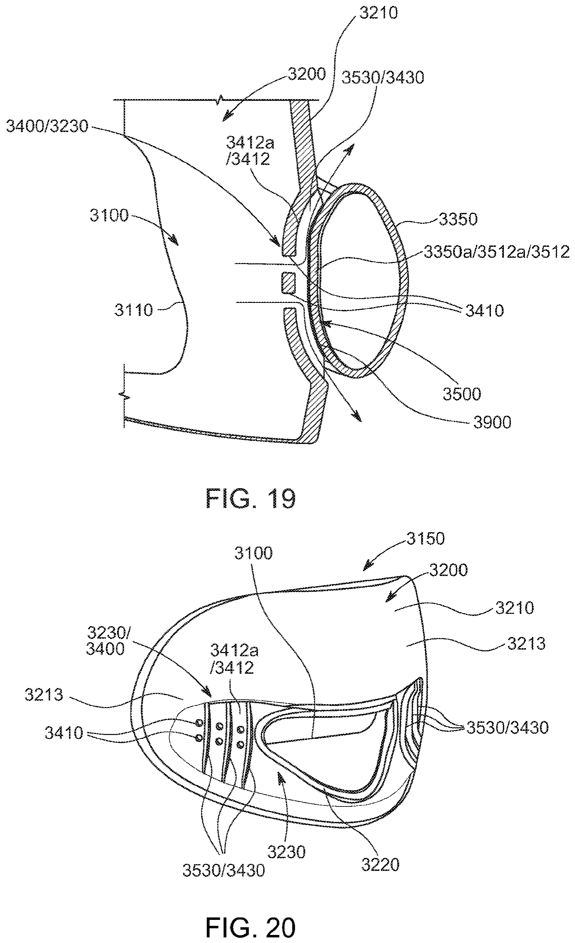

Fig. 19 shows an enlarged cross-sectional view through a first section of the patient interface of Fig. 17.
Fig. 20 shows a perspective view of the patient interface of Fig. 17, showing the patient interface without a positioning and stabilising structure connected to it.

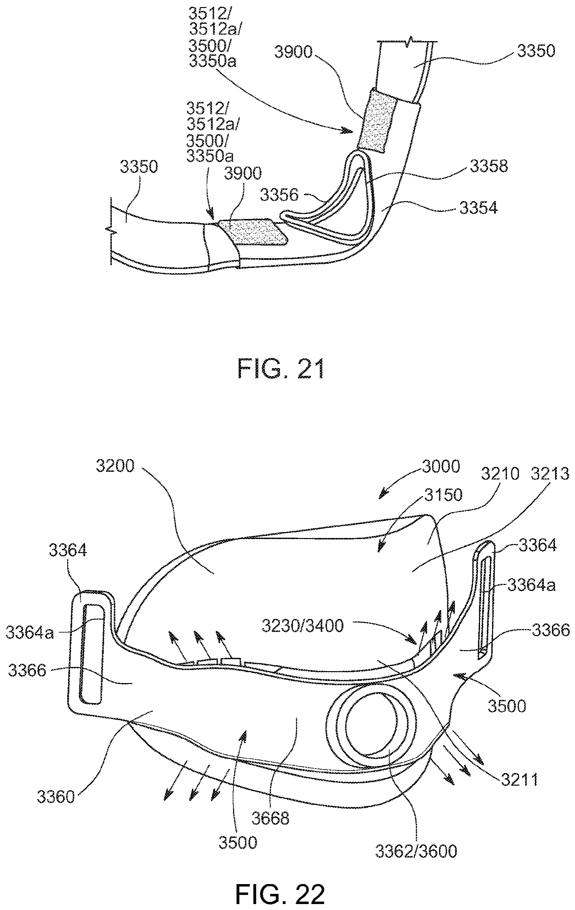

Fig. 21 shows an enlarged perspective view of a positioning and stabilising structure of the patient interface of Fig. 17.
Fig. 22 shows a perspective view of a patient interface, in accordance with another form of the present technology.

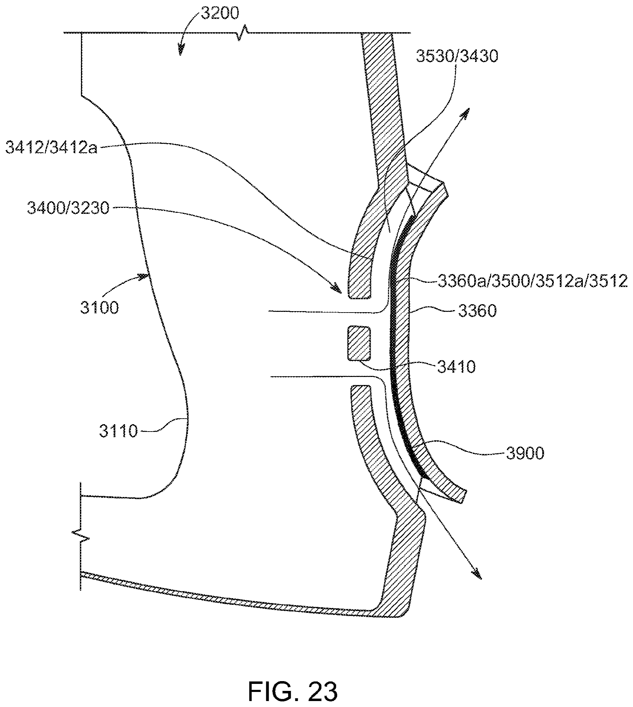

Fig. 23 shows an enlarged cross-sectional view through a first section of the patient interface of Fig. 22.

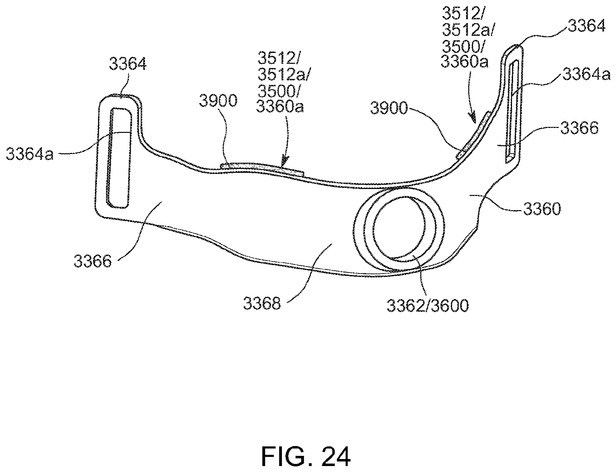

Fig. 24 shows an enlarged perspective view of a positioning and stabilising structure of the patient interface of Fig. 22.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 4.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 4.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000.

### 4.3 PATIENT INTERFACE

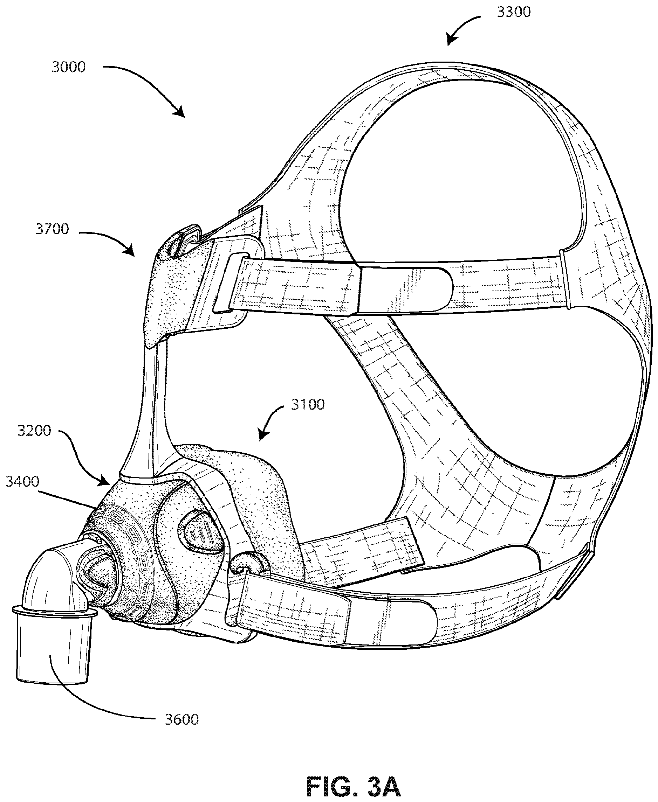

With reference to Fig. 3A, a non-invasive patient interface 3000 in accordance with one aspect of the present technology comprises the following functional aspects: a seal-forming structure 3100, a plenum chamber 3200, a positioning and stabilising structure 3300, a vent structure 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

With reference to Figs. 6A to 24, a patient interface 3000 in accordance with one aspect of the present technology comprises a seal-forming structure 3100, a plenum chamber 3200, a connection port 3600 for connection to an air circuit 4170, and a vent structure 3400, or pair of vent structures 3400, configured to vent a flow of gases from the interior of the plenum chamber 3200. In the illustrated forms, the vent structure 3400, or pair of vent structures 3400, is/are configured to vent the flow of gases in a substantially lateral direction in use.

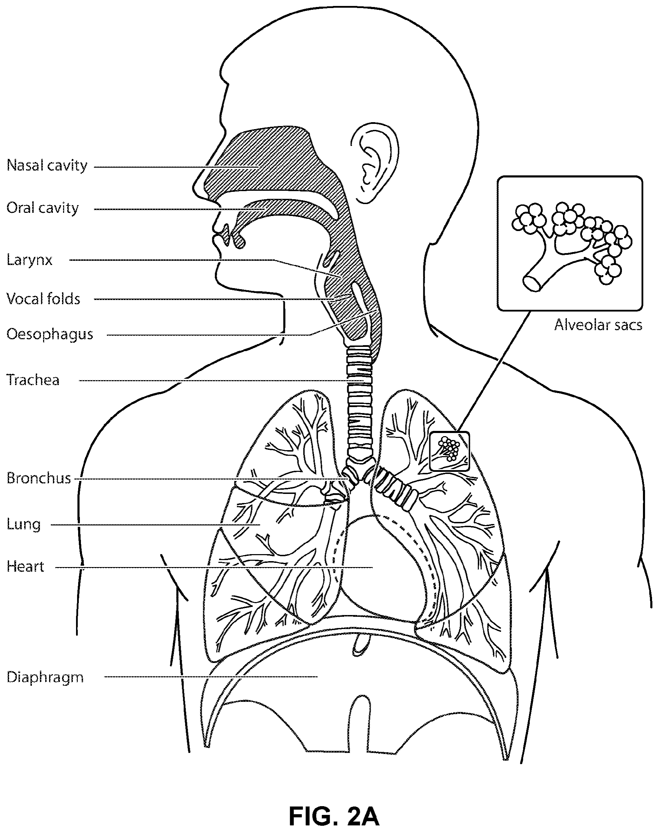

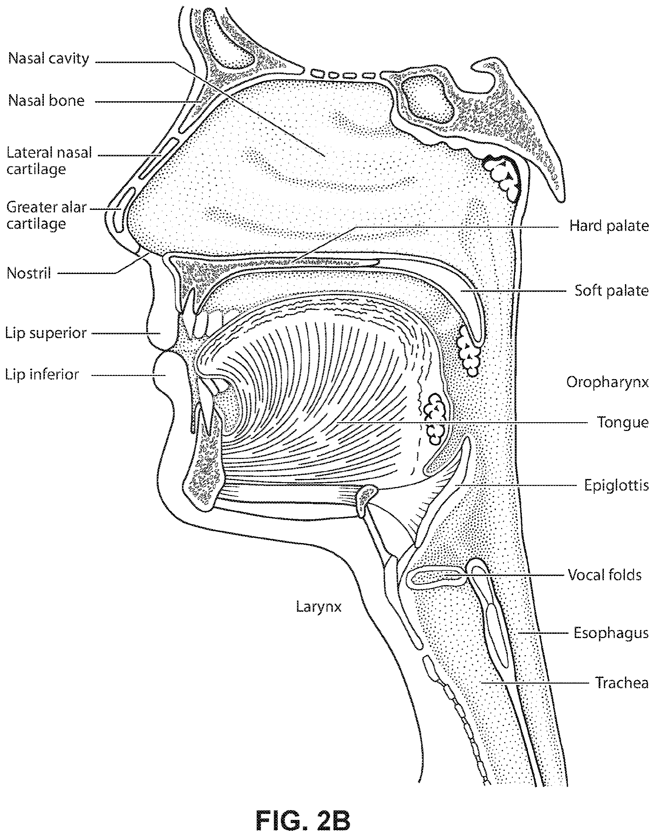

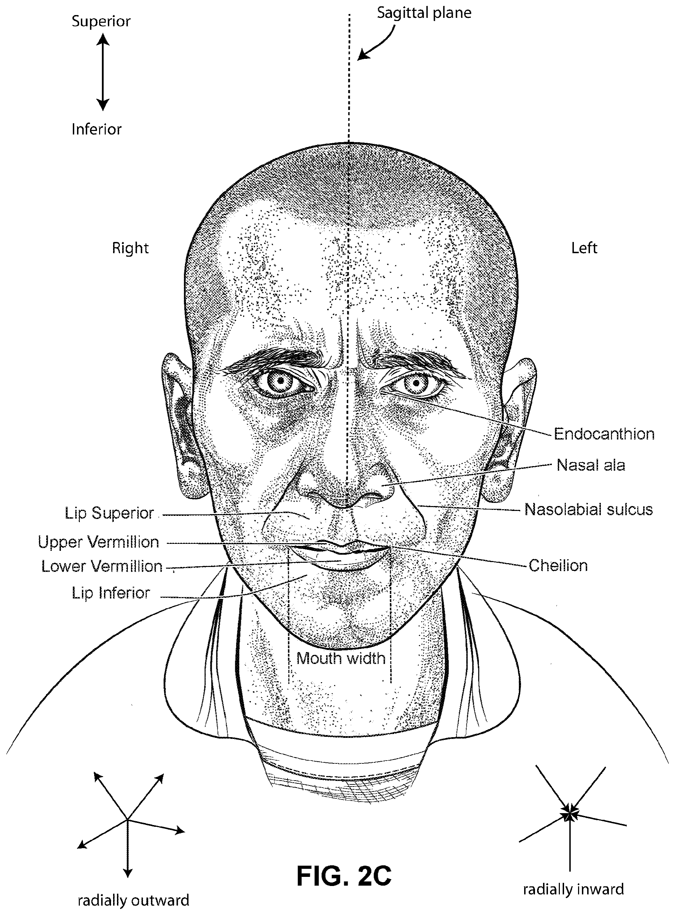

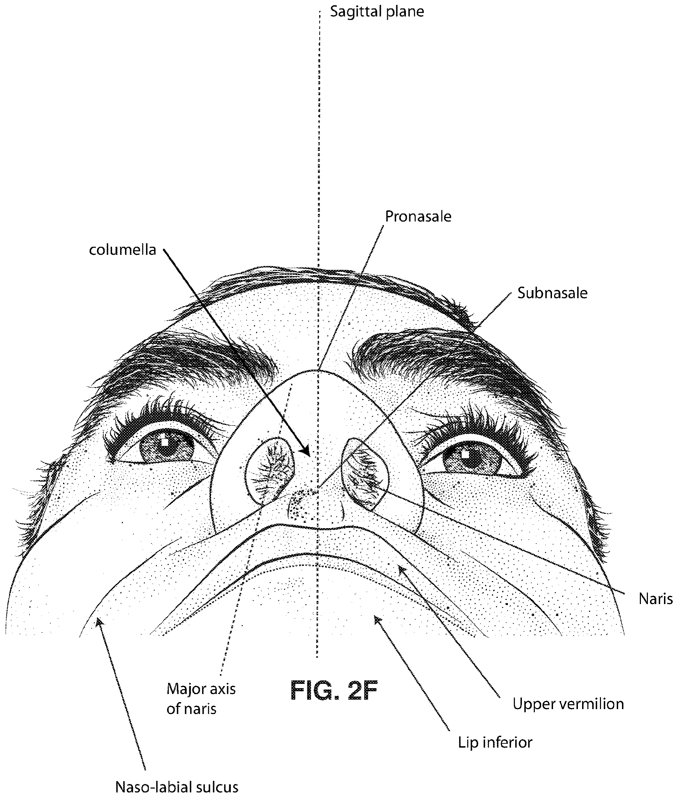

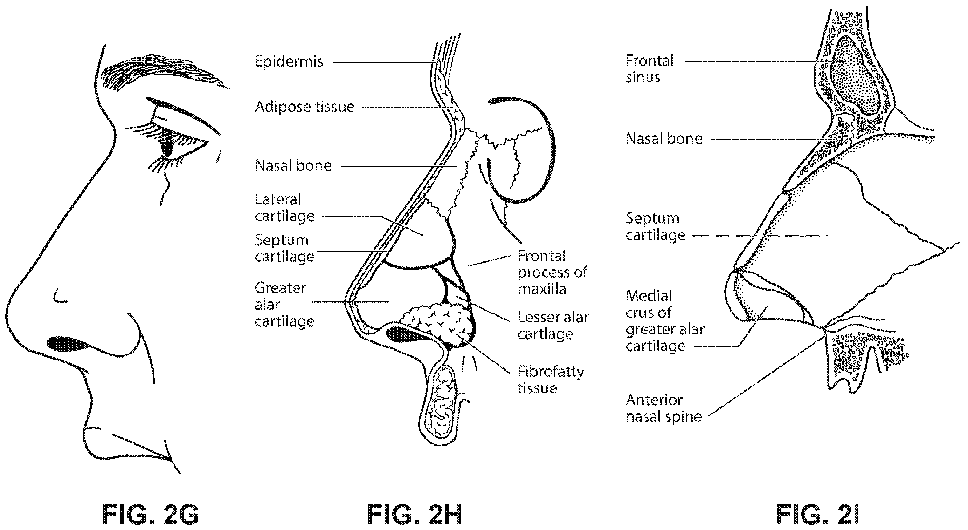

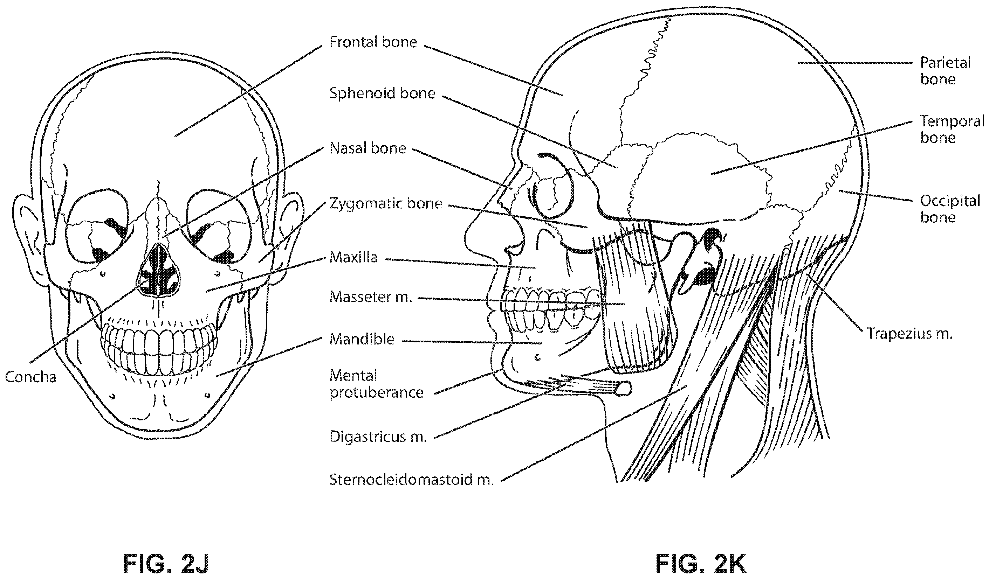

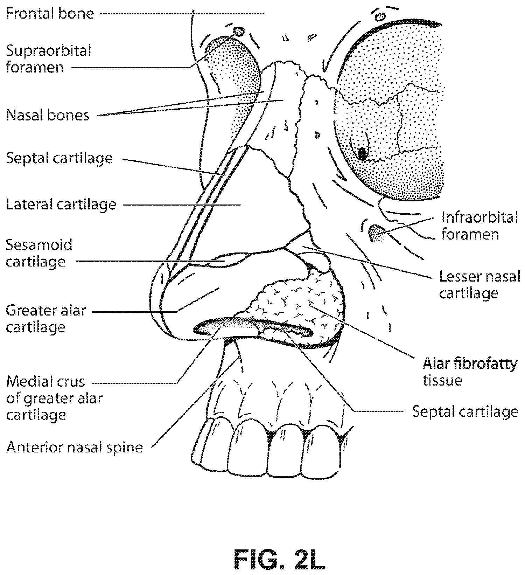

References to a direction, e.g. "lateral direction", and the like, unless the context clearly requires otherwise, are to be understood to refer to anatomical directions with respect to the body when the patient interface 3000 is being worn by the patient in its normal in-use position. Reference to "substantially" in relation to such a direction, for example "substantially lateral direction" may be understood to mean at least partially extending in the relevant direction. In one example, a direction may be "lateral" if the direction generally extends in a direction perpendicular to the mid-sagittal plane of the patient's face (represented in Fig. 2C) and/or relative to the mid-sagittal plane of the plenum chamber (represented in Figs. 3U to 3W). A component extending in a lateral direction may also extend in another direction.

Forms of the technology therefore relate to a patient interface 3000 configured to effect "side venting", i.e. air is vented from a part of the patient interface (for example the plenum chamber 3200 or conduit) in a generally sidewards (i.e. lateral) direction from the patient in use. As will be explained, another component may cause the flow of air to be deflected from this direction before the flow of air dissipates to ambient.

In some forms, the patient interface 3000 comprises an air circuit 4170, e.g. an air delivery tube 4172. In the examples illustrated in Figs. 6A to 16A, and Figs. 17 to 24, the patient interface 3000 is configured to connect to the air delivery tube 4172 (not shown in some of the figures). As shown in these examples, the air delivery tube 4172 connects to the plenum chamber 3200 via the connection port 3600. The connection port 3600 may be provided in the plenum chamber and the air delivery tube 4172 connected directly to the plenum chamber 3200. Alternatively, the connection port 3600 may be provided in another component and the air delivery tube 4172 may connect indirectly to the plenum chamber 3200 via one or more other components. In the example shown in Figs. 22, the patient interface 3000 is configured to connect to an air delivery tube (not shown in this figure). In this example, the air delivery tube can connect to the plenum chamber 3200 via at least one headgear tube 3350 which comprises the connection port 3600.

In some forms of the present technology, for example, as illustrated in Figs. 6A to 24, the patient interface 3000 comprises a deflector 3500, preferably a pair of deflectors 3500, configured to redirect or turn at least a portion of the vented flow of gases before the flow of air dissipates as it joins the ambient body of air. References to "redirect", "turn" and the likes, unless the context clearly requires otherwise, are to be understood as a change in direction between the direction of the vented flow of gases and the direction of the deflected or turned flow of gases, i.e. the direction of the flow of gases after being deflected or turned by the deflector 3500.

In the forms of the present technology illustrated in Figs. 6A to 24, the patient interface 3000 comprises a positioning and stabilising structure 3300 to provide a force to hold the seal-forming structure 3100 in a therapeutically effective position on the patient's face. In certain forms, the positioning and stabilising structure 3300 comprises at least one headgear strap 3310, preferably a plurality of headgear straps 3310 or an assembly of headgear straps, for example, as shown in Figs. 6A, 11A, 12A, 13A, 14A, 15A, and 17. The positioning and stabilising structure 3300 may further comprise a frame 3360, for example as shown in Figs. 22 to 24. In other forms, the positioning and stabilising structure 3300 may comprise one or more headgear tubes 3350 that deliver pressurised air received from a conduit, e.g. the air delivery tube 4172, to the patient's airways, for example through the plenum chamber 3200 and seal-forming structure 3100.

In some forms of the present technology shown in Figs. 6A to 15D, the patient interface 3000 comprises a pair of connectors 3800 to facilitate attachment of the patient interface 3000 to the positioning and stabilising structure 3300.

In forms of the present technology, for example, as shown in Figs. 6A to 9D, Figs. 11A to 15D, and Figs. 17 to 24, the patient interface 3000 may comprise a diffuser 3900 for diffusing the flow of air. This may help reduce and/or prevent noise. In some forms of the present technology, the plenum chamber 3200 and seal-forming structure 3100 are provided by one physical component. In the example illustrated in Figs. 6A to 23, the patient interface 3000 comprises a cushion module 3150. In this example, the cushion module 3150 provides both the plenum chamber 3200 and the seal-forming structure 3100 of the patient interface 3000. The seal-forming structure 3100 and other wall portions of the cushion module 3150 form the plenum chamber 3200 in this example. In other forms of the technology, the plenum chamber 3200 and seal-forming structure 3100 are provided by a plurality of physical components, for example one component for the plenum chamber 3200 and another for the seal-forming structure 3100.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH2O with respect to ambient.

The patient interface 3000 in accordance with one form of the present technology is constructed and arranged to be able to provide a supply of air at a positive pressure of at least 10 cmH2O with respect to ambient.

The patient interface 3000 in accordance with one form of the present technology is constructed and arranged to be able to provide a supply of air at a positive pressure of at least 20 cmH2O with respect to ambient.

### 4.3.1 Plenum chamber

A patient interface 3000 according to some examples of the present technology comprises a plenum chamber 3200 pressurisable to a therapeutic pressure of at least 6 cmH2O above ambient air pressure. The plenum chamber 3200 may receive a flow of air at the therapeutic pressure for breathing by a patient.

The plenum chamber 3200 in some forms of the present technology is at least partially provided by a cushion module 3150 of the patient interface 3000.

The cushion module 3150 according to some examples (such as those shown in Figs. 6A to 24) may comprise a frame portion 3210 and the seal-forming structure 3100.

The plenum chamber 3200 may be at least partially formed by both the frame portion 3210 and the seal-forming structure 3100. The frame portion 3210 may support the seal-forming structure 3100 in position against the patient's face in use. The frame portion 3210 and seal-forming structure 3100 may together partially enclose a volume of space which in use has air therein pressurised above atmospheric pressure, forming the plenum chamber 3200.

In particular, the frame portion 3210 may at least partially form a plenum chamber 3200 pressurisable to a therapeutic pressure of at least 6 cmH2O above ambient air pressure.

In some forms of the present technology, for example as shown in Figs. 10A to 10C, and Figs. 13A to 15D, the frame portion 3210 may comprise one or more laterally projecting connection portions 3212 configured to connect to the positioning and stabilising structure 3300. The laterally projecting connection portions 3212 may also partially form the plenum chamber 3200 along with other portions of the cushion module 3150, that is the laterally projecting connection portions 3212 may be configured to each contain a volume, where the volume inside the laterally projecting connection portions 3212 is part of the volume inside the plenum chamber 3200.

The seal-forming structure 3100 may be provided to the frame portion 3210 and may at least partially form the plenum chamber 3200. The seal-forming structure 3100 may be connected to the frame portion 3210, either permanently connected or removably connected. The seal-forming structure 3100 may be supported by the frame portion 3210.

In certain forms, the plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200..

In some forms, the plenum chamber 3200 is formed from a single homogeneous piece of material. In some forms, the plenum chamber 3200 may be formed from a homogenous piece of material fitted with connectors formed from another material. In other forms, the plenum chamber 3200 is constructed from a plurality of materials, for example one material may be used to form the frame portion 3210 and another material may be used to form the seal-forming structure 3100, with the plenum chamber 3200 comprising at least a part of both the frame portion 3210 and the seal-forming structure 3100.

In certain forms of the present technology, the plenum chamber 3200 does not cover the eyes of the patient in use. In other words, the eyes are outside the pressurised volume defined by the plenum chamber 3200. Such forms tend to be less obtrusive and / or more comfortable for the wearer, which can improve compliance with therapy.

In certain forms of the present technology, the plenum chamber 3200 is constructed from a transparent material, e.g. silicone, a thermoplastic elastomer, a transparent polycarbonate or the like. For example, the majority of the cushion module 3150 is formed from silicone in the examples shown in Figs. 6A to 24. In particular, the frame portion 3210 and seal-forming structure 3100 are both formed from silicone in those examples. The use of a transparent material can reduce the obtrusiveness of the patient interface 3000, and help improve compliance with therapy. The use of a transparent material can aid a clinician to observe how the patient interface 3000 is located and functioning.

In certain forms of the present technology, the plenum chamber 3200 is constructed from a translucent material. The use of a translucent material can reduce the obtrusiveness of the patient interface 3000, and help improve compliance with therapy.

In the exemplary forms of the technology shown in Figs. 6A to 23, the frame portion 3210 may be flexible, for example it may be formed from a material having a relatively low modulus of elasticity. This may allow the frame portion 3210 to flex or bend. The seal-forming structure 3100 may also be flexible, for example formed from the same material, or another material having a relatively low modulus of elasticity. In some examples, the frame portion 3210 and seal-forming structure 3100 are integrally formed and may be formed from the deformable material. The frame portion 3210 may be formed from an elastomeric material, such as silicone. The seal-forming structure 3100 may be formed from the elastomeric material. The frame portion 3210 and seal-forming structure 3100 may comprise one piece. In these examples, the frame portion 3210 and seal-forming structure 3100 are moulded together as a single part formed from an elastomeric material, e.g. silicone. The seal-forming structure 3100 and the frame portion 3210 (or at least a majority of the frame portion 3210) may be formed (e.g. constructed, moulded or the like) together from a single homogenous piece of a deformable material, such as an elastomeric material, e.g. silicone. The frame portion 3210 and the seal-forming structure 3100 may be of unitary construction.

In other examples of the technology the seal-forming structure 3100 may be removably connected to the frame portion 3210. The seal-forming structure 3100 may be connected to the frame portion 3210 by a soft-to-soft, soft-to-hard or a hard-to-hard connection.

In the form of the present technology illustrated in Figs. 6A to 23, the plenum chamber 3200 comprises an inlet 3220 configured to receive a flow of air at the therapeutic pressure for breathing by the patient. In the examples shown in Figs. 6A to 15D, the plenum chamber 2300 comprises an inlet 3220 and a pair of openings 3240, configured to permit fluid communication with an interior of the plenum chamber 3200. In the examples illustrated in Figs. 6A to 15D, the inlet 3220 and the openings 3240 are located on the frame portion 3210. In the examples shown in Figs. 17 to 24, the plenum chamber 2300 comprises the inlet 3220 and does not comprise additional openings 3240, other than that required to convey gas to and from the patient's airways.

In some forms, the inlet 3220 may be circular in shape. For instance, in the examples of Figs. 6A to 9D, the inlet 3220 is substantially circular. In some forms, the inlet 3220 may be a non-circular shape. For example, the inlet 3220 may be generally elliptical or oval-shaped, as shown in Figs. 10A to 13C and Figs. 15 to 15D. As is shown in Fig. 20, in some forms, the inlet 3220 may be generally triangular in shape, with curved corners, when viewed from one angle. The shape of the inlet 3220 may not lie entirely on a plane but instead the outline of the inlet 3220 may form a three-dimensional space curve, as in the form of Fig. 20. In these examples, the inlet 3220 may have a width (i.e. distance between a first lateral edge and a second lateral edge of the inlet 3220) which is greater than the height (i.e. distance between a superior edge and an inferior edge of the inlet 3220).

As illustrated in the examples of Figs 6A to 24, the plenum chamber 3200 comprises an anterior portion 3211. The frame portion 3210 may comprise the anterior portion 3211. The inlet 3220 may be located in the anterior portion 3211.

In some forms, the inlet 3220 or parts thereof may be located on a lateral portion 3213 of the plenum chamber 3200. For example, as shown in Figs. 10A to 13C, Figs. 15A to 15D and Fig. 20, at least part of the inlet 3220 is located on the lateral portion 3213 of the plenum chamber 3200.

The frame portion 3210, as shown in Figs. 10A to 10C, Figs. 13A to 13C and Figs. 15A to 15D, may comprise a pair of laterally projecting connection portions 3212. Each laterally projecting connection portion 3212 comprises an opening 3240 configured to be in fluid communication with an interior of the plenum chamber 3200.

In some examples, a rigidiser may be provided to the frame portion 3210 to make it more rigid (while still being flexible). In some examples, the frame portion 3210 and the seal-forming structure 3100 are formed from a material having a relatively low modulus of elasticity (e.g. silicone, TPE or the like) and the frame portion 3210 comprises a rigidiser. In particular, the patient interface 3000 or cushion module 3150 may comprise a rigidiser. The rigidiser may be provided to the frame portion 3210. The rigidiser may be configured to rigidise the frame portion 3210. The rigidiser may be configured to resist deformation of the frame portion 3210. The rigidiser may be configured to provide support to the frame portion 3210. The rigidiser may be more rigid than the frame portion 3210. For example, the rigidiser may be formed from a material having a modulus of elasticity which is higher than the modulus of elasticity of the material used to form the frame portion 3210.

In the example shown in Figs. 22 to 24, the frame 3360 may act as the rigidiser. Other aspects of the frame 3360 are discussed elsewhere in the specification.

However, in other examples, the frame portion 3210 may be relatively rigid. The relatively rigid frame portion 3210 may be formed from a material having a relatively high modulus of elasticity, for example.

In the example shown in Fig. 17, the plenum chamber 3200 comprises the inlet 3220 and the at least one headgear tube 3350 is configured to be in fluid communication with the inlet 3220 in use. For example, as is perhaps best shown in Fig. 21, the at least one headgear tube 3350 comprises an opening 3356, positioned and shaped so that, the headgear tube 3350 is in fluid communication, in use, with the inlet 3220 through the opening 3356.

Referring to the example of Figs. 17 and 18, the inlet 3220 may be configured to provide a lead-in for the at least one headgear tube 3350 when connecting it to the plenum chamber 3200. The size, location and shape of the inlet 3220 may be selected to provide the lead-in. For instance, parts of the inlet 3220 located on the lateral portions 3213 of the plenum chamber 3200 may be sized and shaped to contour to the shape of the at least one headgear tube 3350. This can facilitate assembly of the patient interface 3000, i.e. facilitate positioning and attachment of the at least one headgear tube 3350 to the plenum chamber 3200.

In addition, the inlet 3220 located on the lateral portion(s) 3213 of the plenum chamber 3200 may provide a lateral flow path to the interior of the plenum chamber 3200. In other words, the inlet 3220 allows the flow of air conveyed by the at least one headgear tube 3350 from the air circuit 4170 to enter the interior of the plenum chamber 3200 in a substantially lateral direction through the inlet 3220.

### 4.3.2 Seal-forming structure

A patient interface 3000 according to some examples of the present technology comprises a seal-forming structure 3100 configured to form a seal with a region of the patient's face surrounding an entrance to the patient's airways. The seal-forming structure 3100 may be configured to maintain said therapeutic pressure in the plenum chamber 3200 throughout the patient's respiratory cycle in use.

In some forms, the seal-forming structure 3100 is configured to form a seal with or around portions of the patient's nose in use. Therefore, it is to be appreciated that in some forms the patient interface 3000 may be a nose-only mask, also referred to as a nasal mask.

In other forms which are not illustrated, the seal-forming structure 3100 may be configured to form a seal in use with the other parts of patient's face, e.g. with or around portions of the patient's nose and with or around portions of the patient's mouth. Therefore, it is to be appreciated that in some forms the patient interface 3000 may be a nose and mouth mask, also referred to as a full face or oro-nasal mask, and forms of the technology are not limited to nose-only or nasal masks.

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 4.3.2.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 4.3.2.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 4.3.2.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 4.3.2.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 4.3.2.5 Forehead region

In one form, the seal-forming structure that forms a seal in use on a forehead region of the patient's face. In such a form, the plenum chamber may cover the eyes in use.

### 4.3.2.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 4.3.2.7 Nose-only Masks

In one form, the patient interface 3000 comprises a seal-forming structure 3100 configured to seal around an entrance to the patient's nasal airways but not around the patient's mouth. The seal-forming structure 3100 may be configured to seal to the patient's lip superior. The patient interface 3000 may leave the patient's mouth uncovered. This patient interface 3000 may deliver a supply of air or breathable gas to both nares of patient 1000 and not to the mouth. This type of patient interface may be identified as a nose-only mask.

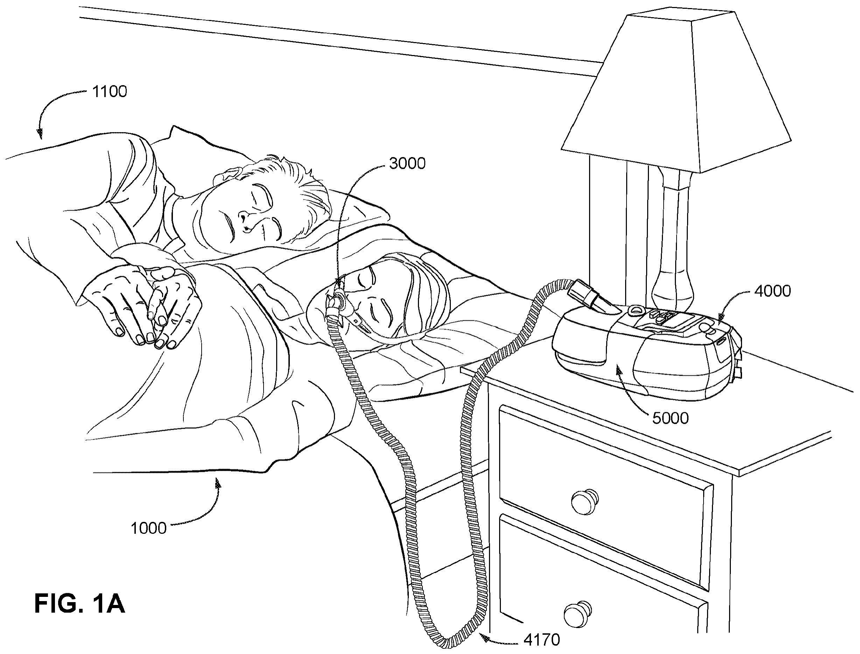

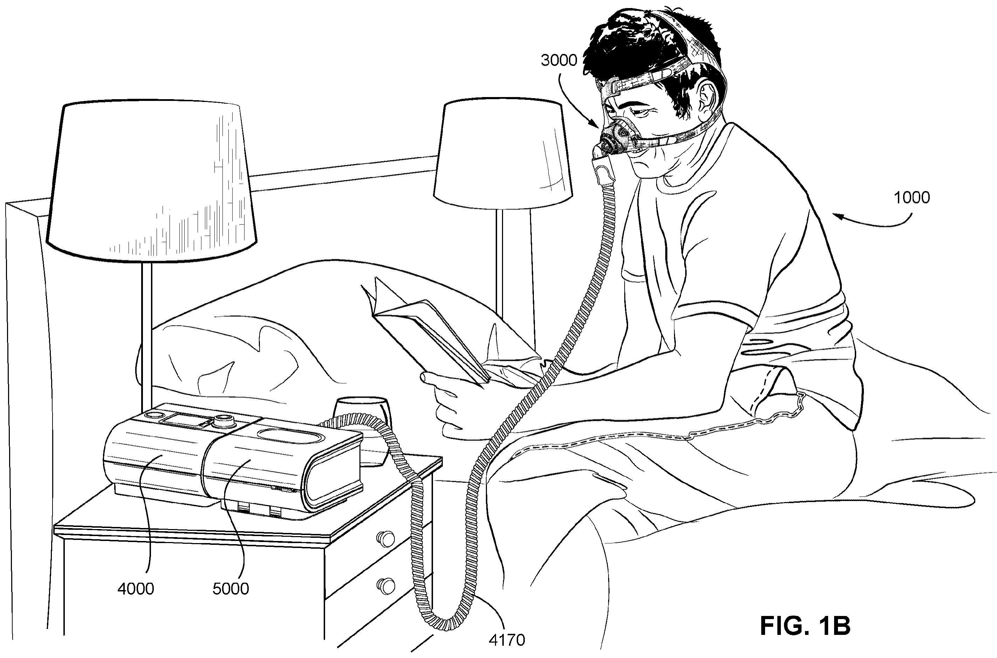

One form of nose-only mask according to the present technology is what has traditionally been identified as a "nasal mask", having a seal-forming structure 3100 configured to seal on the patient's face around the nose and over the bridge of the nose. A nasal mask may be generally triangular in shape. In one form, the non-invasive patient interface 3000 comprises a seal-forming structure 3100 that forms a seal in use to an upper lip region (e.g. the lip superior), to the patient's nose bridge or at least a portion of the nose ridge above the pronasale, and to the patient's face on each lateral side of the patient's nose, for example proximate the patient's nasolabial sulci. The patient interface 3000 shown in Fig. 1B has this type of seal-forming structure 3100. This patient interface 3000 may deliver a supply of air or breathable gas to both nares of patient 1000 through a single orifice.

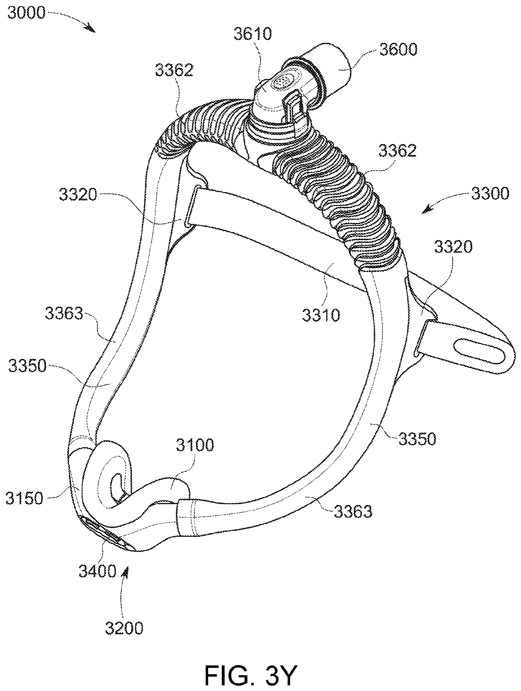

Another form of nose-only mask may seal around an inferior periphery of the patient's nose without engaging the user's nasal ridge. This type of patient interface 3000 may be identified as a "nasal cradle" mask and the seal-forming structure 3100 may be identified as a "nasal cradle cushion", for example. In one form, for example as shown in Fig. 3Y, the seal-forming structure 3100 is configured to form a seal in use with inferior surfaces of the nose around the nares. The seal-forming structure 3100 may be configured to seal around the patient's nares at an inferior periphery of the patient's nose including to an inferior and/or anterior surface of a pronasale region of the patient's nose and to the patient's nasal alae. The seal-forming structure 3100 may seal to the patient's lip superior. The shape of the seal-forming structure 3100 may be configured to match or closely follow the underside of the patient's nose and may not contact a nasal bridge region of the patient's nose or any portion of the patient's nose superior to the pronasale. In one form of nasal cradle cushion, the seal-forming structure 3100 comprises a bridge portion dividing the opening into two orifices, each of which, in use, supplies air or breathable gas to a respective one of the patient's nares. The bridge portion may be configured to contact or seal against the patient's columella in use. Alternatively, the seal-forming structure 3100 may comprise a single opening to provide a flow or air or breathable gas to both of the patient's nares.

In some forms, a nose-only mask may comprise nasal pillows, described above.

Referring to the examples illustrated in Figs. 6A to 15D, the seal-forming structure 3100 is configured to form a seal in use with the underside of the patient's nose, around the patient's nares. In the examples illustrated in Figs. 6A to 15D, the patient interface 3000 is in the form of a nose-only or nasal mask.

In the illustrated forms of Figs. 6A to 15D, the seal-forming structure 3100 comprises at least one hole 3110, or a pair of holes 3110, configured to allow a flow of air at therapeutic pressure to be delivered to the patient's nares. Each hole 3110 aligns in use with a respective nare of the patient. In some examples the seal-forming structure 3100 may comprise a single hole 3110 configured to allow the flow of air to be delivered to both nares of the patient. In examples, the cushion module 3150 comprises the at least one hole 3110. In some examples, the at least one hole 3110 are formed in a central portion of the seal-forming structure 3100.

### 4.3.2.8 Nose and Mouth Masks

In one form, the patient interface 3000 comprises a seal-forming structure 3100 configured to seal around an entrance to the patient's nasal airways and also around the patient's mouth. The seal-forming structure 3100 may be configured to seal to the patient's face proximate a chin region. This patient interface 3000 may deliver a supply of air or breathable gas to both nares and to the mouth of patient 1000. This type of patient interface may be identified as a nose and mouth mask.

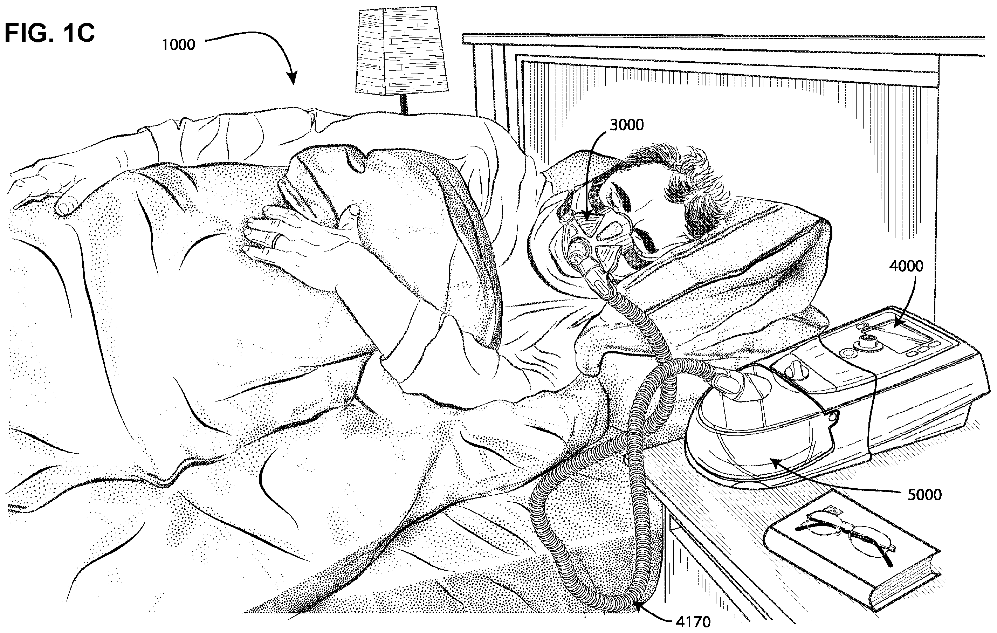

One form of nose and mouth mask according to the present technology is what has traditionally been identified as a "full-face mask", having a seal-forming structure 3100 configured to seal on the patient's face around the nose, below the mouth and over the bridge of the nose. A full-face mask may be generally triangular in shape. In one form the patient interface 3000 comprises a seal-forming structure 3100 that forms a seal in use to a patient's chin-region (which may include the patient's lip inferior and/or a region directly inferior to the lip inferior), to the patient's nose bridge or at least a portion of the nose ridge superior to the pronasale, and to cheek regions of the patient's face. The patient interface 3000 shown in Fig. 1C is of this type. This patient interface 3000 may deliver a supply of air or breathable gas to both nares and mouth of patient 1000 through a single orifice. This type of seal-forming structure 3100 may be referred to as a "full face cushion".

In another form the patient interface 3000 comprises a seal-forming structure 3100 that forms a seal in use on a patient's chin region (which may include the patient's lip inferior and/or a region directly inferior to the lip inferior), to an inferior and/or an anterior surface of a pronasale portion of the patient's nose, to the alae of the patient's nose and to the patient's face on each lateral side of the patient's nose, for example proximate the nasolabial sulci. The seal-forming structure 3100 may also form a seal against a patient's lip superior. A patient interface 3000 having this type of seal-forming structure may have a single opening configured to deliver a flow of air or breathable gas to both nares and mouth of a patient, may have an oral hole configured to provide air or breathable gas to the mouth and a nasal hole configured to provide air or breathable gas to the nares, or may have an oral hole for delivering air to the patient's mouth and two nasal holes for delivering air to respective nares. This type of patient interface 3000 may have a nasal portion and an oral portion, the nasal portion sealing to the patient's face at similar locations to a nasal cradle mask.

In a further form of nose and mouth mask, the patient interface 3000 may comprise a seal-forming structure 3100 having a nasal portion comprising nasal pillows and an oral portion configured to form a seal to the patient's face around the patient's mouth.

In some forms, the seal-forming structure 3100 may have a nasal portion that is separate and distinct from an oral portion. In other forms, a seal-forming structure 3100 may form a contiguous seal around the patient's nose and mouth.

It is to be understood that the above examples of different forms of patient interface 3000 do not constitute an exhaustive list of possible configurations. In some forms a patient interface 3000 may comprise a combination of different features of the above described examples of nose-only and nose and mouth masks.

Referring to the examples illustrated in Figs. 17 to 24, the seal-forming structure 3100 is configured to form a seal in use around the patient's nares and mouth. In these examples, the patient interface 3000 is in the form of a nose and mouth mask or a full face mask.

In some forms, the seal-forming structure 3100 comprises at least one hole 3110, or at least a pair of holes (at least one hole for the nares and another hole for the mouth), configured to allow a flow of air at therapeutic pressure to be delivered to the patient's nares and the mouth. In examples, the cushion module 3150 comprises the at least one hole 3110. In some examples, the at least one hole 3110 are formed in a central portion of the seal-forming structure 3100. In the examples shown in Figs. 17 to 24, the seal-forming structure 3100 comprises a single hole 3110 configured to allow the flow of air to be delivered to both nares and the mouth of the patient.

### 4.3.3 Connection port

Connection port 3600 allows for connection of the patient interface 3000 to the air circuit 4170. As illustrated in the Figs. 6A to 24, the patient interface 3000 comprises a connection port 3600 for connection to the air circuit 4170. In the illustrated examples, the air circuit 4170 is in the form of an air delivery tube 4172.

### 4.3.3.1 Connection to plenum chamber

The air delivery tube 4172 may connect to the plenum chamber 3200 via the connection port 3600 such that the air delivery tube 4172 is in fluid communication with the inlet 3220 in use. In some forms of the present technology, for example, as shown in Figs. 7A to 7B, Figs. 9A to 11C and Figs. 13A to 15D, the connection port 3600 is attached to, or forms, the inlet 3220.

In some forms, the connection port 3600 may be located on an anterior region of the plenum chamber 3200 in use. As illustrated in examples of Figs.6A to 15D, and Figs. 22 to 24, the connection port 3600 is located on a medial region of the plenum chamber 3200 in use. The medial region of the plenum chamber 3200 is located centrally in use, i.e. so that, in use, the mid-sagittal plane of the patient passes through the medial portion. In the examples of Figs. 22 to 24, the frame 3360 provides the connection port 3600. The frame 3360 may comprise an opening 3362. The air circuit (not shown in Figs. 22 to 24) is configured to connect to and be, in use, in fluid communication with the inlet 3220 through the opening 3362 in the frame 3360. That is, in some forms, the air delivery tube 4172 may physically connect to the frame 3360 in order to convey breathable gas into the plenum chamber 3200 through inlet 3220 while, in other forms, the air delivery tube 4172 may pass through the opening 3362 in frame 3360 and physically connect to the inlet 3220 of plenum chamber 3200.

In the examples of Fig. 6A to 15D, and Figs. 22 to 24, the air circuit 4170 connects to the anterior portion 3211. As illustrated, the connection port 3600 may be located in a medial region of the anterior portion 3211. The medial region may be located centrally in use, i.e. so that, in use, the mid-sagittal plane of the patient passes through the medial portion. As shown in Figs. 6A to 15D, the air circuit 4170 connects to the frame portion 3210 via the connection port 3600. In the examples of Figs. 22 to 24, the air circuit (not shown) can connect to the frame 3360. In other forms not shown, the air circuit may connect to the frame 3360 and/or the plenum chamber 3200, e.g. the frame portion 3210.

### 4.3.3.2 Connection to headgear tube

In some forms of the present technology, the air circuit 4170 is connected to the at least one headgear tube 3350. This is explained in more detail below. For example, as shown in Fig. 17, the connection port 3600 is provided on the at least one headgear tube 3350, for example to a part of the headgear tube 3350 that is configured to be positioned on top of the patient's head in use. In this example, the connection port 3600 receives the flow of breathable gas from the air delivery tube 4172 and conveys the flow to the headgear tubes 3350.

### 4.3.4 Positioning and stabilising structure

A patient interface 3000 according to some examples of the present technology comprises a positioning and stabilising structure 3300 to provide a force to hold the seal-forming structure 3100 in a therapeutically effective position on the patient's face. The positioning and stabilising structure 3300 may comprise and function as "headgear" since it engages the patient's head in order to hold the patient interface 3000 in a sealing position.

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another suitable for a small sized head, but not a large sized head.

### 4.3.4.1 Frame

As shown in Figs. 22 to 24, in some forms, the positioning and stabilising structure 3300 comprises a frame 3360. The frame 3360 is configured to facilitate indirect attachment of the plenum chamber 3200 to at least one headgear strap (not shown in these figures) of the positioning and stabilising structure 3300.

The frame 3360 may be structured and arranged to extend across at least a portion of the plenum chamber 3200 in use. The frame 3360 may extend laterally across the anterior portion 3211 and, in some forms, lateral portions 3213 of the plenum chamber 3200. As illustrated in Fig. 22, in some forms, the frame 3360 may extend across the entire width of the plenum chamber 3200 (i.e. the distance between the lateral edges or ends of the frame portion 3210). As shown, the ends of the frame 3360 may extend beyond the lateral edges or ends of the plenum chamber 3200. In other forms, the frame 3360 may be shorter so that the length of the frame is less than the lateral width of the plenum chamber 3200.

As illustrated in Figs. 22 and 24, in some forms, the frame 3360 may be substantially elongate. That is, the distance between superior and inferior edges of the frame 3360 is less, for example substantially less, than the width of the frame 3360 (i.e. the distance between the lateral ends of the frame 3360).

As illustrated in Figs. 22 and 24, in some forms, the opening 3362 may be located on a medial region of the frame 3360. The medial region of the frame 3360 is located centrally in use, i.e. so that, in use, the mid-sagittal plane of the patient passes through the medial portion of the frame 3360.

When viewed from a superior or inferior direction (not shown in the figures), the frame 3360 may be generally arcuate in shape with a concave side of the frame 3360 facing towards the patient's face in use. A surface of the plenum chamber 3200 to which the frame attaches or engages in use, e.g. frame portion 3210, may also be generally arcuate in shape when viewed from the same direction. Therefore, the shape of the frame 3360 and the shape of the frame portion 3210 may have complimentary contours allowing the two components to mate, as shown in Fig. 22. In some forms, the frame 3360 may have a preformed or predefined arcuate shape, while in other forms the frame 3360 may be able to flex into an arcuate shape, or a more arcuate shape, when a tension force from headgear straps is applied to the ends of the frame 3360.

The frame 3360 may comprise at least one connector, preferably a pair of connectors 3364 to facilitate attachment of the frame 3360 to the at least one headgear strap (not shown in Figs. 22 and 23). The at least one headgear strap may be removably attached to the connector 3800. In the form of technology shown, the connectors 3364 each comprise a slot 3364a. The slot 3364a is configured to receive a portion of the headgear strap in use to connect it to the frame 3360.

In the illustrated form, the connectors 3364 are located on lateral ends of the frame 3360. The connectors 3364 may be formed as part of the frame 3360, e.g. integrally formed with the frame, as shown in Figs. 22 and 24. In other forms not shown, the connectors 3364 may be separately formed and removably or permanently attached to the frame 3360.

In the example of Figs. 22 and 24, the frame 3360 comprises a body portion 3368 and a pair of arms 3366 extending laterally outward from the body portion 3368. One of the pair of connectors 3364 may be provided to an end of each arm 3366. The body portion 3368, the arms 3366, and connectors 3364 may be formed together to provide the frame 3360, e.g. integrally formed.

In some forms, patient interface 3000 is in the form of a full face mask, as described earlier, and configured to provide a two-point headgear connection, as opposed to a four-point headgear connection. As illustrated, the frame 3350 is configured to provide a two-point headgear connection, i.e. the frame comprises two connectors 3364: one on each lateral side. Fewer points of connection for headgear may make the patient interface 3000 easier for the patient to put on, reduce feelings of claustrophobia when wearing the patient interface, and reduce the amount of facial marking caused by the effect of the headgear straps contacting the patient's skin and hair.

In other forms not shown, the connector 3364 may comprise any other suitable connector or attachment mechanism, such as clips or buckles. It is to be appreciated that in yet other forms, the at least one headgear strap may be permanently attached to or formed with the frame 3360.

As illustrated in Fig. 22, the frame 3360 may be configured to be held in position with respect to the plenum chamber 3200.

In some examples, the frame 3360 may be attached to the plenum chamber 3200, e.g. the patient-facing (posterior) surface of the frame 3360 may be attached to the frame portion 3210 of the plenum chamber on an anterior side. In the illustrated example, the frame 3360 is removably attached to the frame portion 3210. This may facilitate replacement, cleaning and/or storage of the frame 3360. The frame 3360 may comprise a connector (not shown in Figs. 22 to 24) to facilitate removable attachment of the frame 3360 to the plenum chamber 3200, and hold the frame 3360 in place. For instance, the connector may comprise a lip extending around at least part of opening 3362, which may attach to the plenum chamber 3200 with an interference fit or friction fit. For example, the lip may interlock to the periphery of the inlet 3220 in the plenum chamber 3200. However, in other forms, the connector may comprise any other suitable connector or attachment mechanism to facilitate removable attachment between the two components, including interlocks, clips, hook-and-loop connectors, high friction surfaces, etc.

In some forms, the frame 3360 may not be directly connected to the plenum chamber 3200. For instance, the frame 3360 may be held in place on the plenum chamber 3200 using a force applied to the frame 3360 in a posterior direction. That is, tension of the headgear straps may hold the frame 3360 in place. As described below, the outer (i.e. anterior) surface of the plenum chamber 3200 may comprise a recess 3230, as shown in Fig. 20. The recess may be elongate and extend in a lateral direction across the plenum chamber 3200. The recess may be configured so that, in use, the frame 3360 may nest in the recess to help hold the plenum chamber 3200 in place.

In some forms, the frame 3360 may at least partially form the plenum chamber 3200 with the frame portion 3210 and the seal-forming structure 3100. For instance, a perimeter of the frame opening 3362 may be larger than a perimeter of the inlet 3220. Therefore, a portion of the frame 3360 may form part of the plenum chamber 3200. However, the frame portion 3210 and the seal-forming structure 3100 may form a substantial part of the plenum chamber 3200.

The frame may be formed from a material or combination of materials, for example polymers. Suitable polymers may include thermoplastics or elastomers, e.g. silicone.

### 4.3.4.2 Conduit headgear

### 4.3.4.2.1 Conduit headgear tubes

In some forms of the present technology, the positioning and stabilising structure 3300 comprises one or more headgear tubes 3350 that deliver pressurised air received from a conduit, e.g. the air delivery tube 4172, forming part of the air circuit 4170 from the RPT device to the patient's airways, for example through the plenum chamber 3200 and seal-forming structure 3100. Therefore, the at least one headgear tube 3350 is configured, in use, to convey the flow of air from an air circuit 4170 fluidly connected to the at least one headgear tube 3350 to an interior of the plenum chamber 3200 through the inlet 3220.

In the form of the present technology illustrated in Figs. 3Y and 17, the positioning and stabilising structure 3300 comprises two tubes 3350 that deliver air to the plenum chamber 3200 from the air circuit 4170. The tubes 3350 are configured to position and stabilise the seal-forming structure 3100 of the patient interface 3000 at the appropriate part of the patient's face (for example, the nose and/or mouth) in use. This allows the conduit of air circuit 4170 providing the flow of pressurised air to connect to a connection port 3600 of the patient interface in a position other than in front of the patient's face, for example on top of the patient's head.

In the form of the present technology illustrated in Figs. 3Y and 17, the positioning and stabilising structure 3300 comprises two tubes 3350, each tube 3350 being positioned in use on a different side of the patient's head and extending across the respective cheek region, above the respective ear (superior to the otobasion superior on the patient's head) to the elbow 3610 on top of the head of the patient 1000. This form of technology may be advantageous because, if a patient sleeps with their head on its side and one of the tubes 3350 is compressed to block or partially block the flow of gas along the tube 3350, the other tube 3350 remains open to supply pressurised gas to the patient. In other examples of the technology, the patient interface 3000 may comprise a different number of tubes, for example one tube, or two or more tubes. In one example in which the patient interface has one tube 3350, the single tube 3350 is positioned on one side of the patient's head in use (e.g. across one cheek region) and a strap forms part of the positioning and stabilising structure 3300 and is positioned on the other side of the patient's head in use (e.g. across the other region) to assist in securing the patient interface 3000 on the patient's head.

In the form of the technology shown in Figs. 3Y and 17, the two tubes 3350 are fluidly connected at superior ends to each other and to the connection port 3600. In some examples, the two tubes 3350 are integrally formed while in other examples the tubes 3350 are formed separately but are connected in use and may be disconnected, for example for cleaning or storage. Where separate tubes are used they may be indirectly connected together, for example each may be connected to a T-shaped connector having two arms/branches each fluidly connectable to a respective one of the tubes 3350 and a third arm or opening in the T-shaped connector providing the connection port 3600 for fluid connection to the air circuit 4170 in use.

The tubes 3350 may be formed from a flexible material, such as an elastomer, e.g. silicone or TPE, and/or from one or more textile and/or foam materials. The tubes 3350 may have a preformed shape and may be able to be bent or moved into another shape upon application of a force but may return to the original preformed shape in the absence of said force. The tubes 3350 may be generally arcuate or curved in a shape approximating the contours of a patient's head between the top of the head and the nasal or oral region.

In some examples, the one or more tubes 3350 are crush resistant to resist being blocked if crushed during use, for example if squashed between a patient's head and pillow, especially if there is only one tube 3350. The tubes 3350 may be formed with a sufficient structural stiffness to resist crushing or may be as described in US Patent no. 6,044,844, the contents of which are incorporated herein by reference

Each tube 3350 may be configured to receive a flow of air from the connection port 3600 on top of the patient's head and to deliver the flow of air to the seal-forming structure 3100 at the entrance of the patient's airways. In the example shown in Figs. 3Y and 17, each tube 3350 lies in use on a path extending from the plenum chamber 3200 across the patient's cheek region and superior to the patient's ear to the elbow 3610. For example, a portion of each tube 3350 proximate the plenum chamber 3200 may overlie a maxilla region of the patient's head in use. Another portion of each tube 3350 may overlie a region of the patient's head superior to an otobasion superior of the patient's head. Each of the tubes 3350 may also lie over the patient's sphenoid bone and/or temporal bone and either or both of the patient's frontal bone and parietal bone. The elbow 3610 may be located in use over the patient's parietal bone, over the frontal bone and/or over the junction therebetween (e.g. the coronal suture).

In certain forms of the present technology the patient interface 3000 is configured such that the connection port 3600 can be positioned in a range of positions across the top of the patient's head so that the patient interface 3000 can be positioned as appropriate for the comfort or fit of an individual patient. In some examples, the headgear tubes 3350 are configured to allow movement of an upper portion of the patient interface 3000 (e.g. a connection port 3600) with respect to a lower portion of the patient interface 3000 (e.g. a plenum chamber 3200). That is, the connection port 3600 may be at least partially decoupled from the plenum chamber 3200. In this way, the seal-forming structure 3100 may form an effective seal with the patient's face irrespective of the position of the connection port 3600 (at least within a predetermined range of positions) on the patient's head.

As described above, in some examples of the present technology the patient interface 3000 comprises a seal-forming structure 3100 in the form of a cradle cushion which lies generally under the nose and seals to an inferior periphery of the nose (e.g. an under-the-nose cushion). The positioning and stabilising structure 3300, including the tubes 3350 may be structured and arranged to pull the seal-forming structure 3100 into the patient's face under the nose with a sealing force vector in a posterior and superior direction (e.g. a posterosuperior direction). A sealing force vector with a posterosuperior direction may cause the seal-forming structure 3100 to form a good seal to both the inferior periphery of the patient's nose and anterior-facing surfaces of the patient's face, for example on either side of the patient's nose and the patient's lip superior.

### 4.3.4.2.2 Extendable and non-extendable tube portions

In some examples of the present technology, one or both of the tubes 3350 are not extendable in length. However, in some forms, the tubes 3350 may comprise one or more extendable tube sections, for example formed by an extendable concertina structure. In some forms, the patient interface 3000 may comprise a positioning and stabilising structure 3300 including at least one gas delivery tube comprising a tube wall having an extendable concertina structure. The patient interface 3000 shown in Fig. 3Y comprises tubes 3350, the superior portions of which comprise extendable tube sections each in the form of an extendable concertina structure 3362.

The cross-sectional shape of the non-extendable tube sections 3363 of the tubes 3350 may be circular, elliptical, oval, D-shaped or a rounded rectangle, for example as described in US Patent No. 6,044,844. A cross-sectional shape that presents a flattened surface of tube on the side that faces and contacts the patient's face or other part of the head may be more comfortable to wear than, for example a tube with a circular cross-section.

In some examples of the present technology, the non-extendable tube sections 3363 connects to the plenum chamber 3200 from a low angle. The headgear tubes 3350 may extend inferiorly down the sides of the patient's head and then curve anteriorly and medially to connect to the plenum chamber 3200 in front of the patient's face. The tubes 3350, before connecting to the plenum chamber 3200, may extend to a location at the same vertical position as (or, in some examples, inferior to) the connection with the plenum chamber 3200. That is, the tubes 3350 may project in an at least partially superior direction before connecting with the plenum chamber 3200. A portion of the tubes 3350 may be located inferior to the cushion module 3150 and/or the seal forming structure 3100. The tubes 3350 may contact the patient's face below the patient's cheekbones, which may be more comfortable than contact on the patient's cheekbones and may avoid excessively obscuring the patient's peripheral vision.

### 4.3.4.2.3 Conduit headgear connection port

In certain forms of the present technology, the patient interface 3000 may comprise a connection port 3600 located proximal to a superior, lateral or posterior portion of a patient's head. For example, in the form of the present technology illustrated in Figs. 3Y and 17, the connection port 3600 is located on top of the patient's head (e.g. at a superior location with respect to the patient's head). In this example the patient interface 3000 comprises an elbow 3610 forming the connection port 3600. The elbow 3610 may be configured to fluidly connect with a conduit of an air circuit 4170. The elbow 3610 may be configured to swivel with respect to the positioning and stabilising structure 3300 to at least partially decouple the conduit from the positioning and stabilising structure 3300. In some examples the elbow 3610 may be configured to swivel by rotation about a substantially vertical axis and, in some particular examples, by rotation about two or more axes. In some examples the elbow may comprise or be connected to the tubes 3350 by a ball-and-socket joint. The connection port 3600 may be located in the sagittal plane of the patient's head in use.

Patient interfaces having a connection port that is not positioned anterior to the patient's face may be advantageous as some patients may find a conduit that connects to a patient interface anterior to their face to be unsightly and/or obtrusive. For example, a conduit connecting to a patient interface anterior to the patient's face may be prone to interference with bedclothes or bed linen, particularly if the conduit extends inferiorly from the patient interface in use. Forms of the present technology comprising a patient interface having a connection port positioned superiorly to the patient's head in use may make it easier or more comfortable for a patient to lie or sleep in one or more of the following positions: a side-sleeping position, a supine position (e.g. on their back, facing generally upwards) or in a prone position (e.g. on their front, facing generally downwards). Moreover, connecting a conduit to an anterior portion of a patient interface may exacerbate a problem known as tube drag in which the conduit exerts an undesired force upon the patient interface during movement of the patient's head or the conduit, thereby causing dislodgement away from the face. Tube drag may be less of a problem when force is received at a superior location of the patient's head than anterior to the patient's face proximate to the seal-forming structure (where tube drag forces may be more likely to disrupt the seal).

### 4.3.4.2.4 Headgear Tube Fluid Connections

In the example of Fig. 3Y, the two tubes 3350 are fluidly connected at their inferior ends to the plenum chamber 3200. In certain forms of the technology, the connection between the tubes 3350 and the plenum chamber 3200 is achieved by connection of two rigid connectors. The tubes 3350 and plenum chamber 3200 may be configured to enable the patient to easily connect the two components together in a reliable manner. The tubes 3350 and plenum chamber 3200 may be configured to provide tactile and/or audible feedback in the form of a 're-assuring click' or a similar sound, so that the patient may easily know that each tube 3350 has been correctly connected to the plenum chamber 3200. In one form, the tubes 3350 are formed from a silicone or textile material and the inferior end of each of the silicone tubes 3350 is over-moulded to a rigid connector made, for example, from polypropylene, polycarbonate, nylon or the like. The rigid connector on each tube 3350 may comprise a female mating feature configured to connect with a male mating feature on the plenum chamber 3200. Alternatively, the rigid connector on each tube 3350 may comprise a male mating feature configured to connect to a female mating feature on the plenum chamber 3200. In other examples the tubes 3350 may each comprise a male or female connector formed from a flexible material, such as silicone or TPE, for example the same material from which the tubes 3350 are formed.

In other examples a compression seal is used to connect each tube 3350 to the plenum chamber 3200. For example, a resiliently flexible (e.g. silicone) tube 3350 without a rigid connector may be configured to be squeezed to reduce its diameter so that it can be compressed into a port in the plenum chamber 3200 and the inherent resilience of the silicone pushes the tube 3350 outwards to seal the tube 3350 in the port in an air-tight manner. Alternatively, in a hard-to-hard type engagement between the tube 3350 and the plenum chamber 3200, each tube 3350 and/or plenum chamber 3200 may comprise a pressure activated seal, for example a peripheral sealing flange. When pressurised gas is supplied through the tubes 3350 the sealing flange may be urged against the join between the tubes and a circumferential surface around a port or connector of the plenum chamber 3200 to form or enhance a seal between the tube 3350 and plenum chamber 3200.

The tubes 3350 may be configured to be held in position with respect to the plenum chamber 3200.

Referring to Fig. 17, in some forms, the at least one headgear tube 3350 is attached to the plenum chamber 3200, e.g. the patient-facing (posterior) surface of the headgear tubes 3350 may be attached to the frame portion 3210. In the illustrated example, the at least one headgear tube 3350 is removably attached to the frame portion 3210. This may facilitate replacement, cleaning and/or storage of the headgear conduit 3350.

In the example shown in Fig. 21, the at least one headgear tube 3350 comprises a connector 3358 to facilitate removable attachment of the at least one headgear tube 3350 to the plenum chamber 3200. The connector 3358 may comprises a lip extending around at least part of opening 3356, as shown, which attaches to the plenum chamber 3200 with an interference fit or friction fit. For example, the lip may interlock to the periphery of the inlet 3220 in the plenum chamber 3200. However, in other forms, the connector 3358 may comprise any other suitable connector or attachment mechanism to facilitate removable attachment between the two components, including interlocks, clips, hook-and-loop connectors, high friction surfaces, etc.

In some forms, the at least one headgear tube 3350 may not be directly connected to the plenum chamber 3200. For instance, the at least one headgear tube 3350 may be held in place on the plenum chamber 3200 using a force applied to the at least one headgear tube 3350 in a posterior direction. That is, tension of the headgear straps may hold the frame 3360 in place. As described below, the outer (i.e. anterior) surface of the plenum chamber 3200 may comprise a recess 3230, as shown in Fig. 20. The recess may be elongate and extend in a lateral direction across the plenum chamber 3200. The recess may be configured so that, in use, an inferior part of the tubes 3350 may nest in the recess to help hold the plenum chamber 3200 in place.

In the form of the present technology illustrated in Figs. 17 to 21, the positioning and stabilising structure 3300 comprises two tubes 3350. As shown in Figs. 17 and 18, in some forms, the two tubes 3350 are fluidly connected to each other at inferior ends and to the inlet 3220. The two tubes 3350 may be integrally formed, as shown in Fig. 18, or the two tubes 3350 may be formed separately but be connected in use and able to be disconnected, as shown in Figs. 17 and 21. This may facilitate cleaning, replacement or storage of the tubes 3350.

For example, as shown in Figs. 17 and 21, the positioning and stabilising structure 3300 may comprise a tube portion 3354. The tube portion 3354 may be positioned anterior to the plenum chamber 3200 in use.

The tube portion 3354 may be structured and arranged to extend across at least a portion of the plenum chamber 3200 in use. The tube portion 3354 may extend across the anterior portion 3211 and lateral portions 3213 of the plenum chamber 3200. As illustrated in Figs. 17 and 18, in some forms, the tube portion 3354 may extend across the entire width of the plenum chamber 3200 (i.e. the distance between the lateral edges or ends of the plenum chamber 3200).

In some forms, the tube portion 3354 is configured to connect the inferior ends of the two tubes 3350 to each other. As shown in Fig. 21, the tube portion 3354 may comprise the connector 3358.

In the examples shown in Figs. 17 and 21, the tube portion 3354 is separately formed and the two tubes 3350 are attached to the tube portion 3354. However, in some forms, for example as shown in Fig.18, the tube portion 3354 may be integrally formed with the two tubes 3350 to form a single tube structure.

The tube portion 3354 may be made from different material(s) than the two tubes 3350. For example, the tube portion 3354 may be more rigid or stiffer than the two tubes 3350. Alternatively, the tube portion 3354 may be made from the same materials as is used for the tubes 3350.

In some forms, the tube portion 3354 may be considered to act as the rigidiser for the plenum chamber 3200, as described above.

### 4.3.4.2.5 Conduit headgear straps

In certain forms of the present technology, the positioning and stabilising structure 3300 comprises at least one headgear strap acting in addition to the tubes 3350 to position and stabilise the seal-forming structure 3100 at the entrance to the patient's airways. As shown in Figs. 3Y and 17, the patient interface 3000 comprises a headgear strap 3310 forming part of the positioning and stabilising structure 3300. The headgear strap 3310 may be known as a back strap or a rear headgear strap, for example. The strap 3310 may be connected between the two tubes 3350 positioned on each side of the patient's head and, when worn, passes around the back of the patient's head, for example overlying or lying inferior to the occipital bone of the patient's head in use. The strap 3310 may connect to each tube above the patient's ears. With reference to Figs. 3Y and 17, the positioning and stabilising structure 3300 comprises a pair of tabs 3352 provided to an anterior side of the tubes 3350. In use the strap 3310 may be connected between the tabs 3352. The tabs 3352 facilitate the connection of the strap 3310 of the tubes 3350, for example the tabs 3352 may contain holes through which the strap 3310 can pass. The strap 3310 may be sufficiently flexible to pass around the back of the patient's head and lie comfortably against the patient's head, even when under tension in use.

In other examples of the present technology, one or more further straps may be provided. For example, patient interfaces 3000 according to examples of the present technology which take the form of a full face mask may have two connections to the plenum chamber and/or cushion module on each side of the patient's face, creating a "four-point connection" (as opposed to the two-point connection of Fig. 17). In these forms, the positioning and stabilising structure may comprise a second strap connected between the plenum chamber and/or cushion module and the parts of the positioning and stabilising structure positioned, in use, on the top and/or rear portions of the patient's head. For example the second strap may be positioned inferior to the conduit headgear tubes 3350 when worn and may be configured to lie against the patient's head proximate the patient's neck and/or against posterior surfaces of the patient's neck.

### 4.3.4.3 Headgear rigidiser

As shown in Figs. 6A to 9D and Figs. 11A to 15D, in some forms, the positioning and stabilising structure 3300 comprises a headgear rigidiser, e.g. a rigidiser arm 3330 and/ or vent module 3420. For example, in Figs. 6A to 9D and Figs. 11A to 12E the positioning and stabilising structure 3300 comprises a rigidiser arm 3330. In the examples of Figs. 11A to 12E, the vent module 3420 acts as a headgear rigidiser. In another example, in Figs. 13A to 15D, the positioning and stabilising structure 3300 comprises a vent module 3420 which acts as the headgear rigidiser. In these forms, the vent module 3420 may be considered to act as the rigidiser arm 3330.

### 4.3.4.4 Connectors

In some forms, the patient interface 3000 comprises a pair of connectors 3800 to facilitate attachment of the plenum chamber 3200 to the positioning and stabilising structure 3300.

According to aspects of the present technology, as illustrated in Figs. 6A to 12E, the connectors 3800 may be comprised as part of the vent structure 3400. For example, a portion of the vent module 3420 may be configured to provide the connector 3800. These aspects of the present technology will become more apparent from the description of the forms below.

In certain forms the patient interface 3000 may comprise other types of connectors. For example, in the form illustrated in Figs. 10A to 10C and Figs. 13A to 15D, the plenum chamber 2300 comprises a pair of connectors 3214 configured to connect the plenum chamber 2300 to the conduit portion 3320. The connectors 3214 may be provided to either lateral side of the frame portion 3210. As illustrated, each connector 3214 is provided to a respective laterally projecting connection portion 3212. The connectors 3214, in the illustrated examples, are provided at the opening 3240 of the laterally projecting connection portions 3212.

The patient interface 3000 may have connectors 3214 which also act as connectors 3800, i.e. the connectors that connect the plenum chamber 2300 to the conduit portions 3320 may also act to connect the patient interface 3000 to the positioning and stabilising structure 3300, for example, since the conduit portions 3320 form part of the positioning and stabilising structure 3300. This is the case in the form shown in Figs. 13C and 15D, for example. Here the conduit portion 3320 is formed as part of the positioning and stabilising structure 3300 which is connected to the patient interface 3000 using connectors 3214. Alternatively, the patient interface may have connectors 3214 for connecting the plenum chamber 2300 to the conduit portions 3320 that are separate to the connectors 3800 that connect the patient interface 3000 to the positioning and stabilising structure 3300. In other forms, the connectors 3214 may connect the vent structure 3400, e.g. vent module 3240, to the plenum chamber 3200 and the positioning and stabilising structure 3300 is connected to the patient interface 3000 by separate connectors 3800. In the example illustrated in Figs. 10A to 10C, the connectors 3214 are configured to connect with the vent module 3420, and the vent module 3420 comprises a separate connector 3800 which connects to the headgear strap 3310 in use, as will be described in more detail below.

### 4.3.4.5 Attachment of headgear strap to connector

Referring to Figs. 6A to 6D and Figs. 10A to 10C, the headgear strap 3310 may be removably attached to the connector 3800. For example, in the example of Fig. 10A to 10C, the headgear strap 3310 (not illustrated in these figures) may be removably attached to the connector 3800 which comprises a slot 3820. In the example of Fig. 6A to 6D, the headgear strap 3310 may be removably attached to the connector by buttoning.

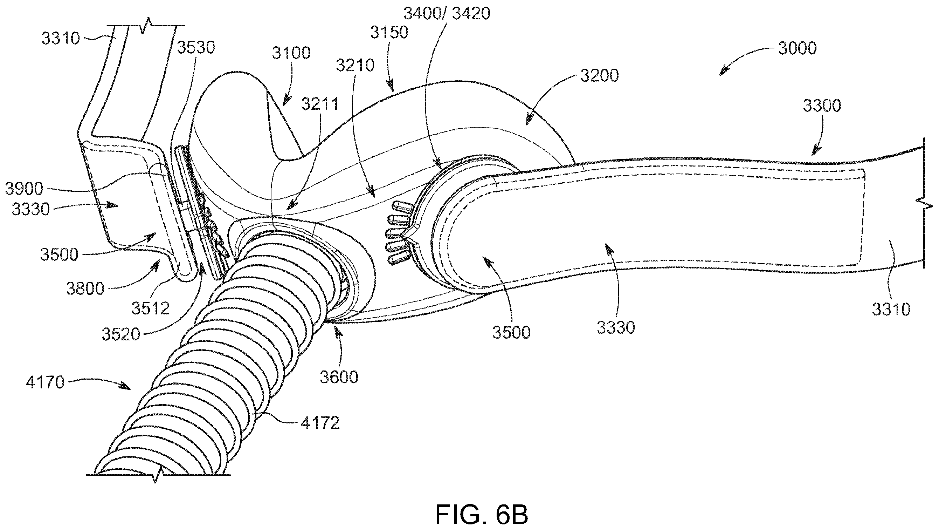

Referring to Fig. 6C-1, the headgear strap 3310 may comprise a button-hole 3312. In this form the headgear strap 3310 comprises a tube- or sleeve-like configuration to receive the insertion of a portion of the connector 3800 into the at least one headgear strap 3310 via the button-hole 3312. Therefore, when connected, a portion of the headgear strap 3310 may encapsulate the connector 3800. Referring to the illustrated form of Figs. 6B, the rigidiser arm 3330 or part of it may act as the connector 3800, which may be connected to the headgear strap 3310, e.g. encapsulated by it. In these forms, the headgear strap 3310 may be removably attached to the rigidiser arm 3330.

As shown in Fig. 6C-1, the button-hole 3312 may be located at the inner surface of headgear strap 3310 and be adapted to receive the connector 3800, e.g. the rigidiser arm 3330, such that it may be inserted into or removed from the interior of headgear strap 3310 which as described above may have a tube- or sleeve-like configuration. The button-hole 3312 may be oriented and/or shaped such that the rigidiser arm 3330 may be inserted and/or removed through such button-hole 3312 in order to assemble the positioning and stabilising structure 3300 while still preventing accidental removal or separation of the rigidiser arm 3330 from the strap 3310 during use. As illustrated in Fig. 6C-1, the button-hole 3312 may have a slit-like configuration, which may be oriented longitudinally or transversely relative to the length of headgear strap 3310. In other words, the elongate extension of the button-hole 3312 may extend substantially coaxial to the longitudinal axis of both headgear strap 3310 and the rigidiser arm 3330. This allows, particularly due to the elasticity of headgear strap 3310, an easy insertion of the rigidiser arm 3330 into the tube- or sleeve-like headgear strap 3310 or part of the headgear strap 3310 while, at the same time, preventing its accidental removal. An end portion of the headgear strap 3310 between the distal tip of the headgear strap 3310 and the button-hole 3312 may wrap over the edge of the rigidiser arm 3330 and functions as an anchor point. This edge of the rigidiser arm 3330 or anchor point may act as a catching member. This end portion of the headgear strap 3310 may also be referred to as the pocketed end 3311. This prevents the headgear strap 3310 from slipping off the inserted rigidiser arm 3330 when the headgear strap 3310 is stretched and adjusted while donning or doffing the patient interface 3000.

### 4.3.5 Vent structure

A patient interface 3000 according to some examples of the present technology comprise a vent structure 3400 configured to allow a flow of gases exhaled by the patient from an interior of the plenum chamber 3200 to ambient. The vent structure 3400 may be configured such that the vent flow rate has a magnitude sufficient to reduce rebreathing of exhaled CO2 by the patient while maintaining the therapeutic pressure in the plenum chamber 3200 in use.

As described above, in the forms of the present technology illustrated in Figs. 6A to 23, the vent structure 3400 may be configured to vent a flow of gases from the interior of the plenum chamber 3200 in a substantially lateral direction in use.

As illustrated in Figs. 6A to 23, each vent structure 3400 is located lateral to the inlet 3220 on the lateral portions or sides of the patient interface 3000, e.g. on the lateral portions 3213 of the plenum chamber 3200.

In some forms, for example those shown in Figs. 10A to 11C, Figs. 13A to 13C and Figs. 15A to 15C, the patient interface 3000 may comprise another vent structure 3400A configured to vent another flow of gases from the interior of the plenum chamber 3200. The vent structure 3400A may be configured to vent the flow of gases in a substantially anterior direction in use. The vent structure 3400A may be located proximate the inlet 3220 which is located in the medial region of the plenum chamber 3200. The vent structure 3400A may be comprised as part of the connection port 3600 which is attached to the inlet 3220.

In preferred forms, the vent structure 3400 comprises a plurality of vent holes 3410. In the embodiments shown in Figs. 6A to 9D,Figs. 11A to 15D, and Figs. 17 to 23, the vent structure 3400 may comprise a vent wall 3412 comprising the vent holes 3410. In some forms, as illustrated in Figs. 6A, 8B, 9B, 11C, the diameter of an inlet opening of each vent hole 3410 may be larger than the diameter of an outlet opening of each vent hole 3410. Therefore, opposing internal walls of each vent hole 3410 may converge toward each other.

In the embodiment of Figs. 10B to 10C, a vent hole 3410 is provided in the form of a flow path, e.g. a channel, defined by an arrangement of vent walls 3414. In other words, in this example, the vent structure 3400 may not comprise multiple discrete vent holes 3410 formed in a vent wall 3412.

### 4.3.5.1 Vent structure location

As illustrated in Figs. 6A to 23, the vent structures 3400 are located in lateral regions of the patient interface 3000, for example lateral to the medial region of the plenum chamber 3200. The vent structures 3400 may face in a substantially lateral direction in use, i.e. be provided on a surface at least partly angled in the lateral direction.

It is typical in prior art patient interfaces or masks for the vent or vent structure to be located in an anterior region, particularly in a medial region of the mask, and for the inlet which connects to the air delivery tube to be located proximate the vent in the same region of the mask. In other words, the vent and inlet are typically located in the front and/or middle regions of the mask. As illustrated in the prior art mask 3000 of Fig. 6, during exhalation, the air entering the mask 3000 from the air circuit 3700 through the inlet 3220 collides with exhaled air from the patient and is diverted through a significant angle, approximately 180° in the case of Fig. 6, to exit the mask 3000 through the vent 3400. In some configurations, this can cause a large amount of shearing and contact between the air flow entering through the inlet 3220 and the air being exhaled through the patient's nares, as indicated by the arrows in Fig. 6. During inhalation or breath pause, the air entering the plenum chamber 3200 through the inlet 3220 and/or the air flow as the patient inhales, collides with air within the plenum chamber 3200. The air within the plenum chamber 3200 may comprise air exhaled by the patient, which flows at a significant angle relative to the direction of flow of air entering the plenum chamber 3200, e.g. at relative angles of approximately 180°. These two streams of air collide, which causes shearing between the air flow entering through the inlet 3220 and the air inside the mask 3000, which may include air exhaled by the patient. This shearing and contact increases turbulence and cyclic noise produced by the patient interface 3000 during use. The amount of shearing and contact, and consequently the amount of turbulence and noise, may be larger during inhalation than during exhalation. This may be because the volume and/or velocity of the air flow entering the mask 3000 through the inlet 3220, e.g. the air flow provided by the air delivery tube 4172, during inhalation, may be larger than the volume and/or velocity of air flow during exhalation. This relatively large incoming air flow during inhalation helps maintain the positive pressure inside the mask 3000 whereas, during exhalation, less air is required to enter through the inlet 3220, e.g. from the air delivery tube 4172, in order to maintain the pressure inside the mask 3000.

In comparison to the prior art, the arrangement of vent structures 3400 of the present technology reduces static sound power and cyclic noise by reducing, in comparison to prior art masks such as illustrated in Fig. 6, the angle that the direction of the flow of gases is diverted between entering the interior of the plenum chamber 3200 via the inlet 3220 and exiting from the interior of the plenum chamber 3200 via the vent structure 3400. For example, the vent arrangement of the prior art mask illustrated in Fig. 6 diverts the air by an angle of approximately 180° (i.e, air enters the plenum chamber 3200 in a roughly posterior direction, and air vents through the vent structure 3400 in a roughly anterior direction). In contrast, in forms of the present technology, the vent arrangement, e.g. location and/or orientation of the vent structures 3400, on the patient interface 3000 is/are configured to reduce the angle that the direction of the flow of gases is turned or diverted from entry to exit. For example, the location and/or orientation of the vent structures 3400 may reduce the angle that the direction of the flow of gases entering the plenum chamber 3200 through the inlet 3220 is turned or diverted to approximately 60°. This may reduce the shearing and turbulence between the air entering the plenum chamber 3200 and the exhaled air, and therefore reduces the noise. The vent arrangement of the present technology may also split the air flow during inhalation as explained in more detail below.

In forms of the present technology with the air circuit 4170 attached to the plenum chamber 3200, as shown in the examples of Figs. 6A to 15D, the vent structures 3400 are laterally spaced apart from the inlet 3220, as opposed to being located proximate to the inlet 3220 on either side thereof. In the example of Fig. 22, the vent structures 3400 are located proximate the lateral edges of the inlet 3220, however, as described herein, the connection port 3600 provided on the frame 3360 effectively reduces the size of the inlet 3220. Therefore, in this example, the vent structures 3400 are also laterally spaced apart from the connection port 3600. Locating the vent structures 3400 further from the inlet 3220 and/or connection port 3600 on the sides of the patient interface 3000 may reduce the angle that the direction of the flow of gases is diverted from entry into the plenum chamber 3200 to exit from the plenum chamber 3200, which then reduces the air shearing, turbulence, and noise. Referring to Figs. 9B, 10B, 11C, 12C, 13C, and 15D, the location and/or orientation of each vent structure 3400 may reduce the angle that the direction of the flow of air is diverted before the flow of air exits the vent structure 3400 in comparison to prior art designs. This may reduce shearing, turbulence and noise compared to the prior art. Sharper turns or diversion, or greater turning or diversion angles of the flow of air may cause more venting noise, as illustrated in the prior art mask of Fig. 6. Locating the vent structure 3400 laterally further from the inlet 3220 and/or connection port 3600 may reduce turbulence caused by mixing of the flow of gases entering the plenum chamber 3200 from the air circuit and the flow of gases entering the plenum chamber 3200 from the patient's nares during exhalation. Turbulence caused close to the vent structures 3400 may negatively impact patient interface acoustics because the noise is produced at the vent structures 3400 and there is limited insulation or absorption of the noise before it reaches the patient or their sleeping partner. In prior art patient interfaces where the location of vent structures is proximate the inlet, such as that shown in Fig. 6, it has been observed that the flow of gases entering the plenum chamber from the air circuit and from the patient's nares collides and causes turbulence that is close to the vent structure. The vent arrangement of forms of the present technology may be divided into two separate, spaced apart vent structures 3400 that direct the flow of gases entering the plenum chamber 3200 from the air circuit 4170 and the patient's nares in separate arcs towards the respective vent structures 3400, as illustrated by the arrows in Figs. 9B, 10B, 11C, 12C, 13C and 15D. This may help diffuse of the flow of gases. It is to be appreciated that the examples of Figs. 17, 18 and 22 have a similar vent arrangement.

The vent structure 3400 may be located on the plenum chamber 3200, for example the frame portion 3210. As shown in Figs. 6A to 9D, Figs. 11A to 12E, and Figs. 18, 20 and 22, the vent structures 3400 may be located on a portion of the plenum chamber 3200 which is proximate to a lateral end of plenum chamber 3200.

In the forms of the technology shown in Figs. 10A to 10C and Figs. 13A to 15D, the vent structures 3400 may be provided to a part of the patient interface 3000 that is located lateral to the plenum chamber 3200 in use. As shown, the vent structures 3400 may also be located lateral to the cushion module 3150 in use. The vent structures 3400 may be spaced apart from the lateral ends of the plenum chamber 3200, e.g. spaced apart from the lateral ends of the cushion module 3150. In these forms, a part of parts of the positioning and stabilising structure 3300 may comprise the vent structures 3400, as described further below.

### 4.3.5.2 Vent module

In some forms, as shown in Figs. 9A to 13C and Figs. 15A to 15D, each vent structure 3400 comprises a vent module 3420. The vent module 3420 is a component which may be formed separately and which is connected to other components of the patient interface 3000 during manufacture or use. For example, the vent module 3420 may be attached to a part of the patient interface 3000, e.g. the plenum chamber 2300, during manufacture. The vent module 3420 may be permanently or removably attached to the patient interface 300. In examples where the vent module 3420 may be removable from the patient interface 3000, this may assist with cleaning or replacing the vent module 3420.

As shown in Figs. 9A to 13C and Figs. 15A to 15D, the vent module 3420 may comprise the vent wall 3412 comprising one or more vent holes 3410 through which air is vented laterally from the patient interface 3000, and one or more other walls. In some examples, the one or more other walls may form the conduit portion 3320.

The vent module 3420 may be configured to attach to a portion of the plenum chamber 3200 comprising the openings 3240 or vent module openings. In some forms, as illustrated in Figs. 10B, 10C, 13C and 15D, the laterally projecting connection portions 3212 comprise the openings 3240. In some forms, as illustrated in Figs. 6C, 6D, 8B, 9B, 11C, 12C, the openings 3240 are provided to the frame portion 3210, for example, the openings 3240 are provided to the anterior portion 3211. The openings 3240 may be provided to lateral regions of the anterior portion 3211, wherein the lateral regions are lateral to the inlet 3220. The lateral regions may face in a substantially lateral direction, or be angled in a lateral direction.

In some examples which are illustrated in Figs. 6A to 15D, the plenum chamber 3200 comprises a rigidiser. The rigidiser may be in the form of the vent module 3420 and/or the connection port 3600. The rigidiser may act to add rigidity to the plenum chamber 3200. For example, the frame portion 3210 is rigidised by the vent module 3420 and/or the connection port 3600. The rigidiser is structured to be a relatively rigid component, for example by being formed of a material having a relatively high modulus of elasticity and/or by being formed in a relatively rigid shape.

### 4.3.5.3 Positioning and stabilising structure comprising vent structure

In some forms of the present technology, for example, as illustrated in Figs. 13A to 15D, the positioning and stabilising structure 3300 comprises the vent structure 3400. In the illustrated examples of Figs. 13A to 15D, the positioning and stabilising structure may comprise the conduit portion 3320 and, in turn, the conduit portion 3320 comprises the vent structure 3400.

As shown in Figs. 13C and 15D, the plenum chamber 3100 may comprise lateral openings 3240 and the conduit portion 3320 is configured to be, in use, in fluid communication with an interior of the plenum chamber 3100 through the lateral openings 3240. Each conduit portion 3320 may connect to each connector 3214 provided to each opening 3240.

In the examples of Figs. 13C and 15D, the headgear strap 3310 may be comprised as part of the conduit portion 3320. For example, the headgear strap 3310 may be permanently attached to the conduit portion 3320. As illustrated in Figs. 13B and 13C, 15C, and 15D, at least a portion of the headgear strap material covers at least a portion of the conduit portion 3320 and may additionally cover at least a portion of the vent structure 3400. The portion of the headgear strap material that covers the vent structure 3400 may act to diffuse the flow of vented air, i.e. it may form the diffuser 3900. In these embodiments, the conduit portion 3320 may be considered to be comprised as part of the positioning and stabilising structure 3300. As illustrated in Figs, 13A to 15D, the conduit portion 3320 is comprised as part of the vent module 3420.

As shown in Figs. 13A, 14A, and 15A, an inferior end section of the positioning and stabilising structure 3300 is connected to the plenum chamber 3200 in use, and a superior end section of the positioning and stabilising structure 3300 is located on a top and/or rear region of the patient's head in use. The conduit portion 3320 may comprise the inferior end section of the positioning and stabilising structure 3300. Therefore, the vent structure 3400 may be located proximate to the inferior end section in use.

In the example of Figs. 13A to 13C, the patient interface 3000 comprises a deflector 3500 as described above. In the example of Figs. 15A to 15D, the patient interface 3000 may be without the deflector 2500 as described above. However, in some forms, as shown in Figs. 15C and 15D, the vent structure 3400, e.g. vent module 3420, may comprise a posterior wall 3514 which is configured to redirect some of the vented air.

In some forms, such as those illustrated in Figs. 13A to 15D, the conduit portion 3320 may be configured to be relatively rigid, for example by being formed from a material having a relatively high modulus of elasticity or by being formed in a shape that promotes rigidity. In some forms, the conduit portion 3320 may be formed from a relatively flexible material and the conduit portion 3320 may comprise a support structure (not shown in the Figures) to add rigidity and to limit and/or prevent occlusion of the conduit portion 3320, which may prevent or restrict air being vented to ambient. For example, this may help limit and/or prevent occlusion by forces applied when the patient is lying on their side. The support structure may be provided by one or more of a reinforcing structure, a thickened region, and a stiffened region.

### 4.3.5.4 Plenum chamber comprising vent structure

In some forms of the present technology, for example, as illustrated in Figs. 17 to 23, the plenum chamber 3200 comprises the vent structure 3400. For example, the vent structure 3400 may be provided lateral to the medial region of the plenum chamber 3200, i.e. in lateral regions of the plenum chamber 3200.

As is perhaps best shown in Fig. 20, the vent holes 3410 may be formed on a lateral portion 3213 of the frame portion 3210 of the plenum chamber 3200. In such forms, the vent wall 3412 is provided by the plenum chamber 3200.

### 4.3.6 Deflector

In some forms of the present technology, for example, as illustrated in Figs. 6A to 13C, the patient interface 3000 comprises a deflector 3500, configured to redirect or turn at least a portion of the vented flow of gases. In certain forms, the patient interface 3000 comprises a pair of deflectors 3500, the deflectors 3500 being arranged, for example symmetrically arranged, on either side of the patient interface 3000.

### 4.3.6.1 Redirecting vented flow of gases in anterior direction

In some forms of the present technology, for example as illustrated in Figs. 6A to 13C, each deflector 3500 is configured to redirect a portion of the laterally vented flow of gases in any direction having an anterior component relative to the patient. That is, the flow of gas redirected by the deflector 3500 has a velocity, which is a vector quantity having a magnitude (speed) and direction. In some forms, a generally forwards direction will be understood to be where the velocity vector of the redirected flow of gas has a positive component in the anterior direction relative to the patient. The velocity vector of the redirected flow of gas may also have a positive component in a perpendicular direction to the anterior direction, e.g. the superior, inferior or lateral directions. For example, the velocity vector may be in any of a superior-anterior direction, an inferior-anterior direction, a superior-anterior-lateral direction or an inferior-anterior-lateral direction.

For the purposes of the ensuing description, when referring to the direction of vented flow of gas redirected by the deflector 3500, any direction having an anterior component relative to the patient (i.e. as discussed above) will be referred to as "an anterior direction" (which is intended to be distinguished from "the anterior direction") for convenience.

In some forms, the direction of the velocity vector of the redirected flow of gas may be in a substantially anterior direction. A substantially anterior direction may be understood to mean a direction in which the largest component of the velocity vector in the frame of reference of the mutually perpendicular axes of the body is the anterior direction. That is, the redirected flow of gas may be more in the anterior (forwards) direction than any other direction.

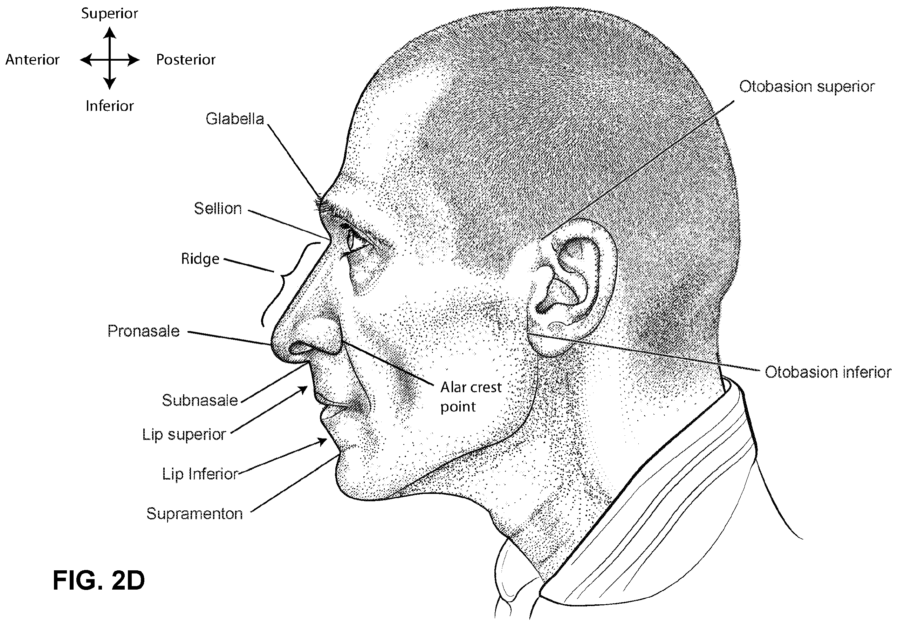

In some forms, each deflector 3500 is configured to redirect a substantial portion of the laterally vented flow of gases in an anterior direction in use. In some forms, each deflector 3500 is configured to redirect all of the laterally vented flow of gases in an anterior direction in use. The anterior direction is represented in Fig. 2D. In other words, the deflector 3500 is configured to redirect a portion, preferably at least a substantial portion, of the air vented sideways in a forward direction away from the patient's face in use.

The deflectors 3500 may be configured to redirect the laterally vented air towards an anterior portion positioned on the mid-sagittal plane of the patient's face in use. In some forms, the deflectors 3500 may be configured to redirect the substantial portion of the laterally vented flow of gases towards a portion of the air delivery tube 4172 in use. These aspects of the present technology will be described in more detail below.

In the illustrated examples of Figs. 6A to 13C, each deflector 3500 comprises an arrangement of one or more deflector walls 3512, 3514, 3516, 3518.

Referring to the examples illustrated in Figs. 6A to 13C, at least a portion of at least one of the deflector walls 3512, 3514, 3516, 3518 is configured to be positioned in the path of the flow of air being vented laterally from at least a portion of the vent structure 3400 in use. This may be referred to as the deflector 3500 "covering" the vent structure. It will be appreciated that the deflector 3500 may be spaced apart from the vent structure 3400 while being located in the flow path of the air being vented from the vent structure 3400. The at least one wall may be a lateral wall 3512 configured to limit / prevent the vented air from flowing in a lateral direction, i.e. in the same direction the air is vented in, which is away from the patient's face in a generally sidewards direction.

As shown in Figs. 6C, 6D, 8B, 9B, 11C, 12C, 13C, the lateral wall 3512 has a vent-facing surface 3512a which is spaced apart from a lateral-facing surface 3412a of the vent wall 3412 in use. This provides a gap 3520 between the lateral wall 3512 and the vent wall 3412 through which the air can exit.

In some forms, as shown in Figs. 6A to 9D and Figs. 13A to 13C, a portion of the lateral wall 3512 which covers a portion of the vent structure 3400 may extend in a direction substantially perpendicular to the direction in which the air is vented from the vent structure 3400. In some forms, as shown in Figs. 6A to 9D and Figs. 13A to 13C, a portion of the lateral wall 3512 which covers a portion of the vent structure 3400 may extend in a direction substantially parallel to the direction in which a surface of the vent wall 3412 extends.

In some forms, the deflector 3500 comprises one or more other deflector walls, e.g. posterior wall 3514, superior wall 3516, inferior wall 3518. The posterior wall 3514 may be configured to limit / prevent air from flowing in a posterior direction, i.e. rearwards direction, for example, towards the patient's face, eyes and/or ears. The superior wall 3516 may be configured to limit / prevent air from flowing in a superior direction, i.e. upwards direction, for example, up along the patient's face. The inferior wall 3518 may be configured to limit / prevent air from flowing in an inferior direction, i.e. downwards direction, for example, down along the patient's face. For example, in some forms, the flow of gases is meant to flow equally out of the gap 3520 between the deflector walls 3512, 3514, 3516, 3518 and vent wall 3412. The distribution of redirected air may be equal as it exits the gap 3520. The gap 3520 may be configured to surround a portion of the vent structure 3400. The gap 3520 may be configured to surround a substantial portion of the vent structure 3400. The gap 3520 may be configured to permit the flow of air such that the angle A between the direction of a superior-most flow of gases and the inferior-most flow of gases may be between approximately 0° and approximately 280°. Fig. 9A illustrates the angle A according to one form of the present technology. The gap 3520 may be configured to permit the flow of air in an anterior direction such that the angle A between the direction of a superior-most flow of gases and the inferior-most flow of gases may be about 0° to about 280°, preferably about 0° to about 180°, and in some examples, about 0° to about 120°, about 0° to about 100°, about 20° to about 180°, about 30° to about 120°, about 10° to about 90°, or about 45° to about 90°.

In some forms, the gap 3520 may be configured such that the angle A may limit / prevent the concentration of the flow of air, and promote some diffusion thereof. In some forms, the gap 3520 may be configured such that the angle A may improve patient comfort during use by limiting / preventing the flow of air towards the patient, for example, towards the patient's face, eyes and/or ears. The flow of air may have a temperature which is lower than ambient, e.g. cool or cold air, and this may cause discomfort to the patient if the flow of air contacts their face, eyes, ears or other temperature sensitive regions. Therefore, the gap 3520 may be configured to provide the angle A between the direction of a superior-most flow of gases and the inferior-most flow of gases that is large enough to limit / prevent the concentration of the flow of air and promote some diffusion, but small enough to limit / prevent patient discomfort resulting from the flow of air making contact with the patient.

The other deflector walls 3514, 3516, 3518 may extend from the lateral-facing surface 3412a. For example, as shown in Figs. 6A to 13C, the other deflector walls 3514, 3516, 3518 may extend in a direction which is substantially perpendicular to the direction in which the lateral-facing surface 3412a extends. The gap 3520 is provided between the other deflector walls 3514, 3516, 3518, the lateral wall 3512 and the vent wall 3412.

In examples, as illustrated in Figs. 6A to 13C, the deflector 3500 may comprise a spacer 3530 to position the lateral wall 3512 above the vent wall 3412 in use. In examples, the spacer 3530 may extend from one of the lateral-facing surface 3412a and the vent-facing surface 3512a. The spacer 3530 may extend in a direction substantially perpendicular to the direction the lateral-facing surface 3412a extends in.

One or more of the other deflector walls 3514, 3516, 3518 may provide the spacer 3530.

In some forms, such as those shown in Figs. 6A to 13C, the deflector 3500 or at least a part of it, e.g. one or more of the deflector walls 3512, 3514, 3516, 3518, may be comprised as part of the vent structure 3400. In some forms illustrated by the examples shown in Figs. 6A to 13C, the deflector 3500 or at least parts of it may be comprised as part of the connector 3800, e.g. one or more of the deflector walls 3512, 3514, 3516, 3518, may be comprised as part of the connector 3800. In some forms, the connectors 3800 are comprised as part of the vent structure 3400. For example, referring to the embodiments of Figs. 6A to 12E, a portion of each vent module 3420 is configured to provide the connector 3800.

### 4.3.6.2 Air impermeable deflector

In certain forms of the technology, such as those illustrated in Figs. 6A to 10C, the deflector 3500 is air impermeable. As shown in Figs. 9A, 9B, 10B, 10C, the lateral wall 3512 deflects the vented air as it exits from the deflector 3500 through the gap 3520 in the directions indicated by the arrows, which have been included for illustrative purposes.

As shown in the example of Figs. 10B and 10C, the deflector walls 3512, 3514, 3516, 3518 are arranged to form a turning portion 3414 which turns or redirects the air which is laterally vented through the vent hole 3410 from the interior of the plenum chamber 3200 in an anterior direction. In the illustrated form of Figs. 10A to 10C, the vent module 3420 is constructed such that the deflector walls 3512, 3514, 3516, 3518 are spaced apart from the vent wall 3412. The air may be turned approximately 180° by the deflector 3500 of this example. Referring to Figs. 10A to 10C, the vent module 3420 comprises a connector 3800. For example, the connector 3800 may be configured on a lateral end of the vent module 3420. The connector 3800, in the illustrated example of Fig. 10A to 10C, is a slot 3810 configured to receive a portion of the headgear strap 3310 in use to connect it to the patient interface 3000.

In the illustrated form of Figs. 6A to 6D, the deflector 3500 does not include the other deflector walls 3514, 3516, 3518. It comprises a lateral wall 3512 and a spacer 3530. In this form, vented air may be directed in a posterior, superior, and/or inferior direction, in addition to an anterior direction. In these forms, an inferior surface of the spacer 3530 may act as a superior wall for the vent holes 3410 located below the spacer 3530, and a superior surface of the spacer may act as an inferior wall for the vent holes located above the spacer 3530.

In some forms, as illustrated in Fig. 7A to 9D, the deflector 3500 may comprise one or more of the other walls 3514, 3516, 3518. For example, the deflector 3500 comprises the posterior wall 3514. The posterior wall 3514 may have a substantially arcuate or curved shape when viewing the patient interface 3000 from a substantially lateral direction, i.e. from the side. The posterior wall 3514 may extend to form at least part of an inferior wall 3518 and superior wall 3516, as shown in Figs. 8A to 8C. In Figs. 7A to 8C, the superior wall 3516 and inferior wall 3518 may extend across a part of the respective superior and inferior regions of the vent structure 3400. Therefore, some air may be redirected upwards and downwards through the gap 3520 between the lateral wall 3512 and the vent wall 3412. However, referring to Figs. 9A to 9D, the superior wall 3516 and inferior wall 3518 may extend across a substantial part of the respective superior and inferior regions of the vent wall 3412. Therefore, the amount of air redirected upwards and downwards may be limited / prevented. This may help concentrate the flow of air which is redirected in the substantially anterior direction.

In the examples of Figs. 6A to 9D, the connector 3800 is comprised as part of the deflector 3500, i.e. the deflector 3500 is configured to act as the connector 3800 to connect the positioning and stabilising structure 3300 to the patient interface 3000.

In the forms shown in Figs. 6A to 9D, the positioning and stabilising structure 3300 comprises a headgear rigidiser. The rigidiser may comprise rigidiser arms 3330, for example on rigidiser arm 3330 on each side of the patient interface for connection to respective headgear straps. The deflector 3500 may be comprised as part of the rigidiser arm 3330, and therefore, part of rigidiser arm 3330 acts as the deflector 3500 and connector 3800. In these forms, the lateral wall 3512 is comprised as part of the rigidiser arm 3332.

In Figs 8A to 9D, the rigidiser arm 3330 is removably attached to the connector 3800, for example, by a snap-fit. The rigidiser arm 3332 comprises an opening 3332 which engages with a projecting portion 3810 of the connector 3800 in order to connect the rigidiser arm 3332 to the patient interface 3000. Therefore, part of the deflector 3500, e.g. the lateral wall 3512, is removably attached to the connector 3800.

In the forms of Figs. 6A to 9D, the headgear strap 3310 may be attached to the rigidiser arm 3330. In the examples of Figs. 6A to 8C, the headgear strap 3310 is attached to the deflector 3500. A portion of the headgear strap 3310 may cover the vent-facing surface 3512a. As illustrated, the headgear strap 3310 may be positioned in use to diffuse the vented air. Although not shown in Figs. 9A to 9D, in this form the headgear strap 3310 is attached to the rigidiser arm 3330, but does not cover the vent-facing surface 3512a. As illustrated, a diffuser 3900 may be attached to the vent-facing surface 3512a such that it is positioned in use to diffuse the vented air. These aspects of the present technology will be described in more detail below.

In Figs. 6A to 6D, the headgear strap 3310 is removably attached to the deflector 3500, for example, by using the button-hole attachment mechanism described above. It is also to be appreciated that mechanical fasteners or snaps, or other suitable attachment mechanisms may be used to removably attach the headgear strap 3310 to the deflector 3500 in other forms.

Referring to the forms of Figs. 8A to 9D, the headgear strap 3310 may be permanently attached to the deflector 3500. For example, welding, laminating, over-moulding, adhesives may be used to achieve permanent attachment.

In some forms, one or more of the deflector walls 3512, 3514, 3516, 3518 and/or the spacer 3530 may be integrally formed as part of the vent module 3420. The vent module 3420 may be attached to a portion of the plenum chamber 3200 which forms the periphery of the opening 3240. In one form shown in Figs. 6A to 6D, the lateral wall 3512, vent wall 3412 and spacer 3530 are integrally formed as the vent module 3420. In another form shown in Figs. 8A to 9D, the vent wall 3412 and the posterior wall 3414, superior wall 3416 and inferior wall 3418 are integrally formed as the vent module 3420.

In some forms, the patient interface 3000 may comprise one or more ribs 3430 provided to or proximate the vent structure 3400 and/or the deflector 3500. For example, Figs. 9A to 9C show a patient interface 3000 in which one or more ribs 3430 are provided to the vent wall 3412. The ribs 3430 may extend outwardly from the surface of the vent wall 3412 and may have lengths extending along the surface of the vent wall 3412 from a posterior region towards an anterior region. The ribs 3430 may provide a plurality of flow paths 3432, e.g. channels, which may help diffuse the flow of air exiting the deflector 3500.

### 4.3.6.3 Deflector with air permeable portions

In some forms, a portion of the deflector 3500 may comprise an air permeable portion 3512b, as shown in Figs. 11A to 13C. For example, the lateral wall 3512 may comprise the air permeable portion 3512b. In some forms, the air permeable portion 3512b may be provided in addition to an air impermeable portion 3512c of the deflector 3500.

As shown in the example of Figs. 11A to 13C, the deflector 3500 may be configured to operate in a similar way as the deflector 3500 of other forms, as described above. It differs in that it includes the air permeable portion 3512b, e.g. in the lateral wall 3512, which may permit some air to permeate therethrough. The air permeable portion 3512b may act to diffuse the vented air flow as part of the flow of air permeates through it. The air permeable portion 3512b may also redirect a part of the flow of air such that it exits the deflector 3500 through the gap 3520. In the examples shown in Figs. 11A to 13C, air exits the deflector 3500 through the gap 3520, and an amount of air may exit through the air permeable portion 3512b. Substantially more air may exit the gap 3520 compared to the air exiting the air permeable portion 3512b. For example, as illustrated in Figs. 11A to 13C, the air permeable portion 3512b is formed from an air permeable material, e.g. a textile material such as headgear strap material, which also acts as a diffuser 3900. Aspects of the diffuser 3900 will be described in more detail below.

In the form of Figs. 11A to 13C, the ribs 3430 may space the headgear strap material apart from the lateral-facing surface 3412a at a predetermined distance (e.g. the height of the ribs). The ribs 3430 may help limit / prevent the air permeable material from sagging towards the vent structure 3400 in use. This may help limit / prevent occlusion of the vent structure 3400 when the headgear strap material becomes water-logged and/or blocked. The spacer 3530 may provide one or more of the ribs 3430, as illustrated in Figs. 11A to 12E.

In the examples illustrated in Figs. 11A to 12E, a substantial portion of the lateral wall 3512 may be air permeable. The lateral wall 3512 may comprise an air impermeable section 3512c. The air impermeable section 3512c may be configured with one or more openings 3512d. The headgear strap material which forms the air permeable portion 3512b, may be attached to the air impermeable section 3512c such that the openings 3512d are covered by the headgear strap material. In the illustrated examples, the headgear strap material may be attached to a surface of the air impermeable section 3512c which is lateral facing.

As shown in Figs. 11A to 12E, in some forms, the deflector 3500 may comprise part of the positioning and stabilising structure 3300. For example, the deflector 3500 may comprise part of the headgear strap 3310. The headgear strap 3310 may be permanently attached to the air impermeable section 3512c, for example, by welding, stitching, or adhesive.

In the form of Figs. 11A to 12E, the headgear strap may also be provided to a rigidiser arm 3330. Referring to the example of Figs. 12A to 12E, the rigidiser arm 3330 may be substantially longer than the rigidiser arm 3330 shown in Figs. 11A to 11C.

In the examples shown in Figs 13A to 13C, the entire lateral wall 3512 may be air permeable.

### 4.3.6.4 Further forms of deflector

In some forms of the present technology, as shown in Figs. 17 to 24, for example, the positioning and stabilising structure 3300 comprises the deflector 3500. That is, a part of the positioning and stabilising structure 3300 acts, in use, to deflect the flow of air flowing out of the vent structure 3400.

In these forms, the vent structure 3400 is at least partially concealed by the deflector 3500, preferably a substantial portion of the vent structure 3400 is concealed (concealed from the point of view of an observer positioned anterior to the patient interface during use). In the forms shown in Figs. 17, 18, 19, 22 and 23, the vent holes 3410 are completely concealed by the deflector 3410. Therefore, in some forms, the patient interface 3000 may comprise a substantially hidden or concealed vent structure 3400. This may improve the aesthetic appeal of the patient interface 3000 which may improve patient compliance with therapy.

The deflector 3500 in these examples has one or more of the same components as the deflector 3500 described above, e.g. a lateral wall 3512, vent-facing surface 3512a, and one or more deflector walls. The vent structure 3400 in these examples also has one or more of the same components as the vent structure 3400 described above, e.g. a vent wall 3412 and a lateral-facing surface 3412a. Similarly to the examples described above, the vent-facing surface 3512a of the deflector 3500 of these examples is spaced apart from the vent wall 3412 in use to create the gap 3520 therebetween for the flow of gases to exit through.

Similar to the examples described above, in the examples shown in Figs. 19 and 23, the vent-facing surface 3512a may extend in a direction substantially perpendicular to the direction in which the air is vented from the vent structure 3400. When viewed in cross-section from a side, as shown in Figs. 19 and 23, in some forms, the vent wall 3412 / lateral facing surface 3412a of the plenum chamber 3200 is substantially concave. When viewed in cross-section from a side, as shown in Figs. 19 and 23, in some forms, the lateral wall 3512 / vent-facing surface 3350a of the deflector 3500 is substantially convex. Therefore, the shape of the lateral facing surface 3412a and the shape of the vent-facing surface 3512a may have complimentary contours. In other forms (not shown in these figures), the lateral facing surface 3412a and/or the vent-facing surface 3512a may have any other suitable shape, for example be substantially planar.

Therefore, in some forms, a portion of the outer surface of the plenum chamber 3200 facing away from the patient in use may comprise a recess 3230. This portion may be sunken or recessed relative to surrounding or adjacent portions of the plenum chamber 3200. This may help make the patient interface 3000 less obtrusive and bulky, and may improve patient comfort and compliance. At least part of a patient interface component, e.g. the frame 3360 or headgear tube(s) 3350 as shown in Figs. 17, 18 and 22, may be positioned, in use, in the recess 3230. The concave vent wall 3412 may provide at least part of the recess 3230, as shown in Figs. 19 to 21. The recess may also help to maintain the frame 3360 or headgear tube(s) 3350 in place during use.

Similar to the examples described above, in the examples illustrated in Figs. 18 and 20, the patient interface 3000 may comprise a spacer 3530 to position the vent-facing surface 3512a of the deflector 3500 spaced apart from the lateral-facing surface 3412a of the plenum chamber 3200 in use. In examples, the spacer 3530 may extend from at least one of the lateral facing surface 3412a and the vent-facing surface 3512a. That is, the spacer 3530 may be formed from a plurality of structures. The spacer 3530 may extend in a direction substantially perpendicular to the direction the lateral-facing surface 3412a extends in.

The spacer 3530 may comprise at least one rib 3430, as shown in the examples of Figs. 18 and 20. Figs. 18 and 20 show one or more ribs 3430, for example a plurality of ribs 3430, projecting outwardly from the vent wall 3412. In an alternative form, the spacer may comprise at least one rib 3430 projecting outwardly from the vent-facing surface 3512a. In forms in which there are a plurality of ribs 3430, the ribs may be arranged in parallel to each other. In the forms of the technology illustrated in Figs. 18 and 20, the ribs are arranged to extend in a substantially superior-inferior direction when the patient interface 3000 is worn by the patient. In other forms, the ribs may be arranged in a different orientation. The spacer 3530 may comprise any other suitable component in other forms not shown, for example, the spacer 3530 may include one or more protrusions.

The ribs 3430 may form between them a plurality of flow paths, e.g. channels, to direct the flow of gases exiting the vent structure 3400 through the gap 3520. This may help diffuse the flow of gases exiting the gap 3520.

The spacer 3530, e.g. rib(s) 3430, may comprise part of the vent structure 3400. The ribs 3430 may be formed as part of the plenum chamber 3200, for example. In other forms, the spacer 3530, e.g. rib(s) 3430, may comprise part of the deflector 3500. For instance, the spacer 3540 may comprise at least one of the deflector walls.

### 4.3.6.4.1 Headgear tube comprises deflector

In some forms, as shown in the examples of Figs. 17 to 21, the positioning and stabilising structure 3100 of the patient interface 3000 comprises at least one headgear tube 3350 to convey the flow of gas from the connection port 3600 to the plenum chamber 3200 and the at least one headgear tube 3350 comprises the deflector 3500. That is, as shown in these figures, a portion of each headgear tube 3350 may act as the deflector 3500. This portion of each headgear tube 3350 is positioned in use to redirect the flow of gases exiting the vent structure 3400. In this example, this portion of each headgear tube 3350 is provided by a vent-facing surface 3350a of the respective headgear tube which forms the vent-facing surface 3512a, described above. This portion of each headgear tube 3350 may be provided by the tube portion 3354. In alternative forms, the deflecting portion of each headgear tube 3350 may be an inferior end potion of the headgear tube 3350, or a portion proximate the inferior end. In use, the deflecting portion of each headgear tube 3350 may be positioned overlaying the patient's cheek region.

In these examples, at least one headgear tube 3350 conceals the vent structure 3400 (concealed from the point of view of an observer positioned anterior to the patient interface during use).

In the illustrated example, the portion of the at least one headgear tube 3350 acting as the deflector 3350 is generally air impermeable. However, as will be explained further below, the at least one headgear tube 3350 may comprise a diffuser 3900. The diffuser 3900, which is considered part of the headgear tube(s) 3350 in these forms, may however be air permeable.

### 4.3.6.4.2 Frame comprises deflector

In some forms, as shown in the examples of Figs. 22 to 24, the positioning and stabilising structure 3100 of the patient interface 3000 comprises a frame 3360 to facilitate engagement with the plenum chamber 3200. In these forms, the frame 3360 comprises the deflector 3500. That is, as shown in Fig. 22, part of the frame 3360 acts as the deflector 3500. Part of the frame 3360 is positioned in use to redirect the flow of gases exiting the vent structure 3400. In this example, the deflecting part of frame 3360 is provided by a vent-facing surface 3360a which forms the vent-facing surface 3512a, described above. The vent-facing surface 3360a is provided on a surface of the frame 3360 facing towards the patient in use.

In these examples, the frame 3360 conceals the vent structure 3400 (from the perspective of an observer positioned anterior to the patient interface 3000).

In the illustrated example, the frame 3360 is generally air impermeable. However, as will be explained further below, the frame 3360 may comprise a diffuser 3900. The diffuser 3900 which is considered part of the frame 3360 in these forms, may however be air permeable.

It is to be appreciated that in other forms not shown, a portion of the frame 3360 may comprise an air permeable portion, similar to the air permeable portion 3512b described above. Therefore, the frame 3360 may comprise an air permeable portion and an air impermeable section in these other forms.

### 4.3.6.5 Redirecting vented flow of gases in superior and/or inferior direction

In some forms, as shown in the examples of Figs. 17 to 23, the deflector 3500 is configured to redirect, in use, at least a portion of the vented flow of gases, for example a substantial portion of the laterally vented flow of gases, in at least one of a superior direction and an inferior direction, for example a substantially superior direction and a substantially inferior direction. Reference to "a superior direction" and "an inferior direction" is intended, similar to the analogous term "an anterior direction" used above, to mean any direction having a superior or interior component respectively relative to the patient. A component extending in a superior direction may also extend in one or more other directions, and similarly, a component extending in an inferior direction may also extend in one or more other directions. For example, a flow of gases redirected in a substantially superior direction may also be redirected in a lateral, and/or anterior direction, but not an inferior direction, and similarly, a flow of gases redirected in a substantially inferior direction may also be redirected in a lateral, and/or anterior direction, but not a superior direction. These examples will be explained further below. Likewise, reference to "substantially superior direction" or "substantially inferior direction" may be understood to mean a direction in which the largest component of the velocity vector in the frame of reference of the mutually perpendicular axes of the body is the superior or inferior direction, respectively.

The shape and/or orientation of the vent-facing surface 3512a, and the shape and/or orientation of an outer surface of the plenum chamber 3200 may dictate the direction(s) the flow of gases exit through the gap 3520. In some forms, the spacer 3430 and/or the ribs 3530 may also dictate the direction(s) the flow of gases exit through the gap 3520. For instance, the convex vent-facing surface 3350a of the at least one headgear tube 3350, or the convex vent-facing surface 3360a of the frame 3360, and the concave vent wall 3412 and/or the lateral-facing surface 3412a may direct flow of gases exiting the gap 3520 in a substantially inferior direction or superior direction, or both as is shown in Figs. 19 and 23.

As illustrated in Figs. 19 and 23, while the flow of gases is redirected in a substantially superior direction and/or inferior direction, the flow of gases may also be redirected in a partially anterior direction. As shown in Fig. 22, the flow of gases may also be redirected in a partially lateral direction. Therefore, the flow of gases may be redirected in a superior-anterior-lateral direction and/or inferior-anterior-lateral direction. This may help direct the vented flow of gases away from the patient's face. This may improve patient comfort and may improve therapy compliance. Therefore, openings in the gap 3520, through which the redirected flow of gases exits, may face in superior-anterior direction and/or inferior-anterior direction.

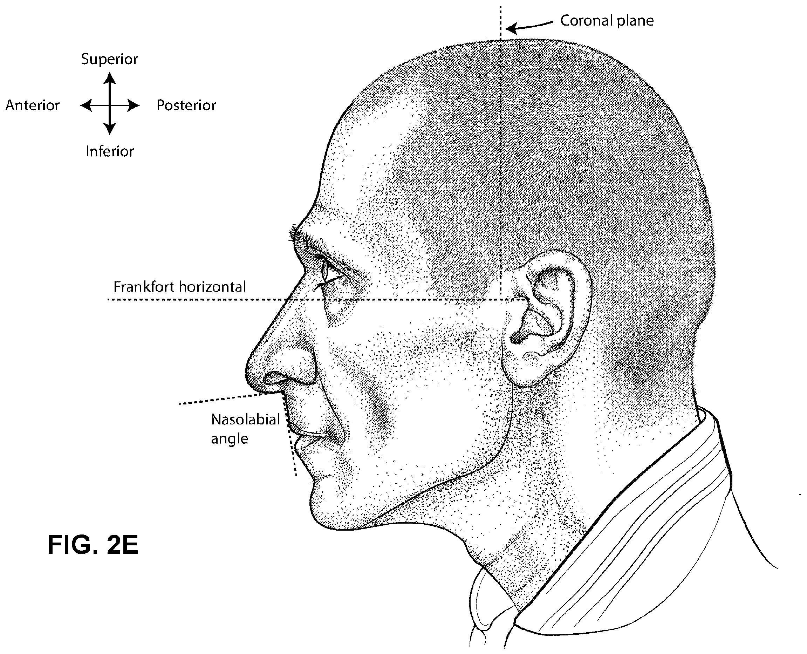

The flow path(s) created by the gap 3520 may extend in a substantially superior and/or inferior direction. However, as shown, in some forms, the flow path(s) may extend partially in an anterior direction. Furthermore, as shown, in some forms, the flow path(s) may extend partially in a lateral direction. Therefore, the flow path(s) extend in superior-anterior-lateral direction and/or inferior-anterior-lateral direction. The flow path(s) may be angled, in use, e.g. in a superior-anterior and/or inferior-anterior direction, relative to the coronal plane of the patient which is shown in Fig. 2E, and in a superior-lateral direction and/or inferior lateral direction, relative to the sagittal plane of the patient which is shown in Fig. 2C.

As shown in Figs. 18 to 20, the rib(s) 3430 may extend in a substantially superior direction. In some forms, the rib(s) 3430 extend to a region of the plenum chamber 3200 which is superior to or above the vent hole(s) 3410. As shown, the rib(s) 3430 extend in a substantially inferior direction. In some forms, the rib(s) 3430 extent to a region of the plenum chamber 3200 which is inferior to or below the vent holes 3410. The direction the ribs 3430 extend in may help limit or avoid trapping of the vented flow of gases in the gap 3520, and help to direct the vent flow in the desired direction.

As shown in Figs. 18 to 20, the rib(s) 3430 may extend between vent holes 3410. For instance, the ribs 3530 extend between a pair of vent holes 3410, as shown. In the illustrated example, the vent structure 3400 may comprise a plurality of pairs of vent holes, e.g. three pairs, which are separated by ribs 3530. However, it is to be appreciated that any number of vent holes 3400 may be provided suitable to provide adequate gas washout while maintaining therapeutic pressure in the plenum chamber 3200 during use. In addition, any number of ribs 3539 may be provided, which extend in one or more other directions.

### 4.3.7 Diffuser

A patient interface 3000 according to some examples of the present technology comprise a diffuser 3900 to diffuse the flow of gases. The diffuser 3900 may be positioned in use to diffuse the flow of gases exiting the vent structure 3400.

In some forms, the deflector 3500 may act as a diffuser 3900, at least in part. That is, the deflector 3500 may act to deflect / redirect and, in doing so, diffuse the vented flow of gases. Alternatively or additionally, the vent structure 3400 may act to vent and diffuse the flow of gases.

In other forms, for example as illustrated in Figs. 6A to 9D, Figs. 11A to 16A, and Figs. 17 to 24, the diffuser 3900 is configured to permit the flow of gases therethrough and to diffuse the flow of gases in doing so. For example, the diffuser 3900 may comprise an air permeable layer, e.g. a textile material. In the illustrated embodiment, the diffuser 3900 is positioned in the path of the flow of gases from the vent structure 3400, for example the diffuser 3900 covers at least a part of the vent structure 3400 in use, preferably the entire vent structure 3400. The diffuser 3900 may be positioned immediately in front of the vent structure 3400 or may be spaced apart from the vent structure 3400 and positioned, in use, in the flow path of the vented gases. The spacer 3530, e.g. ribs 3430, may offset the diffuser 3900 from the vent wall 3412.

The diffuser 3400 may be at least partially concealed by the deflector 3500, e.g. concealed from the perspective of an observer positioned anterior to the patient interface 3000. As shown in Figs. 17 and 22, the diffuser 3900 may be substantially concealed, as illustrated. In some forms, the vent holes 3410 are completely concealed by the deflector 3410, as shown in Figs. 18, 19 and 23. Therefore, in some forms, the patient interface 3000 may comprise a substantially hidden or concealed diffuser 3900. This may improve the aesthetic appeal of the patient interface 3000 which may improve patient compliance with therapy.

In some forms, the spacer 3530, e.g. ribs 3430, is configured to ensure that the diffuser is positioned at a selected distance from the vent wall 3412, and maintained in that position during use. The dimensions of the ribs, particularly the height of the ribs, may set this distance. This distance between the diffuser 3900 and the vent wall 3412 may limit or prevent noise in use.

In some forms, the diffuser 3900 is formed from a textile material, e.g a fleece material. The textile material may be a headgear strap material. In an example, the diffuser 3900 is formed by napping a portion of a surface of the textile material.

In some forms, for example, the forms shown in Figs. 6A to 9D, 11A to 16A and Figs. 17 to 24, the positioning and stabilising structure 3300 comprises the diffuser 3900.

In some forms, for example, the forms shown in Figs. 6A to 8C, and Figs. 11A to 15D, the headgear strap 3310 comprises the diffuser 3900.

In some forms, for example, the forms shown in Figs. 8A to 9D and Figs. 17 to 24, the positioning and stabilising structure 3300 comprises a component. The component may have a vent-facing surface 3512a which is positioned in use in the path of the vent flow of gases exiting the vent structure 3400 in use, as shown. A diffuser may be located on the vent-facing surface 3512a of the component. The component in question may, in some forms, be the frame 3360 while, in other forms, may be the headgear tube 3350.

In some forms, the diffuser 3900 may be removable from the rest of the positioning and stabilising structure 3300. This can facilitate cleaning, replacement and/or storage of components.

In some forms, the diffuser 3900 may be formed with or permanently attached to the positioning and stabilising structure 3300 which is removable from the rest of the patient interface 3000. This may reduce the number of patient interface components, and can facilitate cleaning, replacement and/or storage of components.

### 4.3.7.1 Headgear strap comprising diffuser

In the examples illustrated in Figs. 6A to 8C, and Figs. 11A to 15D, the headgear strap 3310 may comprise the diffuser 3900. That is, part of the headgear strap 3310 acts in use to diffuse the vented flow of gas.

Using the headgear strap 3310 to provide the diffuser 3900 may help reduce manufacturing costs compared to patient interfaces in which the diffuser and headgear straps are separate components. It may also reduce the number of components required to be manufactured, replaced and/or cleaned compared to such interfaces. The diffuser 3900 may be removable with the headgear strap 3310.

In exemplary forms, the headgear strap 3310 comprises a portion configured to cover at least a portion of the vent structure 3400 in use. This cover portion comprises the diffuser 3900. Therefore, the headgear strap 3310 connects to the patient interface 3000 in such a way that it covers at least a part of the vent structure 3400, preferably the entire vent structure 3400. In examples, the headgear strap 3310 may connect to the patient interface 3000 using the button-hole attachment, or the snap-fit connection described above.

In these forms, the diffuser 3900 is formed from a headgear strap material. In Fig. 16B, the diffuser 3900 is formed by napping a portion of a surface of the headgear strap material to form a napped portion 3310a. The headgear strap material may comprise a textile material.

In other forms, for example, as shown in Figs. 16C and 16D, the diffuser 3900 may be formed separately and attached to the headgear strap 3310. For example, the diffuser 3900 may be formed from a textile material. In some forms, the diffuser 3900 may be removably attached to a part of the headgear strap 3310.

### 4.3.7.2 Rigidiser arm comprising diffuser

In some forms, for example, as illustrated in Figs. 8A to 9D, the component of positioning and stabilising structure 3300, as described above, comprises a rigidiser arm 3330.

For example, in some forms, the rigidiser arm 3330 may comprise the diffuser 3900. As illustrated in Fig. 9D, in some forms, the diffuser 3900 may be formed separately, e.g. from a textile material, and attached to the vent-facing surface 3512a of the rigidiser arm 3330 such that the diffuser 3900 covers at least a portion of the vent structure 3400 in use to diffuse the flow of gases exiting the vent structure 3400. In this example, the headgear strap 3310 is attached to another portion of the rigidiser arm 3330. In other words, the headgear strap 3310 does not form the diffuser 3900 in this particular form. The diffuser 3900 may be permanently or removably attached to the vent-facing surface 3512a. Therefore, the diffuser 3900 may be replaced, or it may be removed, washed and re-attached. As already described, in some forms, the deflector 3500 may be removable from the patient interface 3000. Therefore, the diffuser 3900 may be removable with the deflector 3500.

In the embodiment illustrated in Figs. 8A to 9D, the rigidiser arm 3330 (and attached headgear straps 3310) may be removed from the connector 3800 which improves access to the diffuser 3900 for removal, and/or cleaning.

### 4.3.7.3 Headgear tube comprising diffuser

In some forms, for example, as illustrated in Figs. 17 to 21, the component of the positioning and stabilising structure 3300, as described above, comprises at least one headgear tube 3350.

In the examples illustrated in Figs. 17 to 21, the at least one headgear tube 3350 comprises the diffuser 3900. That is, part of the at least one headgear tube 3350 acts in use to diffuse the vented flow of gas.

In these exemplary forms, the headgear tube(s) 3350 comprises a portion or portions configured to be positioned in the path of the vent flow of gases exiting the vent structure 3400 in use. For example, the diffuser 3900 may be located on the cover portion of the headgear tube 3350. As shown in the example of Figs. 17 and 18, the tube portion 3354 of the headgear tubes 3350 provides the cover portions.

The at least one headgear tube 3350 may comprise a vent-facing surface 3350a. The vent-facing surface 3350a may be positioned in use to cover at least a portion of the vent structure 3400 in use, for example, as shown in Figs. 18 and 19. As shown in the example of Figs. 19 and 21, a diffuser 3900 may be located on the vent-facing surface 3350a of the at least one headgear tube 3350. The vent-facing surface 3350a may be comprised as part of the cover portion described above.

The vent-facing surface 3350a may be a patient-facing surface in use. The at least one headgear tube 3350 may also have a non-patient facing surface in use.

In these examples, a portion or portions of the at least one headgear tube 3350 conceals the diffuser 3900, for example the cover portions conceal the diffuser 3900 (again from the perspective of the observer positioned anterior to the patient). Therefore, the vent-facing surface 3350a may conceal the diffuser 3900. In addition, the non-patient facing surface may conceal the diffuser 3900.

The diffuser 3900 may be formed separately and attached to the cover portion(s). In the example of Fig. 19, the diffuser 3900 is attached to the vent-facing surface 3350a of the headgear tube(s) 3350.

In some forms, the diffuser 3900 may be removably attached to the cover portion(s). Therefore, the diffuser 3900 may be removable from the rest of the headgear tube 3350. This can facilitate cleaning, replacement and/or storage of components.

In some forms, the diffuser 3900 may be permanently attached to the headgear tube(s) 3350 using over-moulding, adhesives, laminating, thermoforming, welding, or one or more other well-known methods of attachment. As described above, the headgear tube(s) 3350 may be removable from the rest of the patient interface 3000. Therefore, the diffuser 3900 may be removable from the rest of the patient interface 3000 with the headgear tube 3350. This may reduce the number of patient interface components, and can facilitate cleaning, replacement and/or storage of components.

In other forms, the headgear tube 3350 may be formed with a layer of textile material, for example, at least on a patient contacting surface of the headgear tube 3350. An example of this is a headgear tube 3350 with a textile sleeve, removably or permanently attached thereto, or a headgear tube 3350 that has an integrally formed layer of textile material. The textile layer of textile material may be napped as described above. The napped layer of textile material may form the diffuser 3900. In such forms, the layer of textile material may serve the dual purpose of providing comfort where the headgear tube 3350 contacts the patient's skin, and diffusing the vented flow of gases.

### 4.3.7.4 Frame comprising diffuser

In some forms, for example, as illustrated in Figs. 22 to 24, the component of the positioning and stabilising structure 3300, as described above, comprises a frame 3360.

In the examples illustrated in Figs. 22 to 24, the frame 3360 comprises the diffuser 3900. That is, part of the frame 3360 acts in use to diffuse the vented flow of gas.

In exemplary forms, the frame 3360 comprises a part or parts configured to be positioned in the path of the vent flow of gases exiting the vent structure 3400 in use. For example, the diffuser 3900 may be located on the cover portion of the frame 3360. As shown in the example of Figs. 22 and 24, at least one of the body portion 3368 and the arms 3366 of the frame 3360 provides the cover portions.

The frame 3360 may comprise a vent-facing surface 3360a. The vent-facing surface 3360a may be positioned in use to cover at least a portion of the vent structure 3400 in use, for example, as shown in Figs. 22 and 23. As shown in the example of Figs. 23 and 24, a diffuser 3900 may be located on the vent-facing surface 3360a of the frame 3360. The vent-facing surface 3360a may be comprised as part of the cover portion described above.

The vent-facing surface 3350a may be a patient-facing surface in use. The frame 3360 may also have a non-patient facing surface in use.

In these examples, a part or parts of the frame 3360 conceals the diffuser 3900, for example the cover portions conceal the diffuser 3900. Therefore, the vent-facing surface 3360a may conceal the diffuser 3900. In addition, the non-patient facing surface may conceal the diffuser 3900

The diffuser 3900 may be formed separately and attached to the part(s) of the frame 3360. As shown in Fig. 23, the diffuser may be attached to the vent-facing surface 3360a of the frame 3360.

In some forms, the diffuser 3900 may be removably attached to the part(s) of the frame 3360. Therefore, the diffuser 3900 may be removable from the rest of the frame 3360. This can facilitate cleaning, replacement and/or storage of components.

In some forms, the diffuser 3900 may be permanently attached to the frame 3360 using over-moulding, adhesives, laminating, thermoforming, welding, or one or more other well-known methods of attachment. Therefore, the diffuser 3900 may be removable from the rest of the patient interface 3000 with the frame 3360. This may reduce the number of patient interface components, and can facilitate cleaning, replacement and storage.

### 4.3.8 Decoupling structure(s)

In one form the patient interface 3000 includes at least one decoupling structure, for example, a swivel or a ball and socket.

### 4.3.9 Forehead support

In one form, the patient interface 3000 includes a forehead support 3700.

### 4.3.10 Anti-asphyxia valve

In one form, the patient interface 3000 includes an anti-asphyxia valve.

### 4.3.11 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 4.4 METHOD OF MANUFACTURING HEADGEAR STRAP DIFFUSER

Forms of the technology provide a method of manufacturing a diffuser 3900 comprised as part of a headgear strap 3310 of a positioning and stabilising structure 3300 for holding a patient interface 3000 in a therapeutically effective position on a patient's head. In these forms, the diffuser 3900 may be integrated into the headgear strap 3310.

In certain forms, the method may comprise the following steps, occurring in any order:
(a) forming a diffuser layer 3900 on a surface of a headgear strap material, wherein the diffuser layer 3900 is configured to, in use, diffuse a flow of gases vented through a vent structure 3400 configured on the patient interface 3000; and
(b) forming the headgear strap 3310 from the headgear strap material.

### 4.4.1 Formation of diffuser layer

### 4.4.1.1 Napping headgear strap material

In certain forms, step (a) may comprise napping a portion of the surface of the headgear strap material. This may form napping portion 3310a on the headgear strap 3310. Step (a) may be performed by a napping device comprising a roller configured to nap the headgear strap material as it contacts the roller. Step (a) may further comprise trimming the napped portion 3310a of the surface of the headgear strap material. This trimming step may be performed by the napping device or a trimming device.

In one form, the diffuser 3900 may be integrated into the headgear strap using a localised napping process on the headgear strap material used to form the headgear straps 3310. The localised napping process creates the napped portion 3310a. Napping helps raise a soft, velvety surface comprising a plurality of napped fibres. The napped portion 3310a may comprise the napped fibres. The napped fibres may then be sheared / shaved to achieve a consistent height. Therefore, the height of the napped fibres in the napping portion 3310a is relatively consistent.

In some forms, a roll of fabric, e.g. headgear strap material, may be napped by the napping device and then the fabric may be cut to form the headgear straps 3310 or part thereof. In other forms, the headgear straps 3310 or parts thereof may be cut and then napped using a smaller width of the napping device's roller.

### 4.4.1.2 Using adhesive

In certain forms, step (a) comprises forming a diffuser layer 3900 from a textile material and attaching it to the surface of the headgear strap 3310 using an adhesive.

In one form, the diffuser 3900 may be integrated into the headgear strap 3310 by using adhesive, by potting or by applying an adhesive film.

The diffuser layer 3900 may be formed by cutting a sheet of textile material into the desired shape. This may then be attached to the headgear strap 3310.

It is to be appreciated that in other forms of the present technology, the diffuser layer 3900 may be attached to the surface of the headgear strap 3310 using a fastener or attachment means, e.g. stitching. In some forms, the diffuser layer 3900 may be removably attached to the surface of the headgear strap 3310 using another fastener or attachment mechanism.

### 4.4.2 Formation of headgear strap

In certain forms, step (b) comprises cutting the headgear strap material into headgear straps. Step (b) may be performed by a cutting device. The headgear strap material may comprise a textile material.

### 4.5 METHOD OF MANUFACTURING HEADGEAR CONDUIT DIFFUSER

Forms of the technology provide a method of manufacturing a diffuser 3900 comprised as part of at least one headgear conduit 3350 of a positioning and stabilising structure 3300 for holding a patient interface 3000 in a therapeutically effective position on a patient's head. In these forms, the diffuser 3900 may be integrated into the headgear conduit 3350.

In certain forms, the method may comprise the following steps, occurring in any order:
(a) forming a diffuser layer 3900 on a surface of the headgear conduit 3350, wherein the diffuser layer 3900 is configured to, in use, diffuse a flow of gases vented through a vent structure 3400 configured on the patient interface 3000; and
(b) forming the headgear conduit 3350.

### 4.5.1 Formation of diffuser layer

Step (a) may comprise napping a portion of the surface of the headgear tube 3350, wherein the headgear tube 3350 is formed with a layer of textile material.

Step (a) may comprise trimming the napped portion of the surface of the textile material.

Step (a) may comprise forming a diffuser layer 3900 from a textile material and attaching the diffuser layer 3900 to the surface of the headgear conduit 3350 using an adhesive or other well-known method of attachment.

The diffuser layer 3900 may be formed by cutting the textile material and napping the textile material, occurring in any order.

The textile material may comprise a fleece material.

### 4.5.2 Formation of headgear tube

Step (b) may comprise forming the conduit from one or more materials, e.g. silicone. For instance, a silicone headgear tube 3350 may be formed separately, and the diffuser 3900 is attached to the silicone headgear tube 3350. In other forms, the headgear tube 3350 may be formed to comprise a layer of textile material which is formed into a diffuser layer 39000.

### 4.6 METHOD OF MANUFACTURING FRAME DIFFUSER

Forms of the technology provide a method of manufacturing a diffuser 3900 comprised as part of a frame 3360 of a positioning and stabilising structure 3300 for holding a patient interface 3000 in a therapeutically effective position on a patient's head. In these forms, the diffuser 3900 may be integrated into the frame 3360.

In certain forms, the method may comprise the following steps, occurring in any order:
(a) forming a diffuser layer 3900 on a surface of the frame 3360, wherein the diffuser layer 3900 is configured to, in use, diffuse a flow of gases vented through a vent structure 3400 configured on the patient interface 300; and
(b) forming the frame 3360.

### 4.6.1 Formation of diffuser layer

Step (a) may comprise napping a portion of the surface of the frame 3360, wherein the frame 3360 is formed with a layer of textile material.

Step (a) may comprise trimming the napped portion of the surface of the textile material.

Step (a) may comprise forming a diffuser layer 3900 from a textile material and attaching the diffuser layer 3900 to the surface of the frame 3360 using an adhesive or other well-known method of attachment.

The diffuser layer 3900 may be formed by cutting the textile material.

The diffuser layer 3900 may be formed by cutting the textile material and napping the textile material, occurring in any order.

The textile material may comprise a fleece material.

### 4.6.2 Formation of headgear tube

Step (b) may comprise forming the frame from one or more materials. For instance, frame 3360 may be formed separately, and the diffuser 3900 is attached to the frame 3650. In other forms, the frame 3360 may be formed to comprise a layer of textile material which is formed into a diffuser layer 3900.

### 4.7 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

### 4.7.1 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs, such as with processor control instructions to be executed by one or more processor(s), stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 4.8 AIR CIRCUIT

An air circuit 4170 in accordance with an aspect of the present technology is a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349, which is incorporated herewithin in its entirety by reference.

### 4.9 HUMIDIFIER

### 4.9.1 Humidifier overview

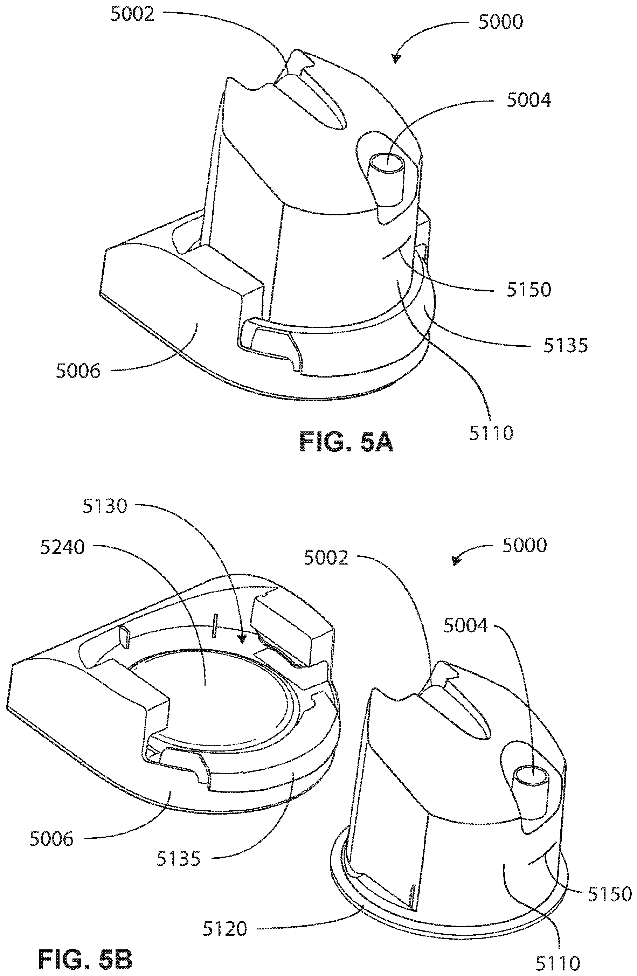

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of air. In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 4.10 GLOSSARY

For the purposes of the present technology disclosure, in certain forms of the present technology, one or more of the following definitions may apply. In other forms of the present technology, alternative definitions may apply.

### 4.10.1 General

Air: In certain forms of the present technology, air may be taken to mean atmospheric air, and in other forms of the present technology air may be taken to mean some other combination of breathable gases, e.g. oxygen enriched air.

Ambient: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

Automatic Positive Airway Pressure (APAP) therapy: CPAP therapy in which the treatment pressure is automatically adjustable, e.g. from breath to breath, between minimum and maximum limits, depending on the presence or absence of indications of SDB events.

Continuous Positive Airway Pressure (CPAP) therapy: Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

Flow rate: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, Qd, is the flow rate of air leaving the RPT device. Total flow rate, Qt, is the flow rate of air and any supplementary gas reaching the patient interface via the air circuit. Vent flow rate, Qv, is the flow rate of air leaving a vent to allow washout of exhaled gases. Leak flow rate, Ql, is the flow rate of leak from a patient interface system or elsewhere. Respiratory flow rate, Qr, is the flow rate of air that is received into the patient's respiratory system.

Flow therapy: Respiratory therapy comprising the delivery of a flow of air to an entrance to the airways at a controlled flow rate referred to as the treatment flow rate that is typically positive throughout the patient's breathing cycle.

Humidifier: The word humidifier will be taken to mean a humidifying apparatus constructed and arranged, or configured with a physical structure to be capable of providing a therapeutically beneficial amount of water (H2O) vapour to a flow of air to ameliorate a medical respiratory condition of a patient.

Leak: The word leak will be taken to be an unintended flow of air. In one example, leak may occur as the result of an incomplete seal between a mask and a patient's face. In another example leak may occur in a swivel elbow to the ambient.

Noise, conducted (acoustic): Conducted noise in the present document refers to noise which is carried to the patient by the pneumatic path, such as the air circuit and the patient interface as well as the air therein. In one form, conducted noise may be quantified by measuring sound pressure levels at the end of an air circuit.

Noise, radiated (acoustic): Radiated noise in the present document refers to noise which is carried to the patient by the ambient air. In one form, radiated noise may be quantified by measuring sound power/pressure levels of the object in question according to ISO 3744.

Noise, vent (acoustic): Vent noise in the present document refers to noise which is generated by the flow of air through any vents such as vent holes of the patient interface.

Oxygen enriched air: Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

Medical Oxygen: Medical oxygen is defined as oxygen enriched air with an oxygen concentration of 80% or greater.

Patient: A person, whether or not they are suffering from a respiratory condition.

Pressure: Force per unit area. Pressure may be expressed in a range of units, including cmH2O, g-f/cm2 and hectopascal. 1 cmH2O is equal to 1 g-f/cm2 and is approximately 0.98 hectopascal (1 hectopascal = 100 Pa = 100 N/m2 = 1 millibar ~ 0.001 atm). In this specification, unless otherwise stated, pressure is given in units of cmH2O.

The pressure in the patient interface is given the symbol Pm, while the treatment pressure, which represents a target value to be achieved by the interface pressure Pm at the current instant of time, is given the symbol Pt.

Respiratory Pressure Therapy: The application of a supply of air to an entrance to the airways at a treatment pressure that is typically positive with respect to atmosphere.

Ventilator: A mechanical device that provides pressure support to a patient to perform some or all of the work of breathing.

### 4.10.1.1 Materials

Silicone or Silicone Elastomer: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

Polycarbonate: a thermoplastic polymer of Bisphenol-A Carbonate.

### 4.10.1.2 Mechanical properties

Resilience: Ability of a material to absorb energy when deformed elastically and to release the energy upon unloading.

Resilient: Will release substantially all of the energy when unloaded. Includes e.g. certain silicones, and thermoplastic elastomers.

Hardness: The ability of a material per se to resist deformation (e.g. described by a Young's Modulus, or an indentation hardness scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

Stiffness (or rigidity) of a structure or component: The ability of the structure or component to resist deformation in response to an applied load. The load may be a force or a moment, e.g. compression, tension, bending or torsion. The structure or component may offer different resistances in different directions. The inverse of stiffness is flexibility.

Floppy structure or component: A structure or component that will change shape, e.g. bend, when caused to support its own weight, within a relatively short period of time such as 1 second.

Rigid structure or component: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH2O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.10.2 Respiratory cycle

Apnea: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

Breathing rate: The rate of spontaneous respiration of a patient, usually measured in breaths per minute.

Duty cycle: The ratio of inhalation time, Ti to total breath time, Ttot.

Effort (breathing): The work done by a spontaneously breathing person attempting to breathe.

Expiratory portion of a breathing cycle: The period from the start of expiratory flow to the start of inspiratory flow.

Flow limitation: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) Flattened: Having a rise followed by a relatively flat portion, followed by a fall.
(ii) M-shaped: Having two local peaks, one at the leading edge, and one at the trailing edge, and a relatively flat portion between the two peaks.
(iii) Chair-shaped: Having a single local peak, the peak being at the leading edge, followed by a relatively flat portion.
(iv) Reverse-chair shaped: Having a relatively flat portion followed by single local peak, the peak being at the trailing edge.

Hypopnea: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

Hyperpnea: An increase in flow to a level higher than normal.

Inspiratory portion of a breathing cycle: The period from the start of inspiratory flow to the start of expiratory flow will be taken to be the inspiratory portion of a breathing cycle.

Patency (airway): The degree of the airway being open, or the extent to which the airway is open. A patent airway is open. Airway patency may be quantified, for example with a value of one (1) being patent, and a value of zero (0), being closed (obstructed).

Positive End-Expiratory Pressure (PEEP): The pressure above atmosphere in the lungs that exists at the end of expiration.

Peak flow rate (Qpeak): The maximum value of flow rate during the inspiratory portion of the respiratory flow waveform.

Respiratory flow rate, patient airflow rate, respiratory airflow rate (Qr): These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

Tidal volume (Vt): The volume of air inhaled or exhaled during normal breathing, when extra effort is not applied. In principle the inspiratory volume Vi (the volume of air inhaled) is equal to the expiratory volume Ve (the volume of air exhaled), and therefore a single tidal volume Vt may be defined as equal to either quantity. In practice the tidal volume Vt is estimated as some combination, e.g. the mean, of the inspiratory volume Vi and the expiratory volume Ve.

Inhalation Time (Ti): The duration of the inspiratory portion of the respiratory flow rate waveform.

Exhalation Time (Te): The duration of the expiratory portion of the respiratory flow rate waveform.

Total Time (Ttot): The total duration between the start of one inspiratory portion of a respiratory flow rate waveform and the start of the following inspiratory portion of the respiratory flow rate waveform.

Typical recent ventilation: The value of ventilation around which recent values of ventilation Vent over some predetermined timescale tend to cluster, that is, a measure of the central tendency of the recent values of ventilation.

Upper airway obstruction (UAO): includes both partial and total upper airway obstruction. This may be associated with a state of flow limitation, in which the flow rate increases only slightly or may even decrease as the pressure difference across the upper airway increases (Starling resistor behaviour).

Ventilation (Vent): A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 4.10.3 Ventilation

Adaptive Servo-Ventilator (ASV): A servo-ventilator that has a changeable, rather than fixed target ventilation. The changeable target ventilation may be learned from some characteristic of the patient, for example, a respiratory characteristic of the patient.

Backup rate: A parameter of a ventilator that establishes the minimum breathing rate (typically in number of breaths per minute) that the ventilator will deliver to the patient, if not triggered by spontaneous respiratory effort.

Cycled: The termination of a ventilator's inspiratory phase. When a ventilator delivers a breath to a spontaneously breathing patient, at the end of the inspiratory portion of the breathing cycle, the ventilator is said to be cycled to stop delivering the breath.

Expiratory positive airway pressure (EPAP): a base pressure, to which a pressure varying within the breath is added to produce the desired interface pressure which the ventilator will attempt to achieve at a given time.

End expiratory pressure (EEP): Desired interface pressure which the ventilator will attempt to achieve at the end of the expiratory portion of the breath. If the pressure waveform template □(□) is zero-valued at the end of expiration, i.e. □(□) = 0 when □ = 1, the EEP is equal to the EPAP.

Inspiratory positive airway pressure (IPAP): Maximum desired interface pressure which the ventilator will attempt to achieve during the inspiratory portion of the breath.

Pressure support: A number that is indicative of the increase in pressure during ventilator inspiration over that during ventilator expiration, and generally means the difference in pressure between the maximum value during inspiration and the base pressure (e.g., PS = IPAP - EPAP). In some contexts, pressure support means the difference which the ventilator aims to achieve, rather than what it actually achieves.

Servo-ventilator: A ventilator that measures patient ventilation, has a target ventilation, and which adjusts the level of pressure support to bring the patient ventilation towards the target ventilation.

Spontaneous/Timed (S/T): A mode of a ventilator or other device that attempts to detect the initiation of a breath of a spontaneously breathing patient. If however, the device is unable to detect a breath within a predetermined period of time, the device will automatically initiate delivery of the breath.

Swing: Equivalent term to pressure support.

Triggered: When a ventilator, or other respiratory therapy device such as an RPT device or portable oxygen concentrator, delivers a volume of breathable gas to a spontaneously breathing patient, it is said to be triggered to do so. Triggering usually takes place at or near the initiation of the respiratory portion of the breathing cycle by the patient's efforts.

### 4.10.4 Anatomy

### 4.10.4.1 Anatomy of the face

Ala: the external outer wall or "wing" of each nostril (plural: alar)

### Alar angle:

Alare: The most lateral point on the nasal ala.

Alar curvature (or alar crest) point: The most posterior point in the curved base line of each ala, found in the crease formed by the union of the ala with the cheek.

Auricle: The whole external visible part of the ear.

(nose) Bony framework: The bony framework of the nose comprises the nasal bones, the frontal process of the maxillae and the nasal part of the frontal bone.

(nose) Cartilaginous framework: The cartilaginous framework of the nose comprises the septal, lateral, major and minor cartilages.

Columella: the strip of skin that separates the nares and which runs from the pronasale to the upper lip.

Columella angle: The angle between the line drawn through the midpoint of the nostril aperture and a line drawn perpendicular to the Frankfort horizontal while intersecting subnasale.

Frankfort horizontal plane: A line extending from the most inferior point of the orbital margin to the left tragion. The tragion is the deepest point in the notch superior to the tragus of the auricle.

Glabella: Located on the soft tissue, the most prominent point in the mid-sagittal plane of the forehead.

Lateral nasal cartilage: A generally triangular plate of cartilage. Its superior margin is attached to the nasal bone and frontal process of the maxilla, and its inferior margin is connected to the greater alar cartilage.

### Lip, lower (labrale inferius):

### Lip, upper (labrale superius):

Greater alar cartilage: A plate of cartilage lying below the lateral nasal cartilage. It is curved around the anterior part of the naris. Its posterior end is connected to the frontal process of the maxilla by a tough fibrous membrane containing three or four minor cartilages of the ala.

Nares (Nostrils): Approximately ellipsoidal apertures forming the entrance to the nasal cavity. The singular form of nares is naris (nostril). The nares are separated by the nasal septum.

Naso-labial sulcus or Naso-labial fold: The skin fold or groove that runs from each side of the nose to the corners of the mouth, separating the cheeks from the upper lip.

Naso-labial angle: The angle between the columella and the upper lip, while intersecting subnasale.

Otobasion inferior: The lowest point of attachment of the auricle to the skin of the face.

Otobasion superior: The highest point of attachment of the auricle to the skin of the face.

Pronasale: the most protruded point or tip of the nose, which can be identified in lateral view of the rest of the portion of the head.

Philtrum: the midline groove that runs from lower border of the nasal septum to the top of the lip in the upper lip region.

Pogonion: Located on the soft tissue, the most anterior midpoint of the chin.

Ridge (nasal): The nasal ridge is the midline prominence of the nose, extending from the Sellion to the Pronasale.

Sagittal plane: A vertical plane that passes from anterior (front) to posterior (rear). The mid-sagittal plane is a sagittal plane that divides the body into right and left halves.

Sellion: Located on the soft tissue, the most concave point overlying the area of the frontonasal suture.

Septal cartilage (nasal): The nasal septal cartilage forms part of the septum and divides the front part of the nasal cavity.

Subalare: The point at the lower margin of the alar base, where the alar base joins with the skin of the superior (upper) lip.

Subnasal point: Located on the soft tissue, the point at which the columella merges with the upper lip in the mid-sagittal plane.

### Supramenton: The point of greatest concavity in the midline of the lower lip between labrale inferius and soft tissue pogonion

### Anatomy of the skull

Frontal bone: The frontal bone includes a large vertical portion, the squama frontalis, corresponding to the region known as the forehead.

Mandible: The mandible forms the lower jaw. The mental protuberance is the bony protuberance of the jaw that forms the chin.

Maxilla: The maxilla forms the upper jaw and is located above the mandible and below the orbits. The frontal process of the maxilla projects upwards by the side of the nose, and forms part of its lateral boundary.

Nasal bones: The nasal bones are two small oblong bones, varying in size and form in different individuals; they are placed side by side at the middle and upper part of the face, and form, by their junction, the "bridge" of the nose.

Nasion: The intersection of the frontal bone and the two nasal bones, a depressed area directly between the eyes and superior to the bridge of the nose.

Occipital bone: The occipital bone is situated at the back and lower part of the cranium. It includes an oval aperture, the foramen magnum, through which the cranial cavity communicates with the vertebral canal. The curved plate behind the foramen magnum is the squama occipitalis.

Orbit: The bony cavity in the skull to contain the eyeball.

Parietal bones: The parietal bones are the bones that, when joined together, form the roof and sides of the cranium.

Temporal bones: The temporal bones are situated on the bases and sides of the skull, and support that part of the face known as the temple.

Zygomatic bones: The face includes two zygomatic bones, located in the upper and lateral parts of the face and forming the prominence of the cheek.

### 4.10.4.2 Anatomy of the respiratory system

Diaphragm: A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

Larynx: The larynx, or voice box houses the vocal folds and connects the inferior part of the pharynx (hypopharynx) with the trachea.

Lungs: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

Nasal cavity: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

Pharynx: The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.10.5 Patient interface

Anti-asphyxia valve (AAV): The component or sub-assembly of a mask system that, by opening to atmosphere in a failsafe manner, reduces the risk of excessive CO2 rebreathing by a patient.

Elbow: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

Frame: Frame will be taken to mean a mask structure that bears the load of tension between two or more points of connection with a headgear. A mask frame may be a non-airtight load bearing structure in the mask. However, some forms of mask frame may also be air-tight.

### Functional dead space: (description to be inserted here)

Headgear: Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a mask plenum chamber will be taken to mean a portion of a patient interface having walls at least partially enclosing a volume of space, the volume having air therein pressurised above atmospheric pressure in use. A shell may form part of the walls of a mask plenum chamber.

Seal: May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

Shell: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery tube, the sub-assembly of components preferably comprises a matched pair of cylindrical conduits. There may be little or no leak flow of air from the swivel in use.

Tie (noun): A structure designed to resist tension.

Vent: (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 4.10.6 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

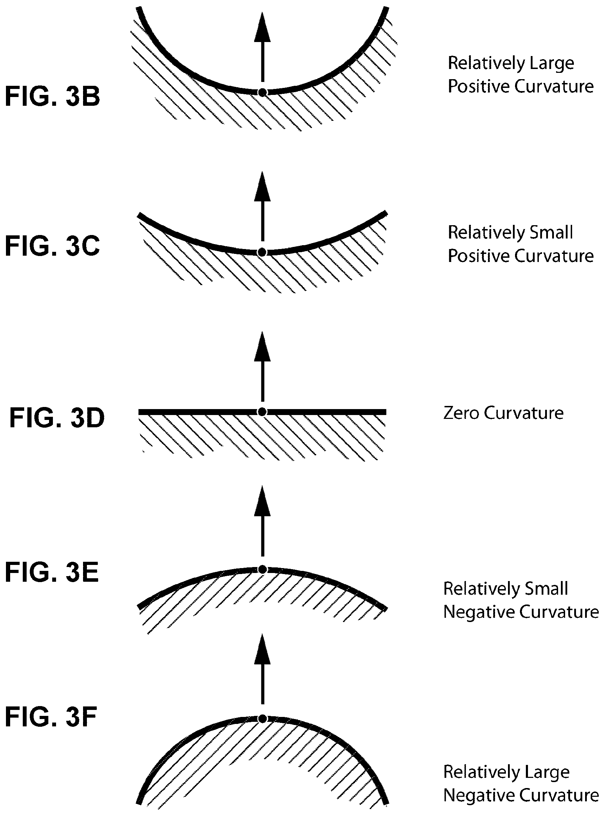

To facilitate describing the shape of the three-dimensional structures and the surfaces, we first consider a cross-section through a surface of the structure at a point, p. See Fig. 3B to Fig. 3F, which illustrate examples of cross-sections at point p on a surface, and the resulting plane curves. Figs. 3B to 3F also illustrate an outward normal vector at p. The outward normal vector at p points away from the surface. In some examples we describe the surface from the point of view of an imaginary small person standing upright on the surface.

### 4.10.6.1 Curvature in one dimension

The curvature of a plane curve at p may be described as having a sign (e.g. positive, negative) and a magnitude (e.g. 1/radius of a circle that just touches the curve at p).

Positive curvature: If the curve at p turns towards the outward normal, the curvature at that point will be taken to be positive (if the imaginary small person leaves the point p they must walk uphill). See Fig. 3B (relatively large positive curvature compared to Fig. 3C) and Fig. 3C (relatively small positive curvature compared to Fig. 3B). Such curves are often referred to as concave.

Zero curvature: If the curve at p is a straight line, the curvature will be taken to be zero (if the imaginary small person leaves the point p, they can walk on a level, neither up nor down). See Fig. 3D.

Negative curvature: If the curve at p turns away from the outward normal, the curvature in that direction at that point will be taken to be negative (if the imaginary small person leaves the point p they must walk downhill). See Fig. 3E (relatively small negative curvature compared to Fig. 3F) and Fig. 3F (relatively large negative curvature compared to Fig. 3E). Such curves are often referred to as convex.

### 4.10.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

Principal curvatures and directions: The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at p are the curvatures in the principal directions.

Region of a surface: A connected set of points on a surface. The set of points in a region may have similar characteristics, e.g. curvatures or signs.

Saddle region: A region where at each point, the principal curvatures have opposite signs, that is, one is positive, and the other is negative (depending on the direction to which the imaginary person turns, they may walk uphill or downhill).

Dome region: A region where at each point the principal curvatures have the same sign, e.g. both positive (a "concave dome") or both negative (a "convex dome").

Cylindrical region: A region where one principal curvature is zero (or, for example, zero within manufacturing tolerances) and the other principal curvature is non-zero.

Planar region: A region of a surface where both of the principal curvatures are zero (or, for example, zero within manufacturing tolerances).

### Edge of a surface: A boundary or limit of a surface or region.

Path: In certain forms of the present technology, 'path' will be taken to mean a path in the mathematical - topological sense, e.g. a continuous space curve from f(0) to f(1) on a surface. In certain forms of the present technology, a 'path' may be described as a route or course, including e.g. a set of points on a surface. (The path for the imaginary person is where they walk on the surface, and is analogous to a garden path).

Path length: In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

Straight-line distance: The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 4.10.6.3 Space curves

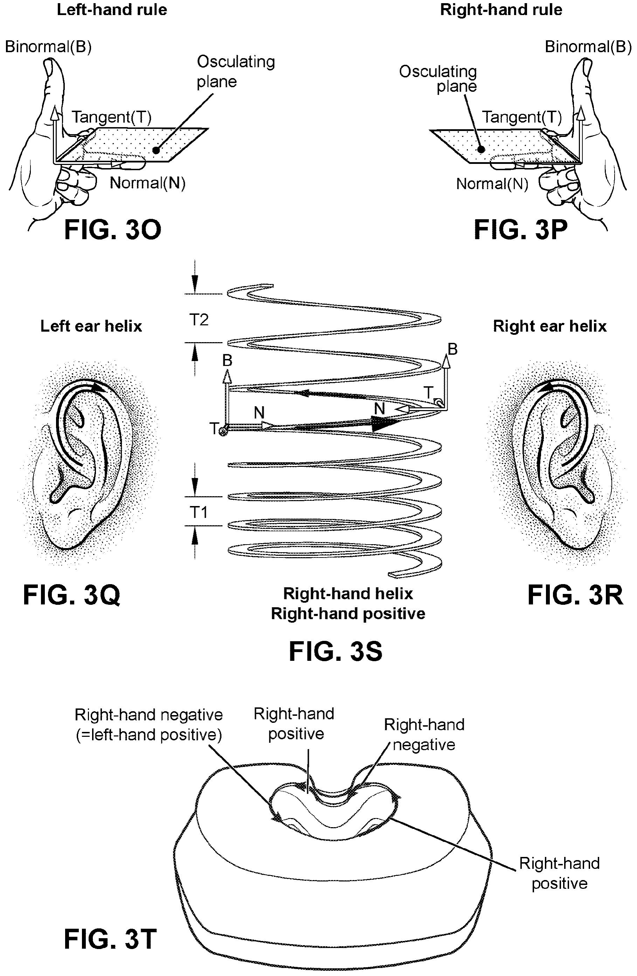

Space curves: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

Tangent unit vector (or unit tangent vector): For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

Unit normal vector: As the imaginary person moves along the curve, this tangent vector itself changes. The unit vector pointing in the same direction that the tangent vector is changing is called the unit principal normal vector. It is perpendicular to the tangent vector.

Binormal unit vector: The binormal unit vector is perpendicular to both the tangent vector and the principal normal vector. Its direction may be determined by a right-hand rule (see e.g. Fig. 3P), or alternatively by a left-hand rule (Fig. 3O).

Osculating plane: The plane containing the unit tangent vector and the unit principal normal vector. See Figures 3O and 3P.

Torsion of a space curve: The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 4.10.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit / tube may comprise a one-dimension hole (e.g. at its entrance or at its exit), and a two-dimension hole bounded by the inside surface of the conduit / tube. See also the two dimensional hole through the structure shown in Fig. 3K, bounded by a surface as shown.

### 4.11 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are incorporated herein by reference in their entirety to disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the spirit and scope of the technology.

### 4.12 REFERENCE SIGNS LIST

- 1000: Patient
- 1100: Bed partner
- 3000: Patient interface
- 3100: Seal-forming structure
- 3110: Holes
- 3150: Cushion module
- 3200: Plenum chamber
- 3210: Frame portion
- 3211: Anterior portion
- 3212: Laterally projecting connection portion
- 3213: Lateral portion
- 3214: Connector
- 3220: Inlet
- 3230: Recess
- 3240: Opening
- 3300: Positioning and stabilising structure
- 3310: Headgear strap
- 3310a: Napped portion
- 3312: Button-hole
- 3320: Conduit portion
- 3330: Rigidiser arm
- 3332: Opening
- 3350: Tube
- 3350a: Vent-facing surface
- 3352: Tab
- 3354: Tube portion
- 3356: Opening
- 3358: Connector
- 3360: Frame
- 3360a: Vent-facing surface
- 3362: Opening
- 3364: Connector
- 3364a: Slot
- 3366: Arm
- 3400: Vent structure
- 3400A: Another vent structure
- 3410: Vent holes
- 3412: Vent wall
- 3412a: Lateral-facing surface
- 3414: Turning portion
- 3420: Vent module
- 3430: Ribs
- 3500: Deflector
- 3512: Lateral wall
- 3512a: Vent-facing surface
- 3512b: Air permeable portion
- 3512c: Air impermeable section
- 3512d: Opening
- 3514: Posterior deflector wall
- 3516: Superior deflector wall
- 3518: Inferior deflector wall
- 3520: Gap
- 3530: Spacer
- 3600: Connection port
- 3800: Connector
- 3810: Projecting portion
- 3820: Slot
- 3900: Diffuser
- 4000: RPT device
- 4010: External housing
- 4012: Upper portion
- 4014: Lower Portion
- 4015: Panel
- 4016: Chassis
- 4018: Handle
- 4020: Pneumatic block
- 4100: Blower housing
- 4110: Air filter
- 4112: Inlet air filter
- 4114: Outlet air filter
- 4120: Muffler
- 4122: Inlet muffler
- 4124: Outlet muffler
- 4140: Pressure generator
- 4142: Blower
- 4144: Motor

The following aspects are preferred embodiments of the invention.
1. A patient interface comprising:
   a plenum chamber pressurisable to a therapeutic pressure of at least 6 cmH2O above ambient air pressure, wherein the plenum chamber comprises an anterior portion comprising an inlet configured to receive a flow of air at the therapeutic pressure for breathing by a patient;
   a seal-forming structure configured to form a seal with a region of the patient's face surrounding an entrance to the patient's airways, wherein the seal-forming structure is configured to maintain said therapeutic pressure in the plenum chamber throughout the patient's respiratory cycle in use;
   a vent structure configured to allow a flow of gases exhaled by the patient from an interior of the plenum chamber to ambient, wherein the vent structure is configured to maintain the therapeutic pressure in the plenum chamber in use, and wherein the vent structure is configured to vent the flow of gases from the interior of the plenum chamber in a substantially lateral direction in use; and
   a deflector configured to redirect, in use, a substantial amount of the laterally vented flow of gases in a direction having an anterior component relative to the patient.
2. The patient interface according to aspect 1, wherein the plenum chamber comprises a medial portion, wherein the medial portion is located so that, in use, the mid-sagittal plane of the patient passes through the medial portion, and wherein the inlet is located on the medial portion.
3. The patient interface according to either aspect 1 or 2, further comprising an air delivery tube which connects to the plenum chamber and is configured to be in fluid communication with the inlet.
4. The patient interface according to any one of aspects 1 to 3, wherein the vent structure comprises a plurality of vent holes.
5. The patient interface according to any one of aspects 1 to 4, wherein the vent structure is located on a portion of the patient interface which is lateral to the inlet in use.
6. The patient interface according to any one of aspects 1 to 5, wherein the vent structure is located on the plenum chamber.
7. The patient interface according to any one of aspects 1 to 6, wherein the vent structure comprises a vent module.
8. The patient interface according to aspect 7 comprising a vent module opening, wherein the vent module is configured to attach to a portion of the patient interface comprising the vent module opening.
9. The patient interface according to any one of aspects 1 to 8 wherein the vent structure is a first vent structure configured to vent a first flow of gases from the interior of the plenum chamber in a first substantially lateral direction in use, and wherein the patient interface comprises a second vent structure configured to vent a second flow of gases from the interior of the plenum chamber in a second substantially lateral direction in use, wherein the first substantially lateral direction is generally opposite to the second substantially lateral direction.
10. The patient interface according to aspect 9, wherein the first vent structure is located lateral to the inlet on one side of the patient interface, and wherein the second vent structure is located lateral to the inlet on the other side of the patient interface.
11. The patient interface according to any one of aspects 1 to 10, wherein the deflector comprises an arrangement of one or more deflector walls.
12. The patient interface according to any one of aspects 1 to 11, wherein the deflector redirects the substantial amount of the laterally vented flow of gases towards a/the medial portion and/or an/the anterior portion of the plenum chamber in use.
13. The patient interface according to any one of aspects 1 to 12, wherein the deflector redirects the substantial amount of the laterally vented flow of gases towards a portion of the air delivery tube in use.
14. The patient interface according to any one of aspects 1 to 13, wherein the deflector is comprised as part of the vent structure.
15. The patient interface according to any one of aspects 1 to 14, wherein the deflector is a first deflector configured to redirect a substantial amount of the first laterally vented flow of gases in an anterior direction in use, and wherein the patient interface comprises a second deflector configured to redirect a substantial amount of the second laterally vented flow of gases in an anterior direction in use.
16. The patient interface according to any one of aspects 1 to 15 comprising a pair of connectors to facilitate attachment of the plenum chamber to a positioning and stabilising structure.
17. The patient interface according to aspect 16, wherein at least one of the connectors is comprised as part of the vent structure.
18. The patient interface according to either aspect 16 or 17, wherein at least one of the connectors is comprised as part of the deflector.
19. The patient interface according to any one of aspects 1 to 18 comprising a positioning and stabilising structure to provide a force to hold the seal-forming structure in a therapeutically effective position on the patient's head.
20. The patient interface according to aspect 19, wherein the positioning and stabilising structure comprises at least one headgear strap.
21. The patient interface according to any one of aspects 16 to 20, wherein a/the headgear strap is removably attached to the connector by buttoning.
22. The patient interface according to aspect 21, wherein the headgear strap comprises a button-hole.
23. The patient interface according to aspect 22, wherein the headgear strap comprises a sleeve-like configuration to receive the insertion of a portion of the connector into the at least one headgear strap via the button-hole.
24. The patient interface according to any one of aspects 19 to 23, wherein the vent structure is located on the positioning and stabilising structure.
25. The patient interface according to aspect 24, wherein the positioning and stabilising structure comprises a conduit portion and the plenum chamber comprises an opening, wherein the conduit portion is configured to be, in use, in fluid communication with an interior of the plenum chamber through the opening, and wherein the vent structure is located on the conduit portion.
26. The patient interface according to aspect 25, wherein the conduit portion comprises a support structure to limit and/or prevent occlusion of the conduit portion.
27. The patient interface according to any one of aspects 1 to 26 comprising a diffuser, wherein the diffuser is positioned in use to diffuse the flow of gases exiting the vent structure.
28. The patient interface according to aspect 27, wherein the diffuser comprises a textile material.
29. The patient interface according to either aspect 27 or 28, wherein a/the headgear strap comprises the diffuser.
30. The patient interface according to any one of aspects 27 to 29, wherein the diffuser forms at least part of the deflector.
31. The patient interface according to any one of aspects 1 to 30, wherein the seal-forming structure is configured to form a seal around the patient's nares but not the patient's mouth in use.
32. The patient interface according to any one of aspects 1 to 31, wherein the seal-forming structure is configured to form a seal in use with the underside of the nose around the nares.
33. A patient interface comprising:
   a plenum chamber pressurisable to a therapeutic pressure of at least 6 cmH2O above ambient air pressure, wherein the plenum chamber comprises an inlet configured to receive a flow of air at the therapeutic pressure for breathing by a patient;
   a seal-forming structure configured to form a seal with a region of the patient's face surrounding an entrance to the patient's airways, wherein the seal-forming structure is configured to maintain said therapeutic pressure in the plenum chamber throughout the patient's respiratory cycle in use;
   a vent structure configured to allow a flow of gases exhaled by the patient from an interior of the plenum chamber to ambient, wherein the vent structure is configured to maintain the therapeutic pressure in the plenum chamber in use; and
   a positioning and stabilising structure to provide a force to hold the seal-forming structure in a therapeutically effective position on the patient's head, wherein the positioning and stabilising structure comprises at least one headgear strap,
   wherein the at least one headgear strap comprises a diffuser, wherein the diffuser is positioned in use to diffuse the flow of gases exiting the vent structure.
34. The patient interface according to aspect 33, wherein the plenum chamber comprises an anterior portion, and wherein the inlet is located in the anterior portion.
35. The patient interface according to either aspect 33 or 34, wherein the plenum chamber comprises a medial portion, wherein the medial portion is located so that, in use, the mid-sagittal plane of the patient passes through the medial portion, and wherein the inlet is located on the medial portion.
36. The patient interface according to any one of aspects 33 to 35 comprising an air delivery tube which connects to the plenum chamber and is configured to be in fluid communication with the inlet.
37. The patient interface according to any one of aspects 33 to 36, wherein the vent structure is configured to vent the flow of gases from the interior of the plenum chamber in a substantially lateral direction in use.
38. The patient interface according to any one of aspects 33 to 37, wherein the vent structure is located on a portion of the patient interface which is lateral to the inlet.
39. The patient interface according to any one of aspects 33 to 38, wherein the vent structure is a first vent structure configured to vent a first flow of gases exhaled by the patient from an interior of the plenum chamber to ambient, and wherein the patient interface comprises a second vent structure configured to vent a second flow of gases exhaled by the patient from an interior of the plenum chamber to ambient.
40. The patient interface according to aspect 39, wherein the first vent structure is configured to vent the first flow of gases from the interior of the plenum chamber in a first substantially lateral direction in use, and wherein the second vent structure is configured to vent the second flow of gases from the interior of the plenum chamber in a second substantially lateral direction in use, wherein the first substantially lateral direction is generally opposite to the second substantially lateral direction.
41. The patient interface according to either aspect 39 or 40, wherein the first vent structure is located lateral to the inlet on one side of the patient interface, and wherein the second vent structure is located lateral to the inlet on the other side of the patient interface.
42. The patient interface according to any one of aspects 33 to 41 comprising a deflector configured to redirect, in use, a substantial amount of the laterally vented flow of gases in a direction having an anterior component relative to the patient.
43. The patient interface according to aspect 42, wherein the deflector is a first deflector configured to redirect a substantial amount of a/the first laterally vented flow of gases in an anterior direction in use, and wherein the patient interface comprises a second deflector configured to redirect a substantial amount of a/the second laterally vented flow of gases in an anterior direction in use.
44. The patient interface according to any one of aspects 33 to 43, wherein a cover portion of the at least one headgear strap covers at least a portion of the vent structure in use.
45. The patient interface according to aspect 44, wherein the diffuser is located on the cover portion.
46. The patient interface according to any one of aspects 33 to 44, wherein the diffuser is removably attached to the at least one headgear strap.
47. The patient interface according to aspect 46, wherein the diffuser is formed from a textile material.
48. The patient interface according to any one of aspects 33 to 45, wherein the diffuser is formed from a headgear strap material.
49. The patient interface according to aspect 48, wherein the diffuser is formed by napping a portion of a surface of the headgear strap material.
50. The patient interface according to any one of aspects 42 to 49, wherein the diffuser forms at least part of the deflector.
51. The patient interface according to any one of aspects 33 to 50 comprising a pair of connectors to facilitate attachment of the plenum chamber to the at least one headgear strap.
52. The patient interface according to aspect 51, wherein the at least one headgear strap is removably attached to the connector by buttoning.
53. The patient interface according to aspect 52, wherein the at least one headgear strap comprises a button-hole.
54. The patient interface according to aspect 53, wherein the at least one headgear strap comprises a sleeve-like configuration to receive the insertion of a portion of the connector into the at least one headgear strap via the button-hole.
55. The patient interface according to any one of aspects 33 to 54, wherein the seal-forming structure is configured to form a seal around the patient's nares but not the patient's mouth in use.
56. The patient interface according to any one of aspects 33 to 55, wherein the seal-forming structure is configured to form a seal in use with the underside of the nose around the nares.
57. A patient interface comprising:
   a plenum chamber pressurisable to a therapeutic pressure of at least 6 cmH2O above ambient air pressure, wherein the plenum chamber comprises an inlet configured to receive a flow of air at the therapeutic pressure for breathing by a patient;
   a seal-forming structure configured to form a seal with a region of the patient's face surrounding an entrance to the patient's airways, wherein the seal-forming structure is configured to maintain said therapeutic pressure in the plenum chamber throughout the patient's respiratory cycle in use;
   a vent structure configured to allow a vent flow of gases exhaled by the patient from an interior of the plenum chamber to ambient, wherein the vent structure is configured to maintain the therapeutic pressure in the plenum chamber in use; and
   a positioning and stabilising structure to provide a force to hold the seal-forming structure in a therapeutically effective position on the patient's head,
   wherein the positioning and stabilising structure comprises a component comprising a vent-facing surface which is positioned in use in the path of the vent flow of gases exiting the vent structure,
   wherein a diffuser is located on the vent-facing surface of the component, and wherein the diffuser is positioned in use to diffuse the vent flow of gases exiting the vent structure.
58. The patient interface according to aspect 57, wherein the component comprises a frame configured to facilitate indirect attachment of the plenum chamber to at least one headgear strap of the positioning and stabilising structure.
59. The patient interface according to aspect 58, wherein the frame is attached to the plenum chamber and the at least one headgear strap is attached to the frame.
60. The patient interface according to either aspect 58 or 59, wherein an air delivery tube is attached to at least one of the frame and the plenum chamber.
61. The patient interface according to any one of aspects 58 to 60, wherein the frame comprises an opening, wherein a/the air delivery tube is configured to be, in use, in fluid communication with the inlet through the opening.
62. The patient interface according to any one of aspects 58 to 61, wherein the diffuser is removably attached to the frame.
63. The patient interface according to any one of aspects 58 to 62 , wherein the frame comprises a pair of connectors to facilitate attachment of the frame to the at least one headgear strap.
64. The patient interface according to aspect 63, wherein the at least one headgear strap is removably attached to the connectors.
65. The patient interface according to aspect 57, wherein the component comprises at least one tube, wherein the at least one tube is configured, in use, to convey the flow of air from an air circuit fluidly connected to the at least one tube to an interior of the plenum chamber through the inlet.
66. The patient interface according to aspect 65, wherein the diffuser is removably attached to the at least one tube.
67. The patient interface according to aspect 57, wherein the component comprises a rigidiser arm, wherein the rigidiser arm is configured to rigidise at least one headgear strap of the positioning and stabilising structure.
68. The patient interface according to aspect 67, wherein the diffuser is removably attached to the rigidiser arm.
69. The patient interface according to any one of aspects 57 to 68, wherein the seal-forming structure is configured to form a seal around the patient's nares but not the patient's mouth in use.
70. The patient interface according to any one of aspects 57 to 68, wherein the seal-forming structure is configured to form a seal in use around the patient's nose and mouth.
71. A positioning and stabilising structure for holding a patient interface in a therapeutically effective position on a patient's head, the positioning and stabilising structure comprising:
   at least one headgear strap,
   wherein the at least one headgear strap comprises a diffuser, wherein the diffuser is configured to diffuse a flow of gases vented from an interior of the patient interface to ambient through a vent structure configured on the patient interface.
72. The positioning and stabilising structure according to aspect 71, wherein a cover portion of the at least one headgear strap covers at least a portion of the vent structure in use.
73. The positioning and stabilising structure according to aspect 72, wherein the diffuser is located on the cover portion.
74. The positioning and stabilising structure according to any one of aspects 71 to 73, wherein the diffuser is formed separately and attached to the at least one headgear strap.
75. The positioning and stabilising structure according to aspect 74, wherein the diffuser is formed from a textile material.
76. The positioning and stabilising structure according to any one of aspects 71 to 73, wherein the diffuser is formed from a headgear strap material.
77. The positioning and stabilising structure according to aspect 76, wherein the diffuser is formed by napping a portion of a surface of the headgear strap material.
78. The positioning and stabilising structure according to any one of aspect 71 to 77, wherein the at least one headgear strap comprises a button-hole.
79. The positioning and stabilising structure according to aspect 78, wherein the at least one headgear strap comprises a sleeve-like configuration to receive the insertion of a portion of a connector into the at least one headgear strap via the button-hole, wherein the connector facilitates attachment of the patient interface to the at least one headgear strap.
80. A positioning and stabilising structure for holding a patient interface in a therapeutically effective position on a patient's head, the positioning and stabilising structure comprising:
   a component comprising a vent-facing surface which is positioned in use in the path of the vent flow of gases exiting a vent structure on the patient interface,
   wherein a diffuser is located on the vent-facing surface of the component, and wherein the diffuser is configured to diffuse the vent flow of gases exiting the vent structure.
81. The positioning and stabilising structure according to aspect 80, wherein the component comprises a frame configured to facilitate indirect attachment of the plenum chamber to at least one headgear strap of the positioning and stabilising structure.
82. The positioning and stabilising structure according to aspect 80, wherein the component comprises at least one tube, wherein the at least one tube is configured, in use, to convey the flow of air from an air circuit fluidly connected to the at least one tube to an interior of the plenum chamber through the inlet.
83. The positioning and stabilising structure according to aspect 80, wherein the component comprises a rigidiser arm, wherein the rigidiser arm is configured to rigidise at least one headgear strap of the positioning and stabilising structure.
84. The positioning and stabilising structure according to any one of aspects 80 to 83, wherein the diffuser is removably attached to the component.
85. A patient interface comprising:
   a plenum chamber pressurisable to a therapeutic pressure of at least 6 cmH2O above ambient air pressure, wherein the plenum chamber comprises an inlet configured to receive a flow of air at the therapeutic pressure for breathing by a patient;
   a seal-forming structure configured to form a seal with a region of the patient's face surrounding an entrance to the patient's airways, wherein the seal-forming structure is configured to maintain said therapeutic pressure in the plenum chamber throughout the patient's respiratory cycle in use;
   a positioning and stabilising structure for holding the seal-forming structure in a therapeutically effective position on a patient's head,
   wherein the positioning and stabilising structure comprises a conduit portion and the plenum chamber comprises an opening, wherein the conduit portion is configured to be, in use, in fluid communication with an interior of the plenum chamber through the opening, and
   wherein the conduit portion comprises a vent structure configured to allow a flow of gases exhaled by the patient from the interior of the plenum chamber to ambient, wherein the vent structure is configured to maintain the therapeutic pressure in the patient interface in use.
86. The patient interface according to aspect 85, wherein the vent structure is configured to vent the flow of gases from the interior of the patient interface in a substantially lateral direction in use.
87. The patient interface according to either aspect 85 or 86, wherein the plenum chamber comprises an anterior portion, and wherein the inlet is located in the anterior portion.
88. The patient interface according to any one of aspects 85 to 87, wherein the plenum chamber comprises a medial portion, wherein the medial portion is located so that, in use, the mid-sagittal plane of the patient passes through the medial portion, and wherein the inlet is located on the medial portion.
89. The patient interface according to any one of aspects 85 to 88 comprising an air delivery tube which connects to the plenum chamber and is configured to be in fluid communication with the inlet.
90. The patient interface according to any one of aspects 85 to 89, wherein the positioning and stabilising structure comprises an inferior end which connects to the plenum chamber in use, and wherein the conduit portion comprises the inferior end.
91. The patient interface according to any one of aspects 85 to 90, wherein the plenum chamber comprises a laterally projecting connection portion, wherein the opening is located on the laterally projecting connection portion.
92. The patient interface according to any one of aspects 85 to 91, wherein the conduit portion is a first conduit portion comprising a first vent structure and the opening is a first opening, wherein the first conduit portion is configured to be, in use, in fluid communication with an interior of the plenum chamber through the first opening, and wherein the positioning and stabilising structure comprises a second conduit portion comprising a second vent structure and the plenum chamber comprises a second opening, wherein the second conduit portion is configured to be, in use, in fluid communication with an interior of the plenum chamber through the second opening.
93. The patient interface according to any one of aspects 85 to 92, wherein the positioning and stabilising structure comprises at least one headgear strap.
94. The patient interface according to aspect 93, wherein the at least one headgear strap is connected to the conduit portion.
95. The patient interface according to any one of aspects 85 to 94, wherein the conduit portion is configured to be relatively rigid.
96. The patient interface according to any one of aspects 85 to 94, wherein the conduit portion is configured to be relatively flexible.
97. The patient interface according to any one of aspects 85 to 96 wherein the conduit portion comprises a support structure to limit and/or prevent occlusion of the conduit portion.
98. The patient interface according to aspect 97, wherein the support structure is provided by one or more of a reinforcing structure, a thickened region, and a stiffened region.
99. The patient interface according to any one of aspects 85 to 98, wherein the vent structure comprises a plurality of vent holes.
100. The patient interface according to any one of aspects 85 to 99, wherein the vent structure is located lateral to the plenum chamber in use.
101. The patient interface according to any one of aspects 85 to 100, wherein the vent structure is located proximate to the inferior end of the positioning and stabilising structure in use.
102. The patient interface according to any one of aspects 95 to 101 comprising a diffuser for diffusing the vented and/or redirected flow of gases.
103. The patient interface according to aspect 102, wherein the diffuser comprises a textile material.
104. The patient interface according to any one of aspects 85 to 103 comprising a deflector configured to redirect, in use, a substantial amount of the laterally vented flow of gases in a direction having an anterior component relative to the patient.
105. The patient interface according to aspect 104, wherein the conduit portion comprises the deflector.
106. The patient interface according to either aspect 85 or 105, wherein a/the diffuser for diffusing the vented and/or redirected flow of gases forms at least part of a/the deflector.
107. The patient interface according to any one of aspects 85 to 106, wherein the seal-forming structure is configured to form a seal around the patient's nares but not the patient's mouth in use.
108. The patient interface according to any one of aspects 85 to 107, wherein the seal-forming structure is configured to form a seal in use with the underside of the nose around the nares.
109. A positioning and stabilising structure for holding a patient interface in a therapeutically effective position on a patient's head, the positioning and stabilising structure comprising:
   a conduit portion configured to be in fluid communication with an interior of the patient interface in use,
   wherein the conduit portion comprises a vent structure configured to allow a flow of gases exhaled by the patient from the interior of the patient interface to ambient, wherein the vent structure is configured to maintain the therapeutic pressure in the interior of the patient interface in use.
110. The positioning and stabilising structure according to aspect 109, wherein the vent structure is configured to vent the flow of gases from the interior of the patient interface in a substantially lateral direction in use.
111. The positioning and stabilising structure according to either aspect 109 or 110 comprising an inferior end which connects to the patient interface in use, and wherein the conduit portion comprises the inferior end.
112. The positioning and stabilising structure according to any one of aspects 109 to 111, wherein the conduit portion is configured to connect to a plenum chamber of the patient interface, wherein the plenum chamber comprises an opening, and wherein the conduit portion is configured to be, in use, in fluid communication with an interior of the plenum chamber through the opening.
113. The positioning and stabilising structure according to aspect 112 comprising a connector configured to facilitate attachment of conduit portion to the opening.
114. The positioning and stabilising structure according to any one of aspects 109 to 113, wherein the conduit portion is a first conduit portion comprising a first vent structure configured to vent a first flow of gases from the interior of the patient interface in a substantially lateral direction in use, wherein the first conduit is configured to be, in use, in fluid communication with an interior of the plenum chamber, and wherein the positioning and stabilising structure comprises a second conduit portion comprising a second vent structure configured to vent a second flow of gases from the interior of the patient interface in a substantially lateral direction in use, wherein the second conduit portion is configured to be, in use, in fluid communication with an interior of the plenum chamber.
115. The positioning and stabilising structure according to aspect 114 when dependent on either aspect 112 or 113, wherein the opening is a first opening configured in the plenum chamber, wherein the first conduit is configured to be, in use, in fluid communication with the interior of the plenum chamber through the first opening, and wherein the plenum chamber comprises a second opening, wherein the second conduit portion is configured to be, in use, in fluid communication with the interior of the plenum chamber through the second opening.
116. The positioning and stabilising structure according to aspect 115 when dependent on aspect 113, wherein the connector is a first connector configured to facilitate attachment of the first conduit portion to the first opening, and wherein the patient interface comprises a second connector configured to facilitate attachment of the second conduit portion to the second opening.
117. The positioning and stabilising structure according to any one of aspects 109 to 117 comprising at least one headgear strap.
118. The positioning and stabilising structure according to aspect 117, wherein the at least one headgear strap is connected to the conduit portion.
119. The positioning and stabilising structure according to any one of aspects 109 to 118, wherein the conduit portion is configured to be relatively rigid.
120. The positioning and stabilising structure according to any one of aspects 109 to 118, wherein the conduit portion is configured to be relatively flexible.
121. The positioning and stabilising structure according to any one of aspects 109 to 120, wherein the conduit portion comprises a support structure to limit and/or prevent occlusion of the conduit portion.
122. The positioning and stabilising structure according to aspect 121, wherein the support structure is provided by one or more of a reinforcing structure, a thickened region, and a stiffened region.
123. The positioning and stabilising structure according to any one of aspects 109 to 122, wherein the vent structure comprises a plurality of vent holes.
124. The positioning and stabilising structure according to any one of aspects 109 to 123, wherein the vent structure is located proximate to an/the inferior end of the positioning and stabilising structure in use.
125. The positioning and stabilising structure according to any one of aspects 109 to 124 comprising a diffuser for diffusing the vented and/or redirected flow of gases, wherein the diffuser is positioned in use to diffuse the flow of gases exiting the vent structure.
126. The positioning and stabilising structure according to aspect 125, wherein the diffuser comprises a textile material.
127. The positioning and stabilising structure according to either aspect 125 or 126, wherein the diffuser comprises a headgear strap material.
128. The positioning and stabilising structure according to aspect 127, wherein at least a portion of the headgear strap material covers at least a portion of the conduit portion and at least a portion of the vent structure.
129. The positioning and stabilising structure according to any one of aspects 109 to 128 comprising a deflector configured to redirect, in use, a substantial amount of the laterally vented flow of gases in a direction having an anterior component relative to the patient.
130. The positioning and stabilising structure according to aspect 129, wherein the conduit portion comprises the deflector.
131. The positioning and stabilising structure according to any one of aspects 109 to 130, wherein a/the diffuser forms at least part of a/ the deflector.
132. A patient interface comprising:
   a plenum chamber pressurisable to a therapeutic pressure of at least 6 cmH2O above ambient air pressure, wherein the plenum chamber comprises an inlet configured to receive a flow of air at the therapeutic pressure for breathing by a patient;
   a seal-forming structure configured to form a seal with a region of the patient's face surrounding an entrance to the patient's airways, wherein the seal-forming structure is configured to maintain said therapeutic pressure in the plenum chamber throughout the patient's respiratory cycle in use;
   a positioning and stabilising structure to provide a force to hold the seal-forming structure in a therapeutically effective position on the patient's head,
   a vent structure configured to allow a flow of gases exhaled by the patient from an interior of the plenum chamber to ambient, wherein the vent structure is configured to maintain the therapeutic pressure in the plenum chamber in use; and
   a deflector configured to redirect a substantial amount of the vented flow of gases in use,
   wherein the positioning and stabilising structure comprises the deflector.
133. The patient interface according to aspect 132, wherein the plenum chamber comprises an anterior portion comprising the inlet.
134. The patient interface according to either aspect 132 or 133, wherein the plenum chamber comprises a medial portion, wherein the medial portion is located so that, in use, the mid-sagittal plane of the patient passes through the medial portion, and wherein the inlet is located on the medial portion.
135. The patient interface according to any one of aspects 132 to 134, wherein the vent structure comprises a plurality of vent holes.
136. The patient interface according to any one of aspects 132 to 135, wherein the vent structure is located on a portion of the patient interface which is lateral to the inlet in use.
137. The patient interface according to any one of aspects 132 to 136, wherein the vent structure is located on the plenum chamber.
138. patient interface according to any one of aspects 132 to 137, wherein the vent structure is configured to vent the flow of gases from the interior of the plenum chamber in a substantially lateral direction in use.
139. The patient interface according to any one of aspects 132 to 138, wherein the vent structure is a first vent structure configured to vent a first flow of gases exhaled by the patient from an interior of the plenum chamber to ambient, and wherein the patient interface comprises a second vent structure configured to vent a second flow of gases exhaled by the patient from an interior of the plenum chamber to ambient.
140. The patient interface according to aspect 139, wherein the first vent structure is configured to vent the first flow of gases from the interior of the plenum chamber in a first substantially lateral direction in use, and wherein the second vent structure is configured to vent the second flow of gases from the interior of the plenum chamber in a second substantially lateral direction in use, wherein the first substantially lateral direction is generally opposite to the second substantially lateral direction.
141. The patient interface according to either aspect 139 or 140, wherein the first vent structure is located lateral to the inlet on one side of the patient interface, and wherein the second vent structure is located lateral to the inlet on the other side of the patient interface.
142. The patient interface according to any one of aspects 132 to 141, wherein the deflector is a first deflector configured to redirect a substantial amount of a/the first flow of gases in a first direction in use, and wherein the positioning and stabilising structure comprises a second deflector configured to redirect a substantial amount of a/the second flow of gases in a second direction in use.
143. The patient interface according to any one of aspects 132 to 142 comprising a spacer to provide a gap between a surface of the vent structure and a surface of the deflector.
144. The patient interface according to aspect 143, wherein the spacer comprises at least one rib.
145. The patient interface according to aspect 144, wherein the at least one rib provides a plurality of flow paths to direct the flow of gases exiting the vent structure.
146. The patient interface according to any one of aspects 143 to 145, wherein the spacer comprises part of the vent structure.
147. The patient interface according to any one of aspects 143 to 145, wherein the spacer comprises part of the deflector.
148. The patient interface according to any one of aspects 132 to 147, wherein the deflector comprises an arrangement of one or more deflector walls.
149. The patient interface according to aspect 148, wherein a/the spacer which provides a gap between a surface of the vent structure and a surface of the deflector, comprises at least one of the deflector walls.
150. The patient interface according to any one of aspects 132 to 149, wherein the position and stabilising structure comprises a diffuser, wherein the diffuser is positioned in use to diffuse the flow of gases exiting the vent structure.
151. The patient interface according to aspect 150, wherein the diffuser comprises a textile material.
152. The patient interface according to any one of aspects 132 to 151, wherein the positioning and stabilising structure comprises at least one tube, wherein the at least one tube is configured, in use, to convey the flow of air from an air circuit fluidly connected to the at least one tube to an interior of the plenum chamber through the inlet.
153. The patient interface according to aspect 152, wherein the at least one tube comprises the deflector, wherein a portion of the at least one tube is positioned in use to redirect the flow of gases exiting the vent structure.
154. The patient interface according to either aspect 152 or 153, wherein the at least one tube comprises a/the diffuser.
155. The patient interface according to any one of aspects 132 to 151 comprising an air delivery tube which connects to the plenum chamber and is configured to be in fluid communication with the inlet.
156. The patient interface according to any one of aspects 132 to 151 and 155, wherein the positioning and stabilising structure comprises a frame configured to facilitate attachment of the plenum chamber to at least one headgear strap of the positioning and stabilising structure.
157. The patient interface according to aspect 156, wherein the frame is attached to the plenum chamber and the frame is attached to the at least one headgear strap.
158. The patient interface according to either aspect 156 or 157 , wherein an/the air delivery tube is attached to at least one of the frame and the plenum chamber.
159. The patient interface according to any one of aspects 156 to 158, wherein the frame comprises an opening, wherein an/the air delivery is configured to be, in use, in fluid communication with the inlet through the opening.
160. The patient interface according to any one of aspects 156 to 169, wherein frame comprises a pair of connectors to facilitate attachment of the patient interface to the at least headgear strap.
161. The patient interface according to any one of aspects 156 to 160, wherein the frame comprises the deflector, wherein a part of the frame is positioned in use to redirect the flow of gases exiting the vent structure.
162. The patient interface according to any one of aspect 156 to 161, wherein the frame comprises a/the diffuser.
163. The patient interface according to any one of aspects 132 to 162, wherein the deflector is configured to redirect a substantial amount of a/the laterally vented flow of gases in at least one of a substantially superior direction and a substantially inferior direction in use.
164. The patient interface according to any one of aspects 132 to 163, wherein the seal-forming structure is configured to form a seal around the patient's nares but not the patient's mouth in use.
165. The patient interface according to any one of aspects 132 to 164, wherein the seal-forming structure is configured to form a seal in use with the underside of the nose around the nares.
166. The patient interface according to any one of aspects 132 to 163, wherein the seal-forming structure is configured to form a seal in use around the patient's nose and mouth.

## Claims

1. A patient interface comprising:
a plenum chamber pressurisable to a therapeutic pressure of at least 6 cmH2O above ambient air pressure, wherein the plenum chamber comprises an inlet configured to receive a flow of air at the therapeutic pressure for breathing by a patient;
a seal-forming structure configured to form a seal with a region of the patient's face surrounding an entrance to the patient's airways, wherein the seal-forming structure is configured to maintain said therapeutic pressure in the plenum chamber throughout the patient's respiratory cycle in use;
a vent structure configured to allow a flow of gases exhaled by the patient from an interior of the plenum chamber to ambient, wherein the vent structure is configured to maintain the therapeutic pressure in the plenum chamber in use; and
a positioning and stabilising structure to provide a force to hold the seal-forming structure in a therapeutically effective position on the patient's head, wherein the positioning and stabilising structure comprises at least one headgear strap,
wherein the at least one headgear strap comprises a diffuser, wherein the diffuser is positioned in use to diffuse the flow of gases exiting the vent structure.

2. The patient interface according to claim 1, wherein the plenum chamber comprises a medial portion and an anterior portion, wherein the medial portion is located so that, in use, the mid-sagittal plane of the patient passes through the medial portion, and wherein the inlet is located on the anterior portion and medial portion.

3. The patient interface according to claim 1 or 2, wherein the vent structure is located on a portion of the patient interface which is lateral to the inlet and is configured to vent the flow of gases from the interior of the plenum chamber in a substantially lateral direction in use.

4. The patient interface according to any one of claims 1 to 3, wherein the vent structure is a first vent structure configured to vent a first flow of gases exhaled by the patient from an interior of the plenum chamber to ambient in a first substantially lateral direction in use, and wherein the patient interface comprises a second vent structure configured to vent a second flow of gases exhaled by the patient from an interior of the plenum chamber to ambient in a second substantially lateral direction in use, wherein the first substantially lateral direction is generally opposite to the second substantially lateral direction.

5. The patient interface according to claim 4, wherein the first vent structure is located lateral to the inlet on one side of the patient interface, and wherein the second vent structure is located lateral to the inlet on the other side of the patient interface.

6. The patient interface according to any one of claims 1 to 5 comprising a deflector configured to redirect, in use, a substantial amount of the laterally vented flow of gases in a direction having an anterior component relative to the patient.

7. The patient interface according to any one of claims 1 to 6, wherein a cover portion of the at least one headgear strap covers at least a portion of the vent structure in use and optionally the diffuser is located on the cover portion.

8. A patient interface comprising:
a plenum chamber pressurisable to a therapeutic pressure of at least 6 cmH₂O above ambient air pressure, wherein the plenum chamber comprises an inlet configured to receive a flow of air at the therapeutic pressure for breathing by a patient;
a seal-forming structure configured to form a seal with a region of the patient's face surrounding an entrance to the patient's airways, wherein the seal-forming structure is configured to maintain said therapeutic pressure in the plenum chamber throughout the patient's respiratory cycle in use;
a vent structure configured to allow a vent flow of gases exhaled by the patient from an interior of the plenum chamber to ambient, wherein the vent structure is configured to maintain the therapeutic pressure in the plenum chamber in use; and
a positioning and stabilising structure to provide a force to hold the seal-forming structure in a therapeutically effective position on the patient's head,
wherein the positioning and stabilising structure comprises a component comprising a vent-facing surface which is positioned in use in a path of the vent flow of gases exiting the vent structure,
wherein a diffuser is located on the vent-facing surface of the component, and wherein the diffuser is positioned in use to diffuse the vent flow of gases exiting the vent structure.

9. The patient interface according to claim 8, wherein the component comprises a frame configured to facilitate indirect attachment of the plenum chamber to at least one headgear strap of the positioning and stabilising structure.

10. The patient interface according to claim 9, wherein the diffuser is removably attached to the frame.

11. The patient interface according to claim 9 or 10, wherein the frame comprises a pair of connectors to facilitate attachment of the frame to the at least one headgear strap.

12. The patient interface according to claim 8, wherein the component comprises at least one tube, wherein the at least one tube is configured, in use, to convey the flow of air from an air circuit fluidly connected to the at least one tube to an interior of the plenum chamber through the inlet and wherein optionally the diffuser is removably attached to the at least one tube.

13. The patient interface according to claim 8, wherein the component comprises a rigidiser arm, wherein the rigidiser arm is configured to rigidise at least one headgear strap of the positioning and stabilising structure and wherein optionally the diffuser is removably attached to the rigidiser arm.

14. A positioning and stabilising structure for holding a patient interface in a therapeutically effective position on a patient's head, the positioning and stabilising structure comprising:
at least one headgear strap,
wherein the at least one headgear strap comprises a diffuser, wherein the diffuser is configured to diffuse a flow of gases vented from an interior of the patient interface to ambient through a vent structure configured on the patient interface.

15. The positioning and stabilising structure according to claim 14, wherein a cover portion of the at least one headgear strap covers at least a portion of the vent structure in use and the diffuser is optionally located on the cover portion.
